# EUROPEAN PATENT APPLICATION

(11) **EP 1 310 511 A2**
(43) Date of publication of application: **14.05.2003**
(21) Application number: 02025060.1
(22) Date of filing: 12.11.2002
(51) Int. Cl.: C07K 7/04, C07K 7/06, C07K 16/18, A61P 17/14

(54) **Oligopeptides for promoting hair growth**

(30) Priority: 13.11.2001 JP 2001347338; 05.12.2001 JP 2001371366; 05.12.2001 JP 2001371175; 13.11.2001 JP 2001347340
(71) Applicant: Sumitomo Electric Industries, Ltd., Osaka-shi, Osaka 541-0041 (JP)
(72) Inventor: Hirai, Yohei, Yokohama Works, Yokohama-shi, Kanagawa 244-8588 (JP); Oka, Yumiko, Yokohama Works, Yokohama-shi, Kanagawa 244-8588 (JP); Tochigi, Keiko, Yokohama Works, Yokohama-shi, Kanagawa 244-8588 (JP); Murakami, Kayoko, Yokohama Works, Yokohama-shi, Kanagawa 244-8588 (JP); Shinagawa, Toko, Yokohama Works, Yokohama-shi, Kanagawa 244-8588 (JP); Takebe, Kyoko, Yokohama Works, Yokohama-shi, Kanagawa 244-8588 (JP)
(74) Representative: Sternagel, Fleischer, Godemeyer & Partner Patentanwälte

(57) **Abstract**

The present invention provides oligopeptides having morphogenesis promoting activity and in particular, hair promoting activity. The oligopeptides may be in monomer form, monomer having a reactive substance bound form or as a polymer, such as a dimer including a homodimer; heterodimer; homotrimer; or heterotrimer. The present invention also provides monoclonal antibodies that specifically recognize a 220kDa antigen of epithelial new hair follicles; hybridomas producing such antibody; and methods and kits for assaying hair growth in mammalian subjects.

## Description

### CROSS-REFERENCE TO RELATED APPLICATIONS

The present application claims the benefit of priority to Japanese application 2001-347340, filed November 13, 2001, Japanese application 2001-347338, filed November 13, 2001, Japanese application 2001-371175 filed December 5, 2001, and Japanese application 2001-371366 filed December 5, 2001, all of which are hereby incorporated herein by reference in their entirety.

### TECHNICAL FIELD

The present invention relates to oligopeptides having morphogenesis activity. In particular, the present invention provides compositions comprising oligopeptides having hair growth promoting activity and methods for promoting hair growth in humans. The present invention also relates to monoclonal antibodies specific for an antigen of epithelial new hair follicle and methods for the evaluation of hair growth promoting activity using such a monoclonal antibody.

### BACKGROUND

The normal morphogenesis of epithelial tissue has been suggested to be controlled by factors derived from mesenchymal cells present around the epithelial tissue. Diseases resulting from the abnormal morphogenesis of epithelial tissue are largely caused by abnormalities of mesenchymal cells. Therefore, an interest has arisen in understanding the mechanism by which mesenchymal cells control the morphogenesis of epithelial tissue.

Epimorphin, disclosed in Japanese Patent Laid-Open Publication No. 25295/94, has 277 to 289 amino acids as a core protein, and has the action of promoting the morphogenesis of epithelial tissue through its action on epithelial cells. It was found that normal tissue formation did not progress when epimorphin failed to function.

Epimorphin has been described in Hirai et al. (1992, *Cell*, 69:471-481); Hirai (1994, *Eur. J. Biochem*, vol. 225, 1133-1139); Hirai, et al. (1998, *J. Cell. Biol.,* 140:159-169); and Hirai, et al. (2001, *J. Cell. Biol*., 153:785-794).

EP 0698666 A2 describes the structure of full length epimorphin as roughly divided into four fragments, beginning from the N-terminus, a coiled coil domain (1), a functional domain (2), a coiled coil domain (3), and hydrophobic domain at the C-terminal. EP0698666A discloses that the functional domain (the domain specified by 104th to 187th amino acids in human epimorphin) participates in cell adhesion and is associated with expression of physiological activity of epimorphin.

U.S. Patent No. 5,726,298, issued March 10, 1998, discloses human and murine epimorphin nucleotide and amino acid sequences. WO98/22505 and EP 1008603A1 describe polypeptides specified by the N-terminal sequence of the 1st to 103rd amino acids of human epimorphin and by the N-terminal sequence of the 1st to 104th amino acids of murine epimorphin.

Native mammalian epimorphin is almost insoluble in an aqueous media such as saline, which causes difficulty in using epimorphin in compositions for human treatment. Japanese Patent Laid-Open Publication No. 25295/1994 discloses a modified form of epimorphin obtained by removing a hydrophobic region at the C-terminus.

In spite of developments in the understanding of epimorphin and morphogenesis of epithelial tissue, there remains a need for means to modify the morphogenesis of epithelial tissue, in particular as it relates to diseases or disorders associated with abnormal morphogenesis.

### DISCLOSURE OF THE INVENTION

The present invention relates to oligopeptides useful for the treatment or amelioration of symptoms of diseases or disorders associated with abnormal morphogenesis. The oligopeptides of the present invention can be used to induce morphogenesis, induce revascularization effect, induce regeneration effect, induce cardiovascular regeneration, and induce endothelial cell growth. The oligopeptides of the. present invention can be used, for example, for the treatment of and/or amelioration of symptoms of burns or wounds or to promote hair growth or prevent hair loss. In particular, the present invention relates to oligopeptides having hair growth promoting activity and to methods of promoting hair growth. The present invention also relates to methods of assaying for hair growth using a monoclonal antibody specific for a 220 kDa antigen of epithelial new follicles and kits comprising a monoclonal antibody of the present invention.

The present invention provides isolated oligopeptides of between about 5 and about 104 amino acid residues in length having hair growth promoting activity, comprising the following amino acid sequence:
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Ala, Tyr, Thr, Pro, Phe, Val, Gly, Leu, Ile or Met, or is deleted from said oligopeptide;
X2 is an amino acid residue of Ile, Gly, Asn, Thr, Val, Ser, Phe, Leu, Ala, Pro, Cys, or Met, or is deleted from said oligopeptide;
X3 is an amino acid residue of Glu, Lys, Gln, Arg, Ala, Val, Trp, Cys, or Asp;
X4 is an amino acid residue of Gln, Pro, Glu, Thr, Arg, Ser, His, Cys, or Lys;
X5 is an amino acid residue of Ser, Trp, Phe, Thr, Cys, Tyr, Pro, Ala, Gly, Val, Leu, Ile, or Met;
X6 is an amino acid residue Cys; a reactive substance-bound Cys or a reactive substance-bound Lys; and
X7 is an amino acid residue of Asp, Glu, His, Ser, Ala, Gly, Asn, Tyr, Arg, or Leu, or is deleted from said oligopeptide, with the proviso that the oligopeptide is not identical to SEQ ID NO:1 or SEQ ID NO:2, and is not identical to oligopeptide Ser-Ile-Glu-Gln-Ser-Cys-Asp or Ser-Ile-Glu-Gln-Ser-Cys.

The present invention also provides isolated oligopeptides of between about 5 and about 104 amino acid residues in length having hair growth promoting activity, comprising the following amino acid sequence:
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Tyr, Thr, or Pro, or is deleted from said oligopeptide;
X2 is an amino acid residue of Ile, Asn, Thr, or Ser, or is deleted from said oligopeptide;
X3 is an amino acid residue of Glu, Ala, Trp, or Asp;
X4 is an amino acid residue of Gln;
X5 is an amino acid residue of Ser, Cys, or Tyr;
X6 is an amino acid residue of Cys; a reactive substance-bound Cys or a reactive substance-bound Lys; and
X7 is an amino acid residue of Asp, Ala, Gly, or Leu, or is deleted from said oligopeptide, with the proviso that the oligopeptide is not SEQ ID NO:2, and is not identical to oligopeptide Ser-Ile-Glu-Gln-Ser-Cys-Asp or Ser-Ile-Glu-Gln-Ser-Cys.

The present invention also provides isolated oligopeptides of between about 7 and about 100 amino acid residues in length having hair growth promoting activity, comprising the following amino acid sequence:
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Ala, Tyr, Thr, Pro, Phe, Val, Gly, Leu, Ile or Met, or is deleted from said oligopeptide;
X2 is an amino acid residue of Ile, Gly, Asn, Thr, Val, Ser, Phe, Leu, Ala, Pro, Cys, or Met, or is deleted from said oligopeptide;
X3 is an amino acid residue of Glu, Lys, Gln, Arg, Ala, Val, Trp, Cys, or Asp;
X4 is an amino acid residue of Gln, Pro, Glu, Thr, Arg, Ser, His, Cys, or Lys;
X5 is an amino acid residue of Ser, Trp, Phe, Thr, Cys, Tyr, Pro, Ala, Gly, Val, Leu, Ile, or Met;
X6 is an amino acid residue Cys; a reactive substance-bound Cys or a reactive substance-bound Lys; and
X7 is an amino acid residue of Asp, Glu, His, Ser, Ala, Gly, Asn, Tyr, Arg, or Leu, or is deleted from said oligopeptide, with the proviso that the oligopeptide is not identical to oligopeptide Ser-Ile-Glu-Gln-Ser-Cys-Asp or Ser-Ile-Glu-Gln-Ser-Cys.

The present invention also provides isolated oligopeptides of between about 7 and about 100 amino acid residues in length having hair growth promoting activity, comprising the following amino acid sequence:
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Tyr, Thr, or Pro, or is deleted from said oligopeptide;
X2 is an amino acid residue of Ile, Asn, Thr, or Ser, or is deleted from said oligopeptide;
X3 is an amino acid residue of Glu, Ala, Trp, or Asp;
X4 is an amino acid residue of Gln;
X5 is an amino acid residue of Ser, Cys, or Tyr;
X6 is an amino acid residue of Cys; and
X7 is an amino acid residue of Asp, Ala, Gly, or Leu, or is deleted from said oligopeptide, with the proviso that the oligopeptide is not identical to oligopeptide Ser-Ile-Glu-Gln-Ser-Cys-Asp or Ser-Ile-Glu-Gln-Ser-Cys.

In some examples, an oligopeptide having hair growth promoting activity comprises T/Y-S/N-E-Q-S-C-A. (SEQ ID NO:3).

In some examples, the present invention provides oligopeptides having hair growth promoting activity which comprise at least an amino acid sequence wherein 1 to 3 amino acid residues are substituted in the amino acid sequence for murine pep7 region, Ser-Ile-Glu-Gln-Ser-Cys-Asp, wherein the amino acid residue to be substituted is other than Cys or the amino acid residue to be substituted is other than the third to sixth amino acid residues Glu-Gln-Ser-Cys. In other examples, the present invention provides oligopeptides wherein 0 to 2 amino acid residues are substituted in the amino acid sequence represented by Glu-Gln-Ser-Cys-Asp; wherein the amino acid residue to be substituted is other than Cys or the amino acid residue to be substituted is other than the third to sixth amino acid residues Glu-Gln-Ser-Cys. In some examples, the present invention provides oligopeptides wherein the first Ser is substituted with a hydrophobic amino acid residue or a neutral amino acid residue in the amino acid sequence of Ser-Ile-Glu-Gln-Ser-Cys-Asp.

In other examples, the present invention provides oligopeptides wherein the first Ser is substituted with Ala, Tyr, Thr, Pro, Phe, Val or Gly in the amino acid sequence of Ser-Ile-Glu-Gln-Ser-Cys-Asp. In some examples, the present invention provides oligopeptides wherein the second Ile is substituted with a neutral amino acid residue or a hydrophobic amino acid residue in the amino acid sequence of Ser-Ile-Glu-Gln-Ser-Cys-Asp. In further examples, the present invention provides oligopeptides wherein the second Ile is substituted with Gly, Asn, Thr, Val, Ser, Phe or Leu in the amino acid sequence of Ser-Ile-Glu-Gln-Ser-Cys-Asp. In some examples, the present invention provides oligopeptides wherein the fifth Ser is substituted with a neutral amino acid residue or a hydrophobic amino acid residue in the amino acid sequence of Ser-Ile-Glu-Gln-Ser-Cys-Asp. In other examples, the present invention provides oligopeptides wherein the fifth Ser is substituted with Trp, Phe, Thr, Cys, Tyr, Pro or Ala in the amino acid sequence of Ser-Ile-Glu-Gln-Ser-Cys-Asp. In further examples, the present invention provides oligopeptides wherein the seventh Asp is substituted with a hydrophilic amino acid residue, Gly, Ala or Leu in the amino acid sequence of Ser-Ile-Glu-Gln-Ser-Cys-Asp. The present invention also provides oligopeptides wherein the seventh Asp is substituted with Glu, His, Ser, Ala, Gly, Asn, Tyr or Leu in the amino acid sequence of Ser-Ile-Glu-Gln-Ser-Cys-Asp. In some examples, the present invention provides oligopeptides wherein the third Glu is substituted with Lys, Gly, Gln, Arg, Ala, Val Asp or Trp in the amino acid sequence of Ser-Ile-Glu-Gln-Ser-Cys-Asp. In other examples, the present invention provides oligopeptides wherein the fourth Gln is substituted with Pro, Glu, Thr, Arg, Ser, His or Lys in the amino acid sequence of Ser-Ile-Glu-Gln-Ser-Cys-Asp. In some examples, the present invention provides oligopeptides wherein the first Ser is substituted with Thr or Tyr, the second Ile is substituted with Ser, Asn or Thr, the third Glu is substituted with Ala, Asp or Trp, the fifth Ser is substituted with Cys or Tyr, and/or the seventh Asp is substituted with Gly, Ala or Leu in the amino acid sequence of Ser-Ile-Glu-Gln-Ser-Cys-Asp. In further examples, the present invention provides oligopeptides wherein 1 to 3 amino acid residues other than the third to sixth amino acid residues Glu-Gln-Ser-Cys are substituted in the amino acid sequence of Ser-Ile-Glu-Gln-Ser-Cys-Asp, the first Ser is substituted with Thr or Tyr, the second Ile is substituted with Ser, Asn or Thr, and/or the seventh Asp is substituted with Gly, Ala or Leu.

In some examples, the present invention provides oligopeptides having hair growth promoting activity which comprise at least an amino acid sequence wherein 1 to 3 amino acid residues are substituted in the amino acid sequence Ser-Ile-Glu-Gln-Cys-Ser-Asp (a mutation of the murine pep7 region wherein Cys is in the 5th amino acid position of the region) wherein the amino acid residue to be substituted is other than Cys or other than the third to sixth amino acid residues Glu-Gln-Cys-Ser. In further examples, the present invention provides oligopeptides wherein 0 to 2 amino acid residues are substituted in the amino acid sequence represented by Glu-Gln-Cys-Ser-Asp; wherein the amino acid residue to be substituted is other than Cys or other than the third to sixth amino acid residues Glu-Gln-Cys-Ser. In some examples, the present invention provides oligopeptides wherein the first Ser is substituted with a hydrophobic amino acid residue or a neutral amino acid residue in the amino acid sequence of Ser-Ile-Glu-Gln-Cys-Ser-Asp. In other examples, the present invention provides oligopeptides wherein the first Ser is substituted with Ala, Tyr, Thr, Pro, Phe, Val or Gly in the amino acid sequence of Ser-Ile-Glu-Gln-Cys-Ser-Asp. In some examples, the present invention provides oligopeptides wherein the second Ile is substituted with a neutral amino acid residue or a hydrophobic amino acid residue in the amino acid sequence of Ser-Ile-Glu-Gln-Cys-Ser-Asp. In further examples, the present invention provides oligopeptides wherein the second Ile is substituted with Gly, Asn, Thr, Val, Ser, Phe or Leu in the amino acid sequence of Ser-Ile-Glu-Gln-Cys-Ser-Asp. In additional examples, the present invention provides oligopeptides wherein the sixth Ser is substituted with a neutral amino acid residue or a hydrophobic amino acid residue in the amino acid sequence of Ser-Ile-Glu-Gln-Cys-Ser-Asp. In some examples, the present invention provides oligopeptides wherein the sixth Ser is substituted with Trp, Phe, Thr, Cys, Tyr, Pro or Ala in the amino acid sequence of Ser-Ile-Glu-Gln-Cys-Ser-Asp. The present invention also provides oligopeptides wherein the seventh Asp is substituted with a hydrophilic amino acid residue, Gly, Ala or Leu in the amino acid sequence of Ser-Ile-Glu-Gln-Cys-Ser-Asp. In some examples, the present invention provides oligopeptides wherein the seventh Asp is substituted with Glu, His, Ser, Ala, Gly, Asn, Tyr or Leu in the amino acid sequence of Ser-Ile-Glu-Gln-Cys-Ser-Asp. In other examples, the present invention provides oligopeptides wherein the third Glu is substituted with Lys, Gly, Gln, Arg, Ala, Val, Asp or Trp in the amino acid sequence of Ser-Ile-Glu-Gln-Cys-Ser-Asp. In some examples, the present invention provides oligopeptides wherein the fourth Gln is substituted with Pro, Glu, Thr, Arg, Ser, His or Lys in the amino acid sequence of Ser-Ile-Glu-Gln-Cys-Ser-Asp. In other examples, the present invention provides oligopeptides wherein the first Ser is substituted with Thr or Tyr, the second Ile is substituted with Ser, Asn or Thr, the third Glu is substituted with Ala, Asp or Trp, the sixth Ser is substituted with Cys or Tyr, and/or the seventh Asp is substituted with Gly, Ala or Leu in the amino acid sequence of Ser-Ile-Glu-Gln-Cys-Ser-Asp. In some examples, the present invention provides oligopeptides wherein 1 to 3 amino acid residues other than the third to sixth amino acid residues Glu-Gln-Cys-Ser are substituted in the amino acid sequence of Ser-Ile-Glu-Gln-Cys-Ser-Asp, the first Ser is substituted with Thr or Tyr, the second Ile is substituted with Ser, Asn or Thr, and/or the seventh Asp is substituted with Gly, Ala or Leu.

In other examples, the present invention provides oligopeptides which comprises at least an amino acid sequence wherein 1 to 3 amino acid residues are substituted in the amino acid sequence Ser-Ile-Glu-Cys-Gln-Ser-Asp (a mutation of the murine pep7 region wherein Cys is in the 4th amino acid position of the region) wherein the amino acid residue to be substituted is other than Cys or wherein the amino acid residue to be substituted is other than the third to sixth amino acid residues Glu-Cys-Gln-Ser. In further examples, the present invention provides an oligopeptide wherein 0 to 2 amino acid residues are substituted in the amino acid sequence represented by Glu-Cys-Gln-Ser-Asp; wherein the amino acid residue to be substituted is other than Cys or wherein the amino acid residue to be substituted is other than the third to sixth amino acid residues Glu-Cys-Gln-Ser. In further examples, the present invention provides oligopeptides wherein the first Ser is substituted with a hydrophobic amino acid residue or a neutral amino acid residue in the amino acid sequence of Ser-Ile-Glu-Cys-Gln-Ser-Asp. In some examples, the present invention provides oligopeptides wherein the first Ser is substituted with Ala, Tyr, Thr, Pro, Phe, Val or Gly in the amino acid sequence of Ser-Ile-Glu-Cys-Gln-Ser-Asp. The present invention further provides oligopeptides wherein the second Ile is substituted with a neutral amino acid residue or a hydrophobic amino acid residue in the amino acid sequence of Ser-Ile-Glu-Cys-Gln-Ser-Asp. In some examples, the present invention provides oligopeptides wherein the second Ile is substituted with Gly, Asn, Thr, Val, Ser, Phe or Leu in the amino acid sequence of Ser-Ile-Glu-Cys-Gln-Ser-Asp. In other examples, the present invention provides oligopeptides wherein the sixth Ser is substituted with a neutral amino acid residue or a hydrophobic amino acid residue in the amino acid sequence of Ser-Ile-Glu-Cys-Gln-Ser-Asp. In some examples, the present invention provides oligopeptides wherein the sixth Ser is substituted with Trp, Phe, Thr, Cys, Tyr, Pro or Ala in the amino acid sequence of Ser-Ile-Glu-Cys-Gln-Ser-Asp. In yet further examples, the present invention provides oligopeptides wherein the seventh Asp is substituted with a hydrophilic amino acid residue, Gly, Ala or Leu in the amino acid sequence of Ser-Ile-Glu-Cys-Gln-Ser-Asp.

In additional examples, the present invention provides oligopeptides wherein the seventh Asp is substituted with Glu, His, Ser, Ala, Gly, Asn, Tyr or Leu in the amino acid sequence of Ser-Ile-Glu-Cys-Gln-Ser-Asp. In some examples, the present invention provides oligopeptides wherein the third Glu is substituted with Lys, Gly, Gln, Arg, Ala, Val, Asp or Trp in the amino acid sequence of Ser-Ile-Glu-Cys-Gln-Ser-Asp. In other examples, the present invention provides oligopeptides wherein the fifth Gln is substituted with Pro, Glu, Thr, Arg, Ser, His or Lys in the amino acid sequence of Ser-Ile-Glu-Cys-Gln-Ser-Asp. In yet other examples, the present invention provides oligopeptides wherein the first Ser is substituted with Thr or Tyr, the second Ile is substituted with Ser, Asn or Thr, the third Glu is substituted with Ala, Asp or Trp, the sixth Ser is substituted with Cys or Tyr, and/or the seventh Asp is substituted with Gly, Ala or Leu in the amino acid sequence of Ser-Ile-Glu-Cys-Gln-Ser-Asp. In other examples, the present invention provides oligopeptides wherein 1 to 3 amino acid residues other than the third to sixth amino acid residues Glu-Cys-Gln-Ser are substituted in the amino acid sequence of Ser-Ile-Glu-Cys-Gln-Ser-Asp, the first Ser is substituted with Thr or Tyr, the second Ile is substituted with Ser, Asn or Thr, and/or the seventh Asp is substituted with Gly, Ala or Leu.

In some examples, the present invention provides oligopeptides wherein 1 to 3 amino acid residues are substituted in the amino acid sequence represented by Ser-Ile-Cys-Glu-Gln-Ser-Asp (a mutation of the murine pep7 region wherein Cys is in the 3rd amino acid position of the region) wherein the amino acid residue to be substituted is other than Cys or other than the third to sixth amino acid residues Cys-Glu-Gln-Ser. In further examples, the present invention provides oligopeptides wherein 0 to 2 amino acid residues are substituted in the amino acid sequence represented by Cys-Glu-Gln-Ser-Asp, wherein the amino acid residue to be substituted is other than Cys or other than the third to sixth amino acid residues Cys-Glu-Gln-Ser. In some examples, the present invention provides oligopeptides wherein the first Ser is substituted with a hydrophobic amino acid residue or a neutral amino acid residue in the amino acid sequence of Ser-Ile-Cys-Glu-Gln-Ser-Asp. In some examples, the present invention provides oligopeptides wherein the first Ser is substituted with Ala, Tyr, Thr, Pro, Phe, Val or Gly in the amino acid sequence of Ser-Ile-Cys-Glu-Gln-Ser-Asp. In other examples, the present invention provides oligopeptides wherein the second Ile is substituted with a neutral amino acid residue or a hydrophobic amino acid residue in the amino acid sequence of Ser-Ile-Cys-Glu-Gln-Ser-Asp. In additional examples, the present invention provides oligopeptides wherein the second Ile is substituted with Gly, Asn, Thr, Val, Ser, Phe or Leu in the amino acid sequence of Ser-Ile-Cys-Glu-Gln-Ser-Asp. In other examples, the present invention provides oligopeptides wherein the sixth Ser is substituted with a neutral amino acid residue or a hydrophobic amino acid residue in the amino acid sequence of Ser-Ile-Cys-Glu-Gln-Ser-Asp. In some examples, the present invention provides oligopeptides wherein the sixth Ser is substituted with Trp, Phe, Thr, Cys, Tyr, Pro or Ala in the amino acid sequence of Ser-Ile-Cys-Glu-Gln-Ser-Asp. In further examples, the present invention provides oligopeptides wherein the seventh Asp is substituted with a hydrophilic amino acid residue, Gly, Ala or Leu in the amino acid sequence of Ser-Ile-Cys-Glu-Gln-Ser-Asp. In other examples, the present invention provides oligopeptides wherein the seventh Asp is substituted with Glu, His, Ser, Ala, Gly, Asn, Tyr or Leu in the amino acid sequence of Ser-Ile-Cys-Glu-Gln-Ser-Asp. In additional examples, the present invention provides oligopeptides wherein the fourth Glu is substituted with Lys, Gly, Gln, Arg, Ala, Val, Asp or Trp in the amino acid sequence of Ser-Ile-Cys-Glu-Gln-Ser-Asp. In some examples, the present invention provides oligopeptides wherein the fifth Gln is substituted with Pro, Glu, Thr, Arg, Ser, His or Lys in the amino acid sequence of Ser-Ile-Cys-Glu-Gln-Ser-Asp. In further examples, the present invention provides oligopeptides wherein the first Ser is substituted with Thr or Tyr, the second Ile is substituted with Ser, Asn or Thr, the fourth Glu is substituted with Ala, Asp or Trp, the sixth Ser is substituted with Cys or Tyr, and/or the seventh Asp is substituted with Gly, Ala or Leu in the amino acid sequence of Ser-Ile-Cys-Glu-Gln-Ser-Cys-Asp. The present invention also provides oligopeptides wherein 1 to 3 amino acid residues other than the third to sixth amino acid residues Cys-Glu-Gln-Ser are substituted in the amino acid sequence of Ser-Ile-Cys-Glu-Gln-Ser-Asp, the first Ser is substituted with Thr or Tyr, the second Ile is substituted with Ser, Asn or Thr, and/or the seventh Asp is substituted with Gly, Ala or Leu.

In some examples, the present invention provides oligopeptides wherein 1 to 3 amino acid residues are substituted in the amino acid sequence represented by Glu-Gln-Ser-Cys-Asp. In other examples, the present invention provides oligopeptides
wherein 1 to 3 amino acid residues are substituted in the amino acid sequence represented by Glu-Gln-Cys-Ser-Asp. In some examples, the present invention provides oligopeptides wherein 1 to 3 amino acid residues are substituted in the amino acid sequence represented by Glu-Cys-Gln-Ser-Asp. In other examples, the present invention provides oligopeptides wherein 1 to 3 amino acid residues are substituted in the amino acid sequence represented by Cys-Glu-Gln-Ser-Asp.

The present invention also provides oligopeptides comprising between about 8 and about 20 amino acid residues and comprising an amino acid sequence selected from the group consisting of: 
Ser-Ile-Glu-Gln-Xaa-Ser-Asp-Gln;
Ser-Ile-Glu-Xaa-Gln-Ser-Asp-Gln; and
Ser-Ile-Xaa-Glu-Gln-Ser-Asp-Gln,
 wherein Xaa is Cys, or a reactive substance-bound Cys.

The present invention also provides oligopeptides comprising an amino acid sequence selected from the group consisting of:

Ser-Ile-Glu-Gln-Cys-Ser-Asp-Gln;
Ser-Ile-Glu-Cys-Gln-Ser-Asp-Gln; and
Ser-Ile-Cys-Glu-Gln-Ser-Asp-Gln.

The present invention also provides oligopeptides comprising an amino acid sequence of any one of SEQ ID NO:3 through SEQ ID NO:124. The present invention also provides oligopeptides comprising the following amino acid sequences: Tyr Asn Glu Gln Ser Cys Asp Arg Glu Glu; Thr Ser Asp Gln Cys Cys Asp Pro Asp Lys; Pro Ser Glu Gln Ser Cys Ala Glu Glu Glu; Ser Asn Glu Gln Ser Cys Ala Val Ala Glu; Thr Thr Glu Gln Ser Cys Ala Val Asp Glu; Ser Ile Glu Gln Ser Cys Gly Gln His Glu; Ser Ser Ala Gln Ser Cys Leu Gln Asp Thr; Tyr Ile Glu Gln Tyr Cys Asp Gln Asp Glu; or Thr Ile Trp Gln Ser Cys Asp Gln Glu Glu.

An oligopeptide of the present invention encompasses oligopeptides comprising natural amino acid residues, non-natural amino acid residues, or a mixture of both. An oligopeptide of the present invention encompasses oligopeptides that are modified with a cross-linking agent. The present invention also encompasses oligopeptide polymers comprising at least two oligopeptides of the present invention that are cross-linked, with the proviso that the polymer is not a homopolymer of SEQ ID NO:1 or SEQ ID NO:2 and with the proviso that the polymer is not a homopolymer of oligopeptide Ser-Ile-Glu-Gln-Ser-Cys-Asp or Ser-Ile-Glu-Gln-Ser-Cys. In some examples, the polymer is a dimer, including a homodimer as well as a heterodimer. In other examples, the polymer is a trimer. The present invention encompasses oligopeptide polymers prepared by cross-linking at least two oligopepetides of the present invention, with the proviso that the polymer is not a homopolymer of SEQ ID NO:1 or SEQ ID NO:2, and is not a homopolymer of oligopeptide Ser-Ile-Glu-Gln-Ser-Cys-Asp or Ser-Ile-Glu-Gln-Ser-Cys.

The present invention encompasses compositions comprising an oligopeptide of the present invention. In some examples, the composition further comprises a pharmaceutically acceptable excipient. In other examples, the composition comprises an agent that enhances transdermal penetration or delivery.

The present invention also provides methods for promoting hair growth in a mammal comprising administering a composition comprising an oligopeptide of the present invention to a mammal in need of hair growth in an amount effective to promote hair growth in said mammal.

The present invention also provides monoclonal antibodies, or fragments thereof, which specifically recognize an antigen of about 220 kDa present in epithelial new follicles. In some examples, the antigen of about 220 kDa present in epithelial new follicles is an antigen which is specifically expressed during the growth period of an imago or the developing period of a fetus. In other examples, the present invention provides a hybridoma deposited with the Patent and Bio-Resource Center of National Institute of Advanced Industrial Science and Technology (Japan) and having an accession number of FERM P-18578 and a monoclonal antibody made by said hybridoma. In other examples, the present invention provides an antigen, or fragment thereof, recognized by the monoclonal antibody made by the hybridoma deposited with the Patent and Bio-Resource Center of National Institute of Advanced Industrial Science and Technology and having an accession number of FERM P-18578

The present invention also provides a hybridoma produced by the method comprising fusing immunocytes of a mammal immunized with an immunogen containing protein extracted from hair collected from the skin of a mammal in the growth period and/or follicles of whiskers of a mammal in a growth period, and myeloma cells of a mammal. The present invention also provides a process for the production of a monoclonal antibody specific for an antigen of about 220 kDa present in epithelial new follicles, which comprises the steps of incubating the hybridoma deposited with the Patent and Bio-Resource Center of National Institute of Advanced Industrial Science and Technology and having an accession number of FERM P-18578 and collecting the monoclonal antibody produced by said hybridoma.

The present invention also provides methods for the evaluation of hair growth promoting activity comprising the steps of; (1) incubating skin tissue derived from a mammal in the presence of a substance to be tested under suitable conditions and for a time effective to promote hair growth; (2) recovering said skin tissue from step (1); and (3) reacting said skin tissue with the monoclonal antibody made by a hybridoma deposited with the Patent and Bio-Resource Center of National Institute of Advanced Industrial Science and Technology and having an accession number of FERM P-18578 or a fragment thereof; and; (4) detecting said monoclonal antibody or a fragment thereof that reacted with the skin tissue.

The present invention also provides kits comprising a monoclonal antibody made by the hybridoma deposited with the Patent and Bio-Resource Center of National Institute of Advanced Industrial Science and Technology and having an accession number of FERM P-18578

### BRIEF DESCRIPTION OF THE DRAWINGS

Fig. 1 shows the result of analysis of epimorphin (EPM) pep7 based library. The mutation ratio is shown as a percentage taking a naturally occurring region of murine epimorphin comprising the pep7 region as 100%.
Fig. 2 shows the result of analysis of the contents of epimorphin (EPM) pep7 based library.
Fig. 3 shows theory values present in epimorphin (EPM) pep7 based library.
Fig.4 shows the result of an analysis for hair growth promoting activity using homodimer oligopeptides as described in Examples 9-10.
Fig. 5 shows the result of a test for hair growth promoting activity using heterodimer oligopeptide and homodimer oligopeptides as described in Examples 9-10.

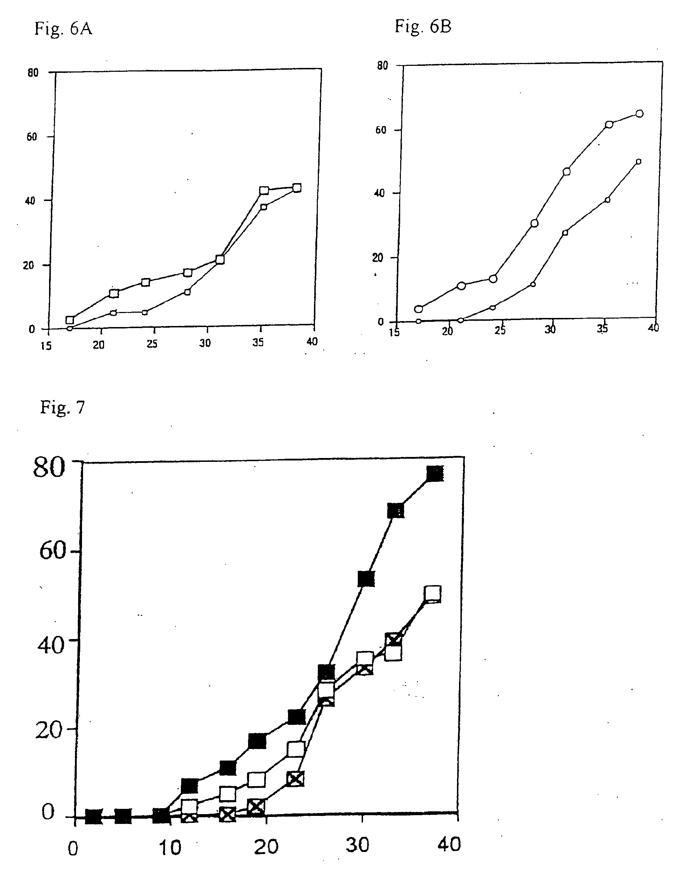

Figs. 6A-6B show a hair growth promoting activity of an oligopeptide of the present invention (represented by the amino acid sequence Ser-Ile-Glu-Gln-Ser-Cys-Asp-Gln-Asp-Glu). Figure 6 (A) shows the result obtained by a biotinylated oligopeptide. Large square shows the result obtained by oligopeptide b7 (b7 is SIEQSCDQDE), and small square shows the result obtained by the control (control is a blank). Figure 6 (B) shows the result obtained by a S-S bridged and biotinylated oligopeptide. Large circle shows the result obtained by oligopeptide ssb7 (cross-linked b7), and small circle shows the result obtained by the control. The vertical axis indicates hair growth score, and the horizontal axis indicates the day from the start of application.
Fig. 7 shows a hair growth promoting activity of the oligopeptide of the present invention (S-S bridged and biotinylated oligopeptide:ss7). In the figure, ■ indicates the S-S bridged and biotinylated oligopeptide, □ indicates the result obtained from the first control (same as the control in Fig.6) and 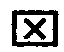 shows the result of the second control (random 7-mer oligopeptide). The vertical axis indicates hair growth score, and the horizontal axis indicates the day from the start of application.

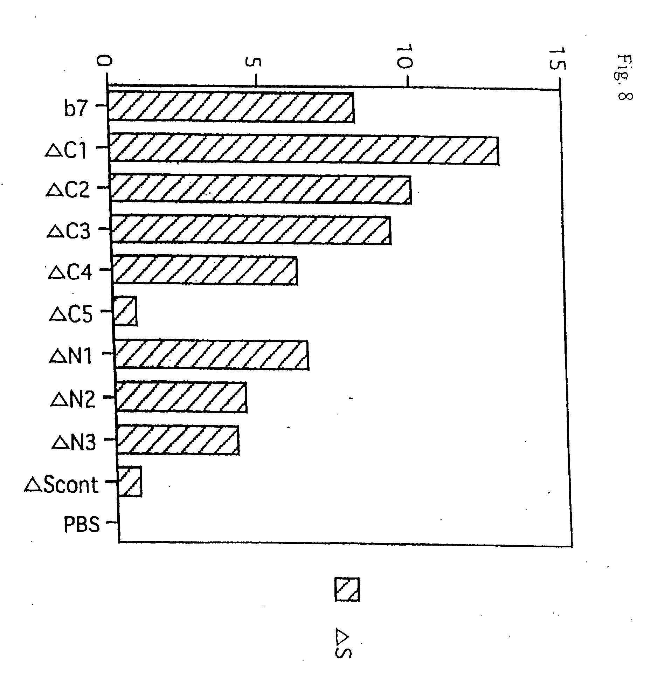

Fig. 8 shows the result of evaluation of b7ΔC1, b7ΔC2, b7ΔC3, b7ΔC4, b7ΔC5, b7ΔN1, b7ΔN2, and b7ΔN3 on IL-8 inducing activity. b7ΔC1 refers to the oligopeptide sequence (SIEQSCDQD); b7ΔC2 refers to the oligopeptide sequence (SIEQSCDQ); b7ΔC3 refers to the oligopeptide sequence (SIEQSCD); b7ΔC4 refers to the oligopeptide sequence (SIEQSC); b7ΔC5 refers to the oligopeptide sequence (SIEQS); b7ΔN1 refers to the oligopeptide sequence (IEQSCDQDE); b7ΔN2 refers to the oligopeptide sequence (EQSCDQDE); and b7ΔN3 refers to the oligopeptide sequence (QSCDQDE). In the figure, Scont indicates the result of blocking reagent, PBS indicates the result of phosphate buffered saline, and the vertical axis indicates the relative value of the secretion amount of IL-8.
Fig. 9 shows the results of evaluation of oligopeptide bk7 obtained by binding biotin to the N-terminal of the oligopeptide represented by the amino acid sequence Lys-Ser-Ile-Glu-Gln-Ser-Cys-Asp-Gln-Asp-Glu and oligopeptide b7 (SIEQSCDQDE) on IL-8 inducing activity, as described in Figs. 17 and 18. In the figure, PBS indicates the result of phosphate buffered saline, and the vertical axis indicates the relative value of the secretion amount of IL-8.
Figs. 10A-10C show the result obtained by analyzing the reaction product of the oligopeptide and the cross-linking agent with gel permeation column as disclosed in the Example 12.
Fig. 11 shows the result of the evaluation of the hair growth promoting activity of the modified and unmodified oligopeptides as disclosed in Example 13. The oligopeptide in one letter code is SIEQSXDQDE, wherein X is an unmodified or modified Cys or Lys as indicated.

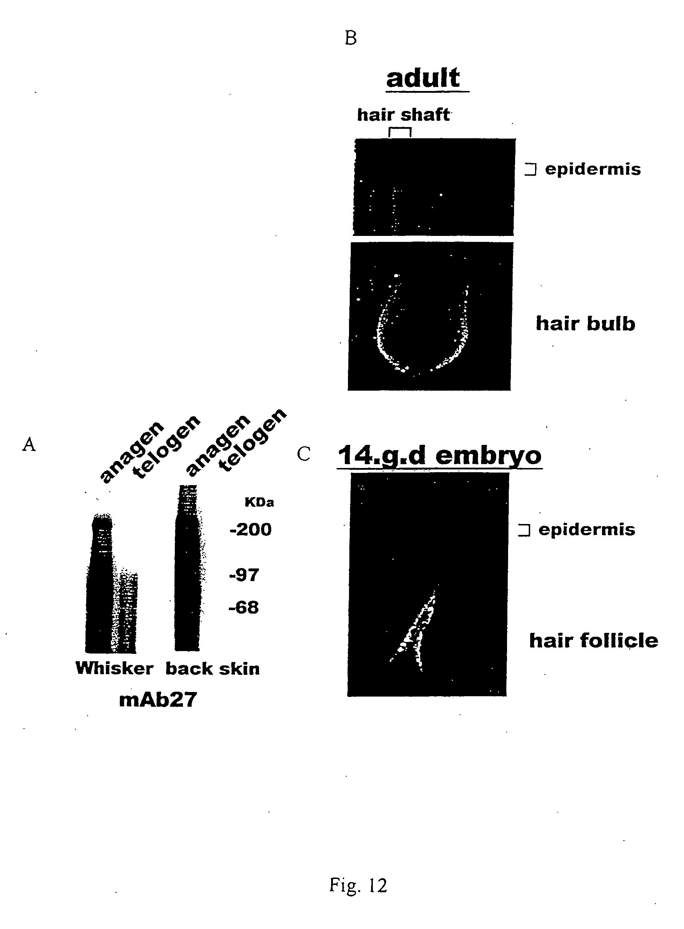

Figs. 12A-12C show the result of the immune analysis using a monoclonal antibody mAb27 obtained in the present invention. Fig. 12 (A) shows the result of detection of the antigen of mAb27 by Western blotting. Each of the lanes from left to right shows the result of the analysis where each protein extracted from the anagen and telogen stage of whisker and the anagen and telogen stage of back skin was subjected to electrophoresis and Western blotting, and then the antigen was detected by using the monoclonal antibody mAb27. Fig. 12B shows the result of histological staining using hair of adult and 12C maxilla of 14th day mouse embryo.
Fig. 13 shows the result of the evaluation of a hair growth promoting activity of oligopeptide using a monoclonal antibody mAb27.
Fig. 14 shows the amino acid sequence (SEQ ID NO: 1) of the human epimorphin coiled coil domain from amino acid residue 1 to amino acid residue 103 as disclosed in EP 0698666A2. The "pep7" region is underlined.
Fig. 15 shows the amino acid sequence (SEQ ID NO:2) of the murine epimorphin coiled coil domain from amino acid residue 1 to amino acid residue 104 as disclosed in EP 0698666A2. The "pep7" region is underlined.

### BEST MODE FOR CARRYING OUT THE INVENTION

The present invention relates to oligopeptides useful for the treatment or amelioration of symptoms of diseases or disorders associated with abnormal morphogenesis. The oligopeptides of the present invention can be used to induce morphogenesis, induce revascularization effect, induce regeneration effect, induce cardiovascular regeneration, and induce endothelial cell growth. The oligopeptides of the present invention can be used for the treatment of and/or amelioration of symptoms of, for example, burns or wounds or to promote hair growth or prevent hair loss. In particular, the present invention relates to oligopeptides having hair growth promoting activity and to methods of promoting hair growth. The present invention also provide antibodies, including polyclonal antibodies, monoclonal antibodies and fragments thereof, that specifically bind an oligopeptide of the present invention that are useful for the detection, quantitation, separation or purification of the oligopeptides.

The present invention also provides monoclonal antibodies specific for a 220 kDa antigen of epithelial new follicles and methods of assaying for hair growth using such monoclonal antibody. The present invention encompasses monoclonal antibodies specific for a 220kDa antigen of epithelial new follicles as produced by the hybridoma deposited with the Patent and Bio-Resource Center of National Institute of Advanced Industrial Science and Technology and having accession number FERM P-18578 (FERM BP-8121).

The present invention is based, in part, upon the observation that oligopeptides of the present invention have hair growth promoting activity as monomers, that is as a single oligopeptide, and in particular as monomers that are capable of dimerizing under conditions suitable for dimerizing, as monomers having a reactive substance bound, and as polymers, such as, a dimer, including homodimers and heterodimers, and trimers. Hair growth promoting activity can be measured by the assay disclosed herein in Example 7 and by means known to those of skill in the art.

The practice of the present invention will employ, unless otherwise indicated, conventional techniques of molecular biology (including recombinant techniques), cell biology, biochemistry, and immunology, which are within the skill of the art. Such techniques are explained fully in the literature, such as, Molecular Cloning: A Laboratory Manual, Second Edition (Sambrook et al., 1989); Oligonucleotide Synthesis (M.J.Gait, ed., 1984); Animal Cell Culture (R.I. Freshney, ed., 1987); Methods in Enzymology (Academic Press, Inc.); Handbook of Experimental Immunology (D,M. Wei & C.C. Blackwell, eds.); Gene Transfer Vectors for Mammalian Cells (J.M. Miller & M.P. Calos, eds., 1987); Current Protocols in Molecular Biology (F.M. Ausubel et al., eds., 1987 and annual updates); PCR : The Polymerase Chain Reaction (Mullis et al., eds., 1994); Current Protocols in Immunology (J.E. Coligan et al., eds., 1991 and annual updates).

### Epimorphin sequences

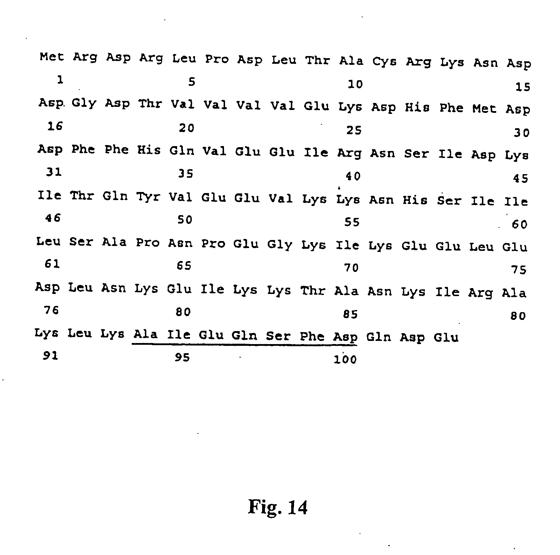

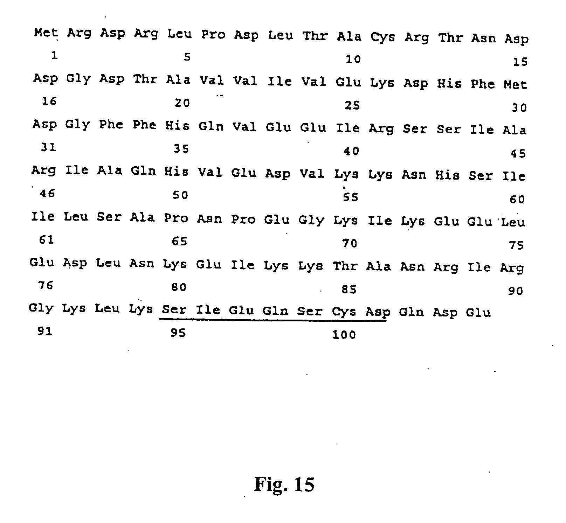

Human and murine epimorphin amino acid sequences are disclosed in U.S. Patent No. 5,726,298. U.S. Patent No. 5,726,298 also discloses isoforms of human and murine epimorphin. Human epimorphin pep 7 amino acid region begins at amino acid position 94 of SEQ ID NOS: 3-5 of U.S. Patent No. 5,726,298 and has the following sequence: Ala-Ile-Glu-Gln-Ser-Phe-Asp (SEQ ID NO:137) and is shown herein in Figure 14. Murine epimorphin pep7 amino acid region begins at amino acid position 95 of SEQ ID NOS: 9-11 of U.S. Patent No. 5,726,298 and has the following sequence: Ser-Ile-Glu-Gln-Ser-Cys-Asp (SEQ ID NO: 138) and is shown herein in Figure 15. In the murine pep7 region, the first Ala of human pep7 is replaced by a Ser and the sixth Phe of human pep7 region is replaced by a Cys.

In the protein of both human and murine epimorphin, the pep7 regions lie in the coiled-coil domain of the respective epimorphins by which polypeptide chains have access to other polypeptide chains for the formation of dimers, even in the absence of Cys in the human epimorphin pep7 region. The present invention is based, in part, upon the observation that mutations of the human epimorphin pep7 region Ala-Ile-Glu-Gln-Ser-Phe-Asp that comprises a replacement of certain amino acid residues with Cys, demonstrate the ability to induce the morphogenesis of new hair follicles in the mouse model disclosed herein in Example 7. The present invention is further based upon the observation that a pep7 region mutant comprising a Cys in position 6, and which has the ability to dimerize with other pep7 regions that comprise a Cys under conditions that permit dimerization, has activity in the mouse model whereas a pep7 region mutant having a replacement of Cys with Phe, that is that has no Cys, has no activity in the mouse model disclosed herein in the Examples.

Based upon the fact that the pep7 amino acid regions of human and murine are highly conserved and that mutations of the human pep7 regions that comprise a Cys demonstrate activity in the mouse model disclosed herein in Example 7, it is predicted that the oligopeptides disclosed herein will induce hair growth and/or prevent hair loss in humans. Oligopeptides in monomer form and as monomers having a reactive substance bound exhibit hair promoting activity. Oligopeptides in dimer form exhibit the highest activity as measured by the assay disclosed herein in Example 7.

### Oligopeptides

Oligopeptides encompassed within the present invention are based on the human or murine epimorphin pep7 amino acid region disclosed in EP 0698666A2 (see also Figures 14 and 15, herein) and U.S. Pat.No. 5,726, 298. Genes encoding human epimorphin and isoforms thereof are disclosed in U.S. Pat. No. 5,726,298 as SEQ ID NOS: 6, 7 and 8. Genes encoding murine epimorphin and isoforms thereof are disclosed in U. S. Pat. No. 5,726,298 as SEQ ID NOS: 12, 13 and 14.

In some examples, an oligopeptide comprises mutations in and around the human or murine pep7 region, that is, mutations occur within the 7 amino acid pep7 region and may additionally occur within 1-4 amino acids in the N-terminal direction and/or in the C-terminal direction to the pep7 region in human or murine epimorphin. As used herein, a "mutation" includes but it not limited to amino acid substitution(s) and/or deletion(s) and/or insertion(s) and/or addition(s). Oligopeptides of the invention comprise between about 5 to about 104 amino acid residues in length, and, in some examples have the amino acid sequence as disclosed herein in SEQ ID NOs: 3-124, with the proviso that the oligopeptide is not identical to SEQ ID NO: 1 or SEQ ID NO:2 and not identical to.oligopeptide Ser-Ile-Glu-Gln-Ser-Cys-Asp or Ser-Ile-Glu-Gln-Ser-Cys and maintain at least one biological activity. In some examples, an oligopeptide consists of a pep7 region mutation, as long as the oligopeptide maintains at least one biological activity. In other examples, an oligopeptide comprises a pep7 region mutation and, in particular, as disclosed herein in SEQ ID NOs: 3-124, as long as the oligopeptide maintains at least one biological activity. The present invention encompasses oligopeptides comprising a pep7 region mutation as disclosed herein and, in particular, as disclosed herein in SEQ ID NOs: 3-124, and in some examples, the amino acid residues in the N-terminal direction and/or the C-terminal direction to the pep7 region mutation in the oligopeptide are the naturally occurring amino acid sequences in the N-terminal direction and/or the C-terminal direction to human or murine epimorphin pep7 region and may be of any length up to and including full length human and murine epimorphin or any isoform of human or murine epimorphin, as long as the oligopeptide maintains at least one biological activity, with the proviso that the oligopeptide is not identical to SEQ ID NO: 1 or SEQ ID NO:2 and not identical to oligopeptide Ser-Ile-Glu-Gln-Ser-Cys-Asp or Ser-Ile-Glu-Gln-Ser-Cys.

The present invention encompasses oligopeptides comprising a pep7 region mutation as disclosed herein and, in particular, as disclosed herein in SEQ ID NOs: 3-124, and in some examples, the amino acid residues in the N-terminal direction and/or the C-terminal direction to the pep7 region mutation in the oligopeptide comprise substitution(s), in particular conservative substitution(s) and/or, deletion(s) and/or, addition(s) and/or insertion(s) to the naturally occurring amino acid sequences in the N-terminal direction and/or the C-terminal direction to human or murine epimorphin pep7 region or any isoform of human or murine epimorphin and may be of any length up to and including full length human and murine epimorphin, as long as the oligopeptide maintains at least one biological activity, with the proviso that the oligopeptide is not identical to SEQ ID NO: 1 or SEQ ID NO:2 and not identical to oligopeptide Ser-Ile-Glu-Gln-Ser-Cys-Asp or Ser-Ile-Glu-Gln-Ser-Cys. In other examples, the amino acid residues in the N-terminal direction and/or the C-terminal direction to the pep7 region mutation in the oligopeptide are the naturally occurring amino acid sequences in the N-terminal direction and/or the C-terminal direction to a mammalian epimorphin pep7 region or comprise substitutions, in particular conservative substitution(s) and/or, deletion(s), and/or addition(s) and/or insertions to naturally occurring amino acid sequences in the N-terminal direction and/or the C-terminal direction to a mammalian epimorphin pep7 region, as long as the oligopeptide maintains at least one biological activity. In some examples, an oligopeptide is soluble in aqueous medium such as saline or water. In other examples, the oligopeptide is capable of dimerizing under suitable conditions.

The present invention encompasses isolated oligopeptides of between about 5 and about 104 amino acid residues in length exhibiting hair growth promoting activity comprising the following amino acid sequence:
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Ala, Tyr, Thr, Pro, Phe, Val, Gly, Leu, Ile or Met, or is deleted from said oligopeptide;
X2 is an amino acid residue of Ile, Gly, Asn, Thr, Val, Ser, Phe, Leu, Ala, Pro, Cys, or Met, or is deleted from said oligopeptide;
X3 is an amino acid residue of Glu, Lys, Gln, Arg, Ala, Val, Trp, Cys, or Asp;
X4 is an amino acid residue of Gln, Pro, Glu, Thr, Arg, Ser, His, Cys, or Lys;
X5 is an amino acid residue of Ser, Trp, Phe, Thr, Cys, Tyr, Pro, Ala, Gly, Val, Leu, Ile, or Met;
X6 is an amino acid residue Cys; a reactive substance-bound Cys or a reactive substance-bound Lys; and
X7 is an amino acid residue of Asp, Glu, His, Ser, Ala, Gly, Asn, Tyr, Arg, or Leu, or is deleted from said oligopeptide, with the proviso that the oligopeptide is not identical to SEQ ID NO:1 or SEQ ID NO:2 and is not identical to oligopeptide Ser-Ile-Glu-Gln-Ser-Cys-Asp or Ser-Ile-Glu-Gln-Ser-Cys.

The present invention also encompasses isolated oligopeptides of between about 5 and about 104 amino acid residues in length having hair growth promoting activity comprising the following amino acid sequence:
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Tyr, Thr, or Pro, or is deleted from said oligopeptide;
X2 is an amino acid residue of Ile, Asn, Thr, or Ser, or is deleted from said oligopeptide;
X3 is an amino acid residue of Glu, Ala, Trp, or Asp;
X4 is an amino acid residue of Gln;
X5 is an amino acid residue of Ser, Cys, or Tyr;
X6 is an amino acid residue of Cys; a reactive substance-bound Cys or a reactive substance-bound Lys; and
X7 is an amino acid residue of Asp, Ala, Gly, or Leu, or is deleted from said oligopeptide, with the proviso that the oligopeptide is not SEQ ID NO:2, and is not identical to oligopeptide Ser-Ile-Glu-Gln-Ser-Cys-Asp or Ser-Ile-Glu-Gln-Ser-Cys.

The present invention also encompasses isolated oligopeptides of between about 7 and about 100 amino acid residues in length exhibiting hair growth promoting activity comprising the following amino acid sequence:
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Ala, Tyr, Thr, Pro, Phe, Val, Gly, Leu, Ile or Met, or is deleted from said oligopeptide;
X2 is an amino acid residue of Ile, Gly, Asn, Thr, Val, Ser, Phe, Leu, Ala, Pro, Cys, or Met, or is deleted from said oligopeptide;
X3 is an amino acid residue of Glu, Lys, Gln, Arg, Ala, Val, Trp, Cys, or Asp;
X4 is an amino acid residue of Gln, Pro, Glu, Thr, Arg, Ser, His, Cys, or Lys;
X5 is an amino acid residue of Ser, Trp, Phe, Thr, Cys, Tyr, Pro, Ala, Gly, Val, Leu, Ile, or Met;
X6 is an amino acid residue of Cys; and
X7 is an amino acid residue of Asp, Glu, His, Ser, Ala, Gly, Asn, Tyr, Arg, or Leu, or is deleted from said oligopeptide, with the proviso that the oligopeptide is not identical to oligopeptide Ser-Ile-Glu-Gln-Ser-Cys-Asp or Ser-Ile-Glu-Gln-Ser-Cys.

The present invention also encompasses isolated oligopeptides of between about 7 and about 100 amino acid residues in length exhibiting hair growth promoting activity comprising the following formula:
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Tyr, Thr, or Pro, or is deleted from said oligopeptide;
X2 is an amino acid residue of Ile, Asn, Thr, or Ser, or is deleted from said oligopeptide;
X3 is an amino acid residue of Glu, Ala, Trp, or Asp;
X4 is an amino acid residue of Gln;
X5 is an amino acid residue of Ser, Cys, or Tyr;
X6 is an amino acid residue of Cys; and
X7 is an amino acid residue of Asp, Ala, Gly, or Leu, or is deleted from said oligopeptide, with the proviso that the oligopeptide is not identical to oligopeptide Ser-Ile-Glu-Gln-Ser-Cys-Asp or Ser-Ile-Glu-Gln-Ser-Cys.

The present invention also encompasses isolated oligopeptides comprising the following amino acid sequence that exhibit hair growth promoting activity:

T/Y-S/N-E-Q-S-C-A. (SEQ ID NO:3).

Isolated oligopeptides comprising an amino acid sequence as shown in any one of SEQ ID NO: 3-124 disclosed herein are encompassed within the present invention. In some embodiments, an oligopeptide will consist of an amino acid sequence as shown in any one of SEQ ID NO: 3-124 and in other embodiments, an oligopeptide will comprise an amino acid sequence as shown in any one of SEQ ID NO: 3-124. As used herein, as "isolated" oligopeptide refers to an oligopeptide that is removed from at least one component with which it is naturally associated.

The present invention further encompasses the following oligopeptides that exhibit hair growth promoting activity:
oligopeptides comprising 7 to 100 amino acid residues in length, which comprise an amino acid sequence wherein 1 to 3 amino acid residues are substituted in the amino acid sequence Ser-Ile-Glu-Gln-Ser-Cys-Asp wherein the amino acid residue to be substituted is other than Cys or the amino acid residue to be substituted is other than the third to sixth amino acid residues Glu-Gln-Ser-Cys;
oligopeptides comprising 5 to 100 amino acid residues in length, which comprise an amino acid sequence wherein 0 to 2 amino acid residues are substituted in the amino acid sequence represented by Glu-Gln-Ser-Cys-Asp; wherein the amino acid residue to be substituted is other than Cys or the amino acid residue to be substituted is other than the third to sixth amino acid residues Glu-Gln-Ser-Cys;
oligopeptides comprising 7 to 100 amino acid residues in length, wherein the first Ser is substituted with a hydrophobic amino acid residue or a neutral amino acid residue in the amino acid sequence of Ser-Ile-Glu-Gln-Ser-Cys-Asp;
oligopeptides comprising 7 to 100 amino acid residues in length wherein the first Ser is substituted with Ala, Tyr, Thr, Pro, Phe, Val or Gly in the amino acid sequence of Ser-Ile-Glu-Gln-Ser-Cys-Asp;
oligopeptides comprising 7 to 100 amino acid residues in length wherein the second Ile is substituted with a neutral amino acid residue or a hydrophobic amino acid residue in the amino acid sequence of Ser-Ile-Glu-Gln-Ser-Cys-Asp;
oligopeptides comprising 7 to 100 amino acid residues in length wherein the second Ile is substituted with Gly, Asn, Thr, Val, Ser, Phe or Leu in the amino acid sequence of Ser-Ile-Glu-Gln-Ser-Cys-Asp;
oligopeptides comprising 7 to 100 amino acid residues in length wherein the fifth Ser is substituted with a neutral amino acid residue or a hydrophobic amino acid residue in the amino acid sequence of Ser-Ile-Glu-Gln-Ser-Cys-Asp;
oligopeptides comprising 7 to 100 amino acid residues in length wherein the fifth Ser is substituted with Trp, Phe, Thr, Cys, Tyr, Pro or Ala in the amino acid sequence of Ser-Ile-Glu-Gln-Ser-Cys-Asp;
oligopeptides comprising 7 to 100 amino acid residues in length wherein the seventh Asp is substituted with a hydrophilic amino acid residue, Gly, Ala or Leu in the amino acid sequence of Ser-Ile-Glu-Gln-Ser-Cys-Asp;
oligopeptides comprising 7 to 100 amino acid residues in length wherein the seventh Asp is substituted with Glu, His, Ser, Ala, Gly, Asn, Tyr or Leu in the amino acid sequence of Ser-Ile-Glu-Gln-Ser-Cys-Asp;
oligopeptides comprising 7 to 100 amino acid residues in length wherein the third Glu is substituted with Lys, Gly, Gln, Arg, Ala, Val Asp or Trp in the amino acid sequence of Ser-Ile-Glu-Gln-Ser-Cys-Asp;
oligopeptides comprising 7 to 100 amino acid residues in length wherein the fourth Gln is substituted with Pro, Glu, Thr, Arg, Ser, His or Lys in the amino acid sequence of Ser-Ile-Glu-Gln-Ser-Cys-Asp;
oligopeptides comprising 7 to 100 amino acid residues in length wherein the first Ser is substituted with Thr or Tyr, the second Ile is substituted with Ser, Asn or Thr, the third Glu is substituted with Ala, Asp or Trp, the fifth Ser is substituted with Cys or Tyr, and/or the seventh Asp is substituted with Gly, Ala or Leu in the amino acid sequence of Ser-Ile-Glu-Gln-Ser-Cys-Asp;
oligopeptides comprising 7 to 100 amino acid residues in length wherein 1 to 3 amino acid residues other than the third to sixth amino acid residues Glu-Gln-Ser-Cys are substituted in the amino acid sequence of Ser-Ile-Glu-Gln-Ser-Cys-Asp, the first Ser is substituted with Thr or Tyr, the second Ile is substituted with Ser, Asn or Thr, and/or the seventh Asp is substituted with Gly, Ala or Leu;
oligopeptides comprising 7 to 100 amino acid residues in length which comprises at least an amino acid sequence wherein 1 to 3 amino acid residues are substituted in the amino acid sequence represented by Ser-Ile-Glu-Gln-Cys-Ser-Asp wherein the amino acid residue to be substituted is other than Cys or other than the third to sixth amino acid residues Glu-Gln-Cys-Ser;
oligopeptides comprising 5 to 100 amino acid residues in length which comprises an amino acid sequence wherein 0 to 2 amino acid residues are substituted in the amino acid sequence represented by Glu-Gln-Cys-Ser-Asp; wherein the amino acid residue to be substituted is other than Cys or other than the third to sixth amino acid residues Glu-Gln-Cys-Ser;
oligopeptides comprising 7 to 100 amino acid residues in length wherein the first Ser is substituted with a hydrophobic amino acid residue or a neutral amino acid residue in the amino acid sequence of Ser-Ile-Glu-Gln-Cys-Ser-Asp;
oligopeptides comprising 7 to 100 amino acid residues in length wherein the first Ser is substituted with Ala, Tyr, Thr, Pro, Phe, Val or Gly in the amino acid sequence of Ser-Ile-Glu-Gln-Cys-Ser-Asp;
oligopeptides comprising 7 to 100 amino acid residues in length wherein the second Ile is substituted with a neutral amino acid residue or a hydrophobic amino acid residue in the amino acid sequence of Ser-Ile-Glu-Gln-Cys-Ser-Asp;
oligopeptides comprising 7 to 100 amino acid residues in length wherein the second Ile is substituted with Gly, Asn, Thr, Val, Ser, Phe or Leu in the amino acid sequence of Ser-Ile-Glu-Gln-Cys-Ser-Asp;
oligopeptides comprising 7 to 100 amino acid residues in length wherein the sixth Ser is substituted with a neutral amino acid residue or a hydrophobic amino acid residue in the amino acid sequence of Ser-Ile-Glu-Gln-Cys-Ser-Asp;
oligopeptides comprising 7 to 100 amino acid residues in length wherein the sixth Ser is substituted with Trp, Phe, Thr, Cys, Tyr, Pro or Ala in the amino acid sequence of Ser-Ile-Glu-Gln-Cys-Ser-Asp;
oligopeptides comprising 7 to 100 amino acid residues in length wherein the seventh Asp is substituted with a hydrophilic amino acid residue, Gly, Ala or Leu in the amino acid sequence of Ser-Ile-Glu-Gln-Cys-Ser-Asp;
oligopeptides comprising 7 to 100 amino acid residues in length wherein the seventh Asp is substituted with Glu, His, Ser, Ala, Gly, Asn, Tyr or Leu in the amino acid sequence of Ser-Ile-Glu-Gln-Cys-Ser-Asp;
oligopeptides comprising 7 to 100 amino acid residues in length wherein the third Glu is substituted with Lys, Gly, Gln, Arg, Ala, Val, Asp or Trp in the amino acid sequence of Ser-Ile-Glu-Gln-Cys-Ser-Asp;
oligopeptides comprising 7 to 100 amino acid residues in length wherein the fourth Gln is substituted with Pro, Glu, Thr, Arg, Ser, His or Lys in the amino acid sequence of Ser-Ile-Glu-Gln-Cys-Ser-Asp;
oligopeptides comprising 7 to 100 amino acid residues in length wherein the first Ser is substituted with Thr or Tyr, the second Ile is substituted with Ser, Asn or Thr, the third Glu is substituted with Ala, Asp or Trp, the sixth Ser is substituted with Cys or Tyr, and/or the seventh Asp is substituted with Gly, Ala or Leu in the amino acid sequence of Ser-Ile-Glu-Gln-Cys-Ser-Asp;
oligopeptides comprising 7 to 100 amino acid residues in length wherein 1 to 3 amino acid residues other than the third to sixth amino acid residues Glu-Gln-Cys-Ser are substituted in the amino acid sequence of Ser-Ile-Glu-Gln-Cys-Ser-Asp, the first Ser is substituted with Thr or Tyr, the second Ile is substituted with Ser, Asn or Thr, and/or the seventh Asp is substituted with Gly, Ala or Leu;
oligopeptides comprising 7 to 100 amino acid residues in length which comprises at least an amino acid sequence wherein 1 to 3 amino acid residues are substituted in the amino acid sequence represented by Ser-Ile-Glu-Cys-Gln-Ser-Asp wherein the amino acid residue to be substituted is other than Cys or wherein the amino acid residue to be substituted is other than the third to sixth amino acid residues Glu-Cys-Gln-Ser;
oligopeptides comprising 5 to 100 amino acid residues in length which comprise an amino acid sequence wherein 0 to 2 amino acid residues are substituted in the amino acid sequence represented by Glu-Cys-Gln-Ser-Asp; wherein the amino acid residue to be substituted is other than Cys or wherein the amino acid residue to be substituted is other than the third to sixth amino acid residues Glu-Cys-Gln-Ser;
oligopeptides comprising 7 to 100 amino acid residues in length wherein the first Ser is substituted with a hydrophobic amino acid residue or a neutral amino acid residue in the amino acid sequence of Ser-Ile-Glu-Cys-Gln-Ser-Asp;
oligopeptides comprising 7 to 100 amino acid residues in length wherein the first Ser is substituted with Ala, Tyr, Thr, Pro, Phe, Val or Gly in the amino acid sequence of Ser-Ile-Glu-Cys-Gln-Ser-Asp;
oligopeptides comprising 7 to 100 amino acid residues in length wherein the second Ile is substituted with a neutral amino acid residue or a hydrophobic amino acid residue in the amino acid sequence of Ser-Ile-Glu-Cys-Gln-Ser-Asp;
oligopeptides comprising 7 to 100 amino acid residues in length wherein the second Ile is substituted with Gly, Asn, Thr, Val, Ser, Phe or Leu in the amino acid sequence of Ser-Ile-Glu-Cys-Gln-Ser-Asp;
oligopeptides comprising 7 to 100 amino acid residues in length wherein the sixth Ser is substituted with a neutral amino acid residue or a hydrophobic amino acid residue in the amino acid sequence of Ser-Ile-Glu-Cys-Gln-Ser-Asp;
oligopeptides comprising 7 to 100 amino acid residues in length wherein the sixth Ser is substituted with Trp, Phe, Thr, Cys, Tyr, Pro or Ala in the amino acid sequence of Ser-Ile-Glu-Cys-Gln-Ser-Asp;
oligopeptides comprising 7 to 100 amino acid residues in length wherein the seventh Asp is substituted with a hydrophilic amino acid residue, Gly, Ala or Leu in the amino acid sequence of Ser-Ile-Glu-Cys-Gln-Ser-Asp;
oligopeptide comprising 7 to 100 amino acid residues in length wherein the seventh Asp is substituted with Glu, His, Ser, Ala, Gly, Asn, Tyr or Leu in the amino acid sequence of Ser-Ile-Glu-Cys-Gln-Ser-Asp;
oligopeptides comprising 7 to 100 amino acid residues in length wherein the third Glu is substituted with Lys, Gly, Gln, Arg, Ala, Val, Asp or Trp in the amino acid sequence of Ser-Ile-Glu-Cys-Gln-Ser-Asp;
oligopeptides comprising 7 to 100 amino acid residues in length wherein the fifth Gln is substituted with Pro, Glu, Thr, Arg, Ser, His or Lys in the amino acid sequence of Ser-Ile-Glu-Cys-Gln-Ser-Asp;
oligopeptides comprising 7 to 100 amino acid residues in length wherein the first Ser is substituted with Thr or Tyr, the second Ile is substituted with Ser, Asn or Thr, the third Glu is substituted with Ala, Asp or Trp, the sixth Ser is substituted with Cys or Tyr, and/or the seventh Asp is substituted with Gly, Ala or Leu in the amino acid sequence of Ser-Ile-Glu-Cys-Gln-Ser-Asp;
oligopeptides comprising 7 to 100 amino acid residues in length wherein 1 to 3 amino acid residues other than the third to sixth amino acid residues Glu-Cys-Gln-Ser are substituted in the amino acid sequence of Ser-Ile-Glu-Cys-Gln-Ser-Asp, the first Ser is substituted with Thr or Tyr, the second Ile is substituted with Ser, Asn or Thr, and/or the seventh Asp is substituted with Gly, Ala or Leu;
oligopeptides comprising 7 to 100 amino acid residues in length which comprises at least an amino acid sequence wherein 1 to 3 amino acid residues are substituted in the amino acid sequence represented by Ser-Ile-Cys-Glu-Gln-Ser-Asp wherein the amino acid residue to be substituted is other than Cys or other than the third to sixth amino acid residues Cys-Glu-Gln-Ser;
oligopeptides comprising 5 to 100 amino acid residues in length which comprises an amino acid sequence wherein 0 to 2 amino acid residues are substituted in the amino acid sequence represented by Cys-Glu-Gln-Ser-Asp, wherein the amino acid residue to be substituted is other than Cys or other than the third to sixth amino acid residues Cys-Glu-Gln-Ser;
oligopeptides comprising 7 to 100 amino acid residues in length wherein the first Ser is substituted with a hydrophobic amino acid residue or a neutral amino acid residue in the amino acid sequence of Ser-Ile-Cys-Glu-Gln-Ser-Asp;
oligopeptides comprising 7 to 100 amino acid residues in length wherein the first Ser is substituted with Ala, Tyr, Thr, Pro, Phe, Val or Gly in the amino acid sequence of Ser-Ile-Cys-Glu-Gln-Ser-Asp;
oligopeptides comprising 7 to 100 amino acid residues in length wherein the second Ile is substituted with a neutral amino acid residue or a hydrophobic amino acid residue in the amino acid sequence of Ser-Ile-Cys-Glu-Gln-Ser-Asp;
oligopeptides comprising 7 to 100 amino acid residues in length wherein the second Ile is substituted with Gly, Asn, Thr, Val, Ser, Phe or Leu in the amino acid sequence of Ser-Ile-Cys-Glu-Gln-Ser-Asp;
oligopeptides comprising 7 to 100 amino acid residues in length wherein the sixth Ser is substituted with a neutral amino acid residue or a hydrophobic amino acid residue in the amino acid sequence of Ser-Ile-Cys-Glu-Gln-Ser-Asp;
oligopeptides comprising 7 to 100 amino acid residues in length wherein the sixth Ser is substituted with Trp, Phe, Thr, Cys, Tyr, Pro or Ala in the amino acid sequence of Ser-Ile-Cys-Glu-Gln-Ser-Asp;
oligopeptides comprising 7 to 100 amino acid residues in length wherein the seventh Asp is substituted with a hydrophilic amino acid residue, Gly, Ala or Leu in the amino acid sequence of Ser-Ile-Cys-Glu-Gln-Ser-Asp;
oligopeptides comprising 7 to 100 amino acid residues in length wherein the seventh Asp is substituted with Glu, His, Ser, Ala, Gly, Asn, Tyr or Leu in the amino acid sequence of Ser-Ile-Cys-Glu-Gln-Ser-Asp;
oligopeptides comprising 7 to 100 amino acid residues in length wherein the fourth Glu is substituted with Lys, Gly, Gln, Arg, Ala, Val, Asp or Trp in the amino acid sequence of Ser-Ile-Cys-Glu-Gln-Ser-Asp;
oligopeptides comprising 7 to 100 amino acid residues in length wherein the fifth Gln is substituted with Pro, Glu, Thr, Arg, Ser, His or Lys in the amino acid sequence of Ser-Ile-Cys-Glu-Gln-Ser-Asp;
oligopeptides comprising 7 to 100 amino acid residues in length wherein the first Ser is substituted with Thr or Tyr, the second Ile is substituted with Ser, Asn or Thr, the fourth Glu is substituted with Ala, Asp or Trp, the sixth Ser is substituted with Cys or Tyr, and/or the seventh Asp is substituted with Gly, Ala or Leu in the amino acid sequence of Ser-Ile-Cys-Glu-Gln-Ser-Asp;
oligopeptides comprising 7 to 100 amino acid residues in length wherein 1 to 3 amino acid residues other than the third to sixth amino acid residues Cys-Glu-Gln-Ser are substituted in the amino acid sequence of Ser-Ile-Cys-Glu-Gln-Ser-Asp, the first Ser is substituted with Thr or Tyr, the second Ile is substituted with Ser, Asn or Thr, and/or the seventh Asp is substituted with Gly, Ala or Leu;
oligopeptides comprising 5 to 100 amino acid residues in length which comprises at least an amino acid sequence wherein 1 to 3 amino acid residues are substituted in the amino acid sequence represented by Glu-Gln-Ser-Cys-Asp;
oligopeptides comprising 5 to 100 amino acid residues in length which comprises at least an amino acid sequence wherein 1 to 3 amino acid residues are substituted in the amino acid sequence represented by Glu-Gln-Cys-Ser-Asp;
oligopeptides comprising 5 to 100 amino acid residues in length which comprises at least an amino acid sequence wherein 1 to 3 amino acid residues are substituted in the amino acid sequence represented by Glu-Cys-Gln-Ser-Asp; and
oligopeptides comprising 5 to 100 amino acid residues in length which comprises at least an amino acid sequence wherein 1 to 3 amino acid residues are substituted in the amino acid sequence represented by Cys-Glu-Gln-Ser-Asp.

Without wanting to be bound to theory, it is theorized that to obtain an oligopeptide having amino acid substitutions which will have a similar morphogenesis activity, such as for example, a similar hair growth promoting activity, with the unsubstituted oligopeptide, it is preferred that the newly added amino acid residue in the pep7 region of epimorphin has a similar property to the deleted amino acid residue. Specifically, 5 types (7 positions in total, that is the 7 underlined positions of S I E Q S C D Q D E) of amino acid residues of Ser (at 2 positions), Ile, Glu, Gln (at 2 positions) and Asp can be substituted in the amino acid sequence of a pep7 region from murine epimorphin. Among them, Ser (serine) can be substituted with Thr (threonine) belonging to hydroxyamino acids; Ile (isoleucine) can be substituted with Gly (glycine), Ala (alanine), Val(valine) or Leu(leucine) which are aliphatic amino acids; Glu (glutamic acid) can be substituted with Asp (aspartic acid) which is acidic amino acids; Gln (glutamine) can be substituted with Asn (asparagine) which is an amide; and Asp (aspartic acid) can be substituted with Glu (glutamic acid) which is acidic amino acids. These are preferred examples of substitution, and the amino acid can be substituted with any other amino acid as long as at least one biological activity is maintained.

Oligopeptides comprising the following amino acid sequences demonstrated hair growth promoting activity as measured in the assay described herein in Example 7, which measures the amount of mAb27 antigen in skin in vitro. Ser-Ile-Glu-Gln-Cys-Ser-Asp (SEQ ID NO:4); Ser-Ile-Glu-Cys-Gln-Ser-Asp (SEQ ID NO:5); Ser-Ile-Cys-Glu-Gln-Ser-Asp (SEQ ID NO:6); Tyr-Asn-Glu-Gln-Ser-Cys-Asp (SEQ ID NO:7); Thr-Ser-Asp-Gln-Cys-Cys-Asp (SEQ ID NO:8); Ser-Ile-Glu-Gln-Ser-Cys-Gly (SEQ ID NO:9); Ser-Ser-Ala-Gln-Ser-Cys-Leu (SEQ ID NO:10); Tyr-Ile-Glu-Gln-Tyr-Cys-Asp (SEQ ID NO:11); Thr-Ile-Trp-Gln-Ser-Cys-Asp (SEQ ID NO:12); Thr-Thr-Glu-Gln-Ser-Cys-Ala (SEQ ID NO:13); Pro-Ser-Glu-Gln-Ser-Cys-Ala (SEQ ID NO:14); and

Ser-Asn-Glu-Gln-Ser-Cys-Ala (SEQ ID NO:15).

Additional oligopeptides comprising amino acid sequences demonstrating hair growth promoting activity are shown in Table 1 below in one letter amino acid code (wherein the designation ? refers to an undetermined amino acid and * refers to a stop codon).

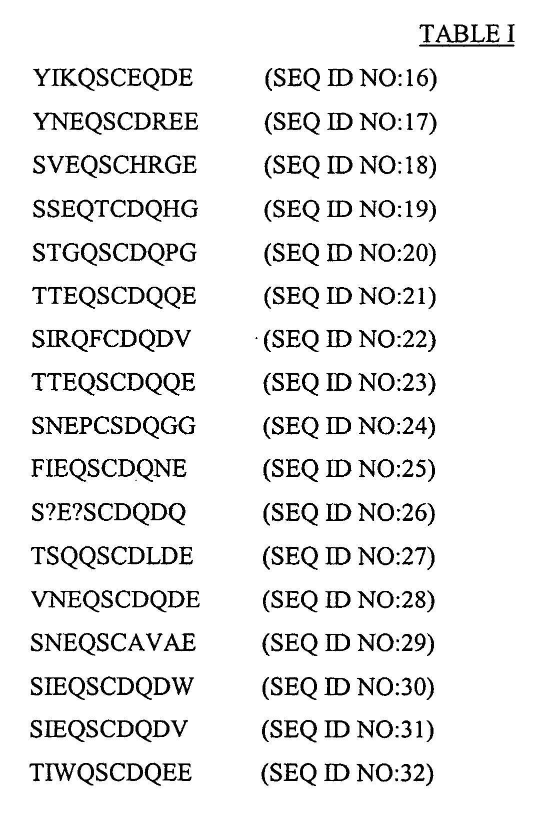

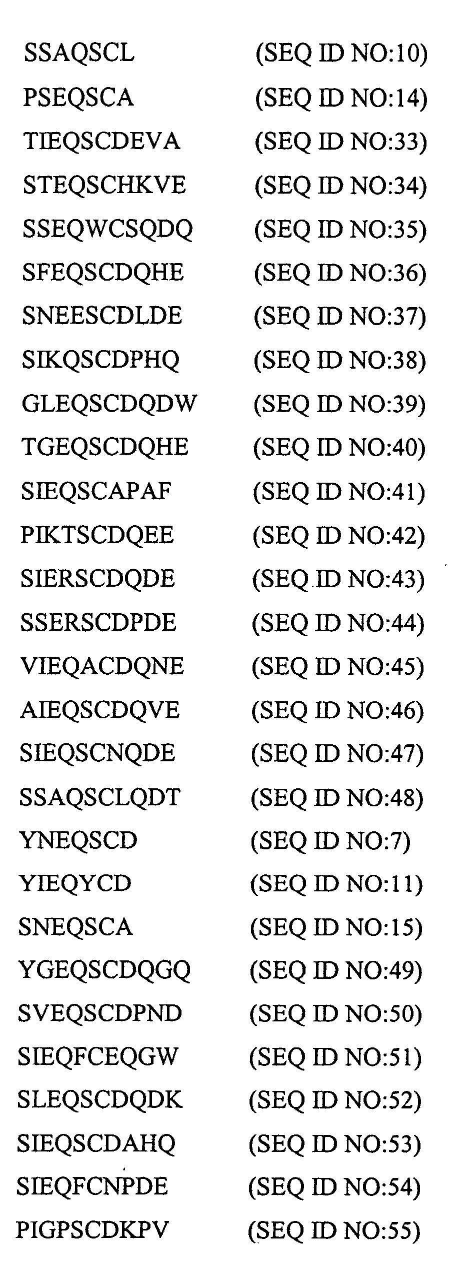

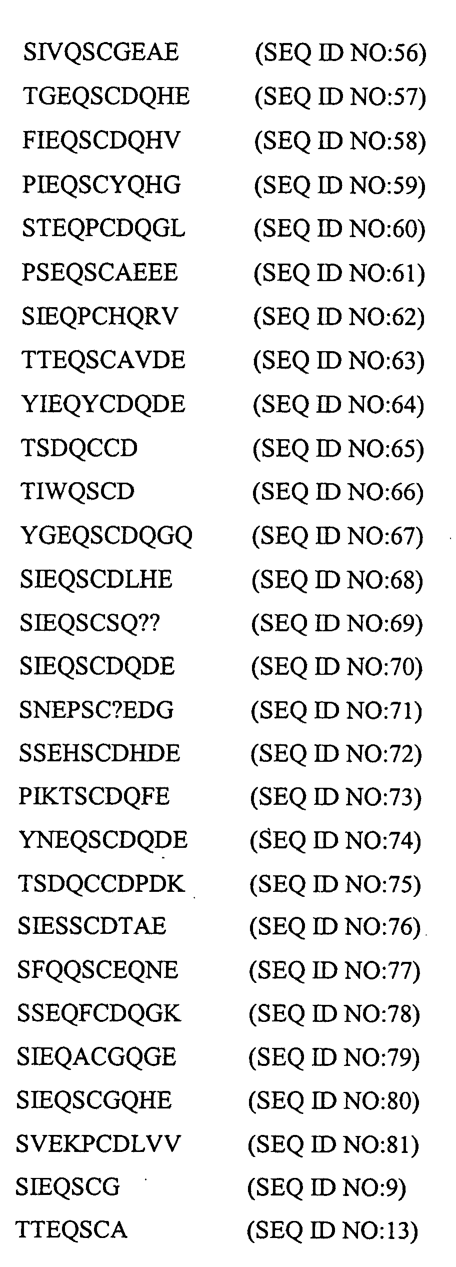

Additional oligopeptides comprising the following amino acid sequences demonstrating hair growth promoting activity are encompassed withing the present invention. Ser-Ile-Glu-Gln-Cys-Ser-Asp-Gln  (SEQ ID NO:82); Ser-Ile-Glu-Cys-Gln-Ser-Asp-Gln (SEQ ID NO:83); Ser-Ile-Cys-Glu-Gln-Ser-Asp-Gln (SEQ ID NO:84); Thr-Ser-Glu-Gln-Ser-Cys-Ala (SEQ ID NO:85); Thr-Asn-Glu-Gln-Ser-Cys-Ala (SEQ ID NO:86); Tyr-Ser-Glu-Gln-Ser-Cys-Ala (SEQ ID NO:87); Tyr-Asn-Glu-Gln-Ser-Cys-Ala (SEQ ID NO:88); Thr-Ser-Glu-Gln-Cys-Ser-Ala (SEQ ID NO:89); Thr-Asn-Glu-Gln-Cys-Ser-Ala (SEQ ID NO:90); Tyr-Ser-Glu-Gln-Cys-Ser-Ala (SEQ ID NO:91); Tyr-Asn-Glu-Gln-Cys-Ser-Ala  (SEQ ID NO:92); Thr-Ser-Glu-Cys-Gln-Ser-Ala  (SEQ ID NO:93); Thr-Asn-Glu-Cys-Gln-Ser-Ala (SEQ ID NO:94); Tyr-Ser-Glu-Cys-Gln-Ser-Ala (SEQ ID NO:95); Tyr-Asn-Glu-Cys-Gln-Ser-Ala  (SEQ ID NO:96); Thr-Ser-Cys-Glu-Gln-Ser-Ala (SEQ ID NO:97); Thr-Asn-Cys-Glu-Gln-Ser-Ala (SEQ ID NO:98); Tyr-Ser-Cys-Glu-Gln-Ser-Ala (SEQ ID NO:99); Tyr-Asn-Cys-Glu-Gln-Ser-Ala (SEQ ID NO:100); Glu-Gln-Ser-Cys-Asp (SEQ ID NO:101); Glu Gln-Cys-Ser-Asp (SEQ ID NO:102); Glu-Cys-Gln-Ser-Asp (SEQ ID NO:103); and

Cys Glu Gln Ser Asp  (SEQ ID NO:104).

Additional oligopeptides comprising the following amino acid sequences demonstrated hair growth promoting activity and are encompassed within the present invention. Ser-Ile-Glu-Gln-Xaa-Ser-Asp-Gln  (SEQ ID NO:105); Ser-Ile-Glu-Xaa-Gln-Ser-Asp-Gln (SEQ ID NO:106); and Ser-Ile-Xaa-Glu-Gln-Ser-Asp-Gln  (SEQ ID NO:107); wherein Xaa represents a reactive substance-bound Cys or a reactive substance-bound Lys.

Table II below provides a characterization of the structure of oligopeptides having hair growth providing activity. The amino acid SIEQSCD is the pep7 region of murine epimorphin.

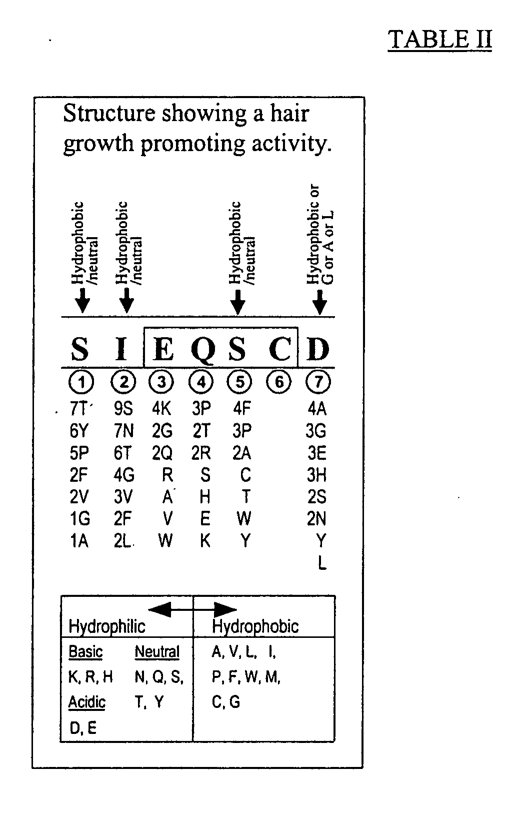

In order to produce oligopeptides in *E. coli,* an oligopeptide library was prepared. Table III provides information about the oligopeptide library prepared. In Table III, the design value of the library is represented by the lower "theory value". The actually prepared oligopeptides are represented by the upper "appearance" value.

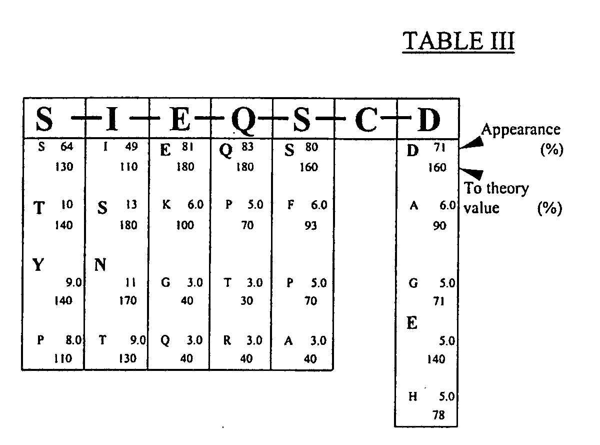

In some examples an oligopeptide of the present invention is about 5 to about 104 amino acid residues in length, and in other examples about 5 to about 100 amino acid residues in length and in additional examples, an oligopeptide is about 7 to about 100 amino acid residues in length. In yet further examples, an oligopeptide is about 7 to about 104 amino acid residues in length. In additional examples, an oligopeptide is about 5 to about 40 amino acid residues in length, about 6 to about 30 amino acid residues in length, about 7 to about 20 amino acid residues in length, about 7 to about 15 amino acid residues in length, about 7 to about 12 amino acid residues in length, about 7 to about 10 amino acid residues in length, about 8 to about 20 amino acid residues in length, about 8 to about 15 amino acid residues in length, about 8 to about 12 amino acid residues in length, and about 8 to about 10 amino acid residues in length.

In some embodiments, an oligopeptide of the present invention is at least 5, at least 6, at least 7, at least 8, at least 9, at least 10, at least 11, at least 12, at least 13, at least 14; at least 15, at least 16, is at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, is at least 37, at least 38, at least 39, at least 40, at least 41, at least 42, at least 43, at least 44, at least 45, at least 46, at least 47, at least 48, at least 49 or at least about 50 amino acid residues in length. In other embodiments, an oligopeptide of the present invention is up to at least 50, up to at least 55, up to at least 60, up to at least 65, up to at least 70, up to at least 75, up to at least 80, up to at least 85, up to at least 90, up to at least 95, up to at least 100, up to at least 101, up to at least 102, up to at least 103 or up to at least 104 amino acid residues in length. It may be desirable when using larger oligopeptides to include agents that enhance or facilitate endermic absorption of oligopeptides. Such agents include, for example, agents that enhance transdermal penetration and/or delivery. Such agents are known in the art and described herein.

In some examples, the lower limit of the length of an oligopeptide of the invention is at least 5, at least 6, at least 7, at least 8, at least 9, at least 10, at least 11, at least 12, at least 13, at least 14, or at least 15 amino acid residues in length, and the upper limit is up to at least 15, up to at least 20, up to at least 25, up to at least 30, up to at least 35, up to at least 40, up to at least 45, or up to at least 50 amino acid residues in length, wherein the upper limit and lower limit are selected independently.

The type of amino acid residue in an oligopeptide of the present invention is not particularly limited, and may be any of natural type amino acid residue, non-natural type amino acid residue, or derivatives thereof. The amino acid may be L-amino acid, D-amino acid or a mixture thereof. The type of the amino acid may be any of α-amino acid, β-amino acid, γ-amino acid or δ-amino acid. α-amino acid, which is a natural type amino acid, is preferred.

The non-natural type amino acid used herein covers all of the amino acids other than 20 types of the natural type amino acids which constitute a natural protein (Gly, L-Ala, L-Val, L-Leu, L-Ile, L-Ser, L-Thr, L-Asp, L-Glu, L-Asn, L-Gln, L-Lys, L-Arg, L-Cys, L-Met, L-Phe, L-Tyr, L-Trp, L-His, L-Pro). Specific examples include (1) non-natural type amino acid wherein an atom in a natural type amino acid is substituted with another substance, (2) an optical isomer as to a side chain of natural type amino acid, (3) non-natural type amino acid obtained by introducing a substituent into a side chain of natural type amino acid, and (4) non-natural type amino acid obtained by substituting the side chain of natural type amino acid to alter hydrophobic property, reactivity, charge, size of the molecule, hydrogen bonding ability and the like.

An oligopeptide may be in free form, or may be provided as an acid addition salt or base addition salt.

Oligopeptide amino acid residues may comprise naturally occurring amino acid residues, non-naturally occurring amino acid residues, or a mixture of naturally occurring and non-naturally occurring amino acid residues. In some embodiments, an oligopeptide may be modified, such as for example, by adding a reactive substance, such as a cross-linking agent, such that the oligopeptide is capable of dimerizing or polymerizing with an oligopeptide. The present invention encompasses oligopeptide monomers, oligopeptide monomers having a reactive substance bound, and oligopeptide polymers, such as dimers, including homodimers (an oligopeptide dimerized with an identical oligopeptide) and heterodimers, (an oligopeptide dimerized with a different oligopeptide). In some embodiments, a reactive substance is bound to a Cys or a Lys within the oligopeptide and in further embodiments, a reactive substance is bound to a Cys or a Lys within the pep 7 region of an oligopeptide.

Biological activity of an oligopeptide can be measured, for example, by assaying the morphogenesis-accelerating activity against MDCKII cells derived from kidney, as described in EP 1008603A1. Briefly, MDCKII cells are added to a mixture containing a test polypeptide, collagen and appropriate culture conditions that produce a collagen gel and cells are allowed to incubate under appropriate conditions. A positive result is indicated by the formation of tubular structures in a three dimensional manner in the collagen gel.

Japanese Application publication HEI 6-25,295 also describes an assay for measuring morphogenesis activity of epithelial tissues. U.S. Pat. No. 5,726,298 describes an assay for determining epithelial growth. Briefly, pulmonary epithelium tissue isolated from fetal mice is subjected to three dimensional cultivation on nucleopore membrane and continued growth of the tubular form of the pulmonary epithelium is measured in the presence of a test molecule, such as an oligopeptide of the present invention.

Biological activity of an oligopeptide can also be measured by, for example, measuring the ability of an oligopeptide to promote hair growth or exhibit hair growth promoting activity such as by, stimulating hair generation, inducing the formation of a greater number of hair strands and/or increasing the diameter of the hair strand and/or lengthening the hair strand and/or preventing, retarding or arresting the process of hair loss, and/or by inducing new follicle in number or size or both, an assay for which is disclosed herein in Examples 7 and 8 and/or inducing hair follicle cell proliferation as measured by the *in vitro* assay disclosed in U.S. Patent No. 5,616,471.

More specifically, a hair growth promoting activity can be evaluated by detecting or measuring the monoclonal antibody, or antibody fragment thereof, specific for the 220 kDa antigen of epithelial new follicle which reacts with the skin tissue, and thereby measuring the amount of 220 kDa antigen expressed on epithelial new follicles in response to administration of an oligopeptide of the present invention.

One of ordinary skill in the art can easily ascertain that the oligopeptides of the present invention have a hair growth promoting activity by test methods, such as the one described in detail in the Examples of the present specification or alterations or modifications to said test method. Other test methods include those disclosed in U.S. Patent No. 5,616,471.

Examples of such test methods disclosed herein include but are not limited to the below.
(1) C3H and C57BL/6 mice are known to have sustained telogen for about 50 days from the 45th day after the birth to around the 95th day. Their hair cycle is easily judged based on the skin color changes, i.e., from pink in telogen to gray or black in anagen. "Anagen" refers to the active stage of a hair follicle and "telogen" refers to the resting phase of a hair follicle. A hair growth promoting activity can be evaluated by using this mice and evaluating whether or not the administration of the test substance promotes the transition from telogen to anagen.
(2) A hair growth promoting activity can be evaluated by using a monoclonal antibody, or a fragment thereof (for example, a monoclonal antibody produced by the hybridoma having an accession number FERM P-18578) which specifically recognizes an antigen present in epithelial new follicles (for example, about 220 kDa antigen). Specifically, skin tissue from an organism is cultured in the presence of a test substance, such as an oligopeptide encompassed within the present invention, and the skin tissue is collected, and reacted with a monoclonal antibody produced by the hybridoma having an accession number FERM P-18578, or a fragment thereof. A hair growth promoting activity can be evaluated by detecting or measuring the monoclonal antibody or a fragment thereof which reacted with the skin tissue, and thereby measuring the amount of the antigen expressed on epithelial new follicles. An increase in the amount of epithelial new follicles is correlated with hair growth promoting activity in humans.

Conditions suitable for dimerization as used herein refer to conditions whereby an oligopeptide of the invention binds another oligopeptide. In some embodiments, an oligopeptide binds covalently with another oligopeptide. Dimerization conditions are known in the art and include conditions whereby a sulfhydryl group of a cysteine residue in an oligopeptide forms a covalent bond with a sulfhydryl group of a cysteine residue or amino group of a lysine residue of an oligopeptide.

A reactive substance-bound Cys or reactive substance-bound Lys refers to a Cys or Lys amino acid residue that has bound to it a substance, such as for example, a cross-linking agent, that is capable of reacting and binding with a functional group such a -SH group or -NH2 group. In some embodiments, the reactive substance is a cross-linking agent. In other embodiments, the reactive substance is a bifunctional cross-linking agent. In some embodiments of the invention, an oligopeptide exhibiting morphogenic activity, such as for example, exhibiting hair growth promoting activity is in the form of a monomer and, in particular, a monomer that is capable of dimerization under suitable conditions. In other embodiments of the invention, an oligopeptide is in the form of reactive-substance bound monomer that is capable of dimerization under suitable conditions. In additional embodiments of the invention, an oligopeptide is in the form of a polymer, such as, dimers such as homodimers or heterodimers, and trimers.

Accordingly, the present invention provides oligopeptide polymers having morphogenic promoting activity, such as, hair growth promoting activity, comprising cross-linked oligopeptides wherein at least one oligopeptide of said oligopeptide polymer comprises between about 5 and about 104 amino acid residues in length and comprises the following amino acid sequence:
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Ala, Tyr, Thr, Pro, Phe, Val, Gly, Leu, Ile or Met, or is deleted from said oligopeptide;
X2 is an amino acid residue of Ile, Gly, Asn, Thr, Val, Ser, Phe, Leu, Ala, Pro, Cys, or Met, or is deleted from said oligopeptide;
X3 is an amino acid residue of Glu, Lys, Gln, Arg, Ala, Val, Trp, Cys, or Asp;
X4 is an amino acid residue of Gln, Pro, Glu, Thr, Arg, Ser, His, Cys, or Lys;
X5 is an amino acid residue of Ser, Trp, Phe, Thr, Cys, Tyr, Pro, Ala, Gly, Val, Leu, Ile, or Met;
X6 is an amino acid residue Cys; a reactive substance-bound Cys or a reactive substance-bound Lys; and
X7 is an amino acid residue of Asp, Glu, His, Ser, Ala, Gly, Asn, Tyr, Arg, or Leu, or is deleted from said oligopeptide, with the proviso that the polymer is not a homopolymer of SEQ ID NO: 1 or SEQ ID NO:2 and is not a homopolymer of oligopeptide Ser-Ile-Glu-Gln-Ser-Cys-Asp or Ser-Ile-Glu-Gln-Ser-Cys.

In some embodiments, an oligopeptide polymer having morphogenic promoting activity comprises at least one oligopeptide comprising between about 5 to about 104 amino acid residues having the following amino acid sequence:
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Tyr, Thr, or Pro, or is deleted from said oligopeptide;
X2 is an amino acid residue of Ile, Asn, Thr, or Ser, or is deleted from said oligopeptide;
X3 is an amino acid residue of Glu, Ala, Trp, or Asp;
X4 is an amino acid residue of Gln;
X5 is an amino acid residue of Ser, Cys, or Tyr;
X6 is an amino acid residue of Cys; a reactive substance-bound Cys or a reactive substance-bound Lys; and
X7 is an amino acid residue of Asp, Ala, Gly, or Leu, or is deleted from said oligopeptide, with the proviso that the oligopeptide polymer is not a homopolymer of SEQ ID NO:2 and is not a homopolymer of oligopeptide Ser-Ile-Glu-Gln-Ser-Cys-Asp or Ser-Ile-Glu-Gln-Ser-Cys.

The present invention also provides oligopeptide polymers having morphogenic promoting activity, such as, hair growth promoting activity comprising cross-linked oligopeptides wherein at least one oligopeptide of said oligopeptide polymer comprises between about 7 and about 100 amino acid residues in length and comprises the following amino acid sequence:
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Ala, Tyr, Thr, Pro, Phe, Val, Gly, Ala, Leu, Ile or Met, or is deleted from said oligopeptide;
X2 is an amino acid residue of Ile, Gly, Asn, Thr, Val, Ser, Phe, Leu, Ala, Pro, Cys, or Met, or is deleted from said oligopeptide;
X3 is an amino acid residue of Glu, Lys, Gln, Arg, Ala, Val, Trp, Cys, or Asp;
X4 is an amino acid residue of Gln, Pro, Glu, Thr, Arg, Ser, His, Cys, or Lys;
X5 is an amino acid residue of Ser, Trp, Phe, Thr, Cys, Tyr, Pro, Ala, Gly, Val, Leu, Ile, or Met;
X6 is an amino acid residue of Cys; and X7 is an amino acid residue of Asp, Glu, His, Ser, Ala, Gly, Asn, Tyr, Arg, or Leu, or is deleted from said oligopeptide, with the proviso that the oligopeptide polymer is not a homopolymer of oligopeptide Ser-Ile-Glu-Gln-Ser-Cys-Asp or Ser-Ile-Glu-Gln-Ser-Cys.

The present invention also provides oligopeptide polymers having morphogenic promoting activity, such as, hair growth promoting activity comprising cross-linked oligopeptides wherein at least one oligopeptide of said oligopeptide polymer comprises between about 7 and about 100 amino acid residues in length and comprises the following amino acid sequence:
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Tyr, Thr, or Pro, or is deleted from said oligopeptide;
X2 is an amino acid residue of Ile, Asn, Thr, or Ser, or is deleted from said oligopeptide;
X3 is an amino acid residue of Glu, Ala, Trp, or Asp;
X4 is an amino acid residue of Gln;
X5 is an amino acid residue of Ser, Cys, or Tyr;
X6 is an amino acid residue of Cys; and
X7 is an amino acid residue of Asp, Ala, Gly, or Leu, or is deleted from said oligopeptide, with the proviso that the oligopeptide polymer is not a homopolymer of oligopeptide Ser-Ile-Glu-Gln-Ser-Cys-Asp or Ser-Ile-Glu-Gln-Ser-Cys.

In additional examples, the oligopeptide polymer comprises an oligopeptide of the present invention that comprises any one of oligopeptides SEQ ID NO:3 to SEQ ID NO:124 alone, that is as a polymer of a single oligopeptide (that is, a homopolymer) or as a polymer of a mixture of oligopeptides of the present invention (heteropolymer). In some examples of the present invention, an oligopeptide polymer is a homodimer; heterodimer; homotrimer; or heterotrimer.

The present invention encompasses modified oligopeptides. The term "modified" in the present invention includes chemical modification and biological modification. Examples of the modification include introduction of a functional group such as alkylation, esterification, halogenation, and amination, or conversion of a functional group such as oxidation, reduction, addition, and elimination, or introduction of a sugar compound (a monosaccharide, disaccharide, oligosaccharide, or polysaccharide) or a lipid compound, phosphorylation, biotinylation. However, the modifications are not limited to these examples. Accordingly, the present invention provides modified oligopeptides and methods of promoting hair growth in a mammalian subject comprising administering a composition comprising a modified oligopeptide to a mammalian subject in need of hair growth in an amount effective to promote hair growth in said mammal.

An example of a modified oligopeptide includes a biotinylated oligopeptide, and a more preferred example includes an oligopeptide of which N-terminal is bound by biotin with or without a spacer. In the above modified oligopeptide, an appropriate chemical modification may be added to the biotin as long as the desirable physiological activity is maintained. A method for producing the biotinylated oligopeptide is specifically shown in the Examples of the present specification. In order to introduce biotin to the N-terminal by means of a spacer having an appropriate length, for example, NHS-Biotin or NHS-LC-Biotin (available from Pierce) can be used.

Another preferred example of a modified oligopeptide includes an oligopeptide dimerized by sulfhydryl group of cysteine residue therein. The dimerization reaction spontaneously proceeds under air atmosphere to form a dimer. However, not all of the oligopeptides form a dimer. The dimerized oligopeptide may be a homodimer oligopeptide obtained by cross-linking the same oligopeptides, or a heterodimer oligopeptide obtained by cross-linking different 2 types of oligopeptides. A composition comprising an oligopeptide or oligopeptides of the present invention for use in promoting morphogenesis activity, such as, hair growth, may in the form of a mixture of the same or different monomers and in particular monomers that are capable of dimerizing under suitable conditions, monomer(s) having a reactive substance bound thereto and polymers, such as dimers.

A polymer of the present invention, such as a homodimer or heterodimer, can be obtained by cross-linking any oligopeptides of the present invention mentioned above, including modified oligopeptides, by a cross-linking agent.

The cross-linking agents used in the present invention preferably include a bifunctional cross-linking agent which can activate sulfhydryl group of cysteine residue in an oligopeptide and form a covalent bond with sulfhydryl group of cysteine residue in another oligopeptide.

Examples of the bifunctional cross-linking agent which can be used in the present invention include bismaleimide compounds, such as compounds wherein N atom of a maleimide group is bound to both ends of a lower alkyl group (for example, C1-C10, preferably C1-C8 alkyl group) optionally having a substituent such as a hydroxyl group. Examples of bismaleimide compounds include 1,4-bismaleimidyl-2,3-dihydroxybutane, 1,6-bismaleimidehexane, bismaleimidethane, and 1,4-bismaleimidebutane.

Further, monomer oligopeptides wherein a cross-linking agent is bound to a cysteine residue in any of the aforementioned oligopeptide (including modified oligopeptides), and methods of using such monomers to promote hair growth in a mammalian subject also fall within the scope of the present invention.

Furthermore, in addition to dimer oligopeptides, polymer oligopeptides such as trimers or more fall within the scope of the present invention. For example, the present invention encompasses polymer oligopeptides, such as trimers or more which are obtained by using a cross-linking agent which can cross-link 3 or more peptides.

The above oligopeptides (including modified oligopeptides) may be in free form, or may be provided as acid addition salts or base addition salts. Examples of the acid addition salts include mineral acid salts such as hydrochloride, sulfate, nitrate, and phosphate; organic acid salts such as para-toluenesulfonate, methanesulfonate, citrate, oxalate, maleate, and tartrate. Examples of the base addition salts include metal salts such as sodium salt, a potassium salt, a calcium salt, and a magnesium salt; an ammonium salt; organic ammonium salts such as a methyl ammonium salt, and a trimethyl ammonium salt. The oligopeptide may form a salt with amino acids such as glycine, or may form a counter ion in the molecule.

Further, these oligopeptides or salts thereof may exist in a form of a hydrate or a solvate. The above oligopeptides have plural asymmetric carbon atoms. Although the stereochemistry of each asymmetric carbon atoms is not limited, it is preferable that the amino acid reside is L-amino acid. Stereoisomers such as optical isomers or diastereomers based on the asymmetric carbon atoms, any mixtures of the stereoisomers, and racemates fall within the scope of the present invention.

The oligopeptide of the present invention can be synthesized by a conventional chemical technique for peptide synthesis, such as solid phase or liquid phase method. There are various kinds of references about protective groups for amino groups or the like and condensation agents for a condensation reaction in the field of peptide synthesis, and accordingly, these references can be referred to for the synthesis. In the solid phase method, commercially available various peptide synthesizers can be utilized. The synthesis can be efficiently carried out by performing protection and deprotection of functional groups as necessary. As for a method for introducing and removing a protective group, for example, Protective Groups in Organic Synthesis, T.W.Greene, John Wiley & Sons, Inc. 1981 and the like can be referred to.

By applying biological methods known to those of skilled in the art such as a gene expression procedure, a desired oligopeptide can be obtained by constructing a recombinant vector containing a DNA sequence encoding the above oligopeptide, preparing a microorganism (transformant) transformed by the vector, and separating optionally and purifying the oligopeptide from culture of the transformant. The method for producing the oligopeptides is not limited to these chemical and biological methods. Methods for producing modified oligopeptides including chemical modification and biological modification are well known to one of ordinary skill in the art, and any methods can be used.

The present invention also provides host cells comprising (i.e., transformed with) the nucleic acid encoding the oligopeptides described herein. Both prokaryotic and eukaryotic host cells can be used as long as sequences requisite for maintenance in that host, such as appropriate replication origin(s), are present. For convenience, selectable markers are also provided. Host systems are known in the art. Prokaryotic host cells include bacterial cells, for example, *E.coil*, *B.subtilis*, and mycobacteria. Among eukaryotic host cells are yeast, insect, avian, plant, *C.elegans* (or nematode) and mammalian host. Examples of fungi (including yeast) host cells are *S.cerevisiae*, *Kluyveromyces lactis (K.lactis),* species of *Candida* including *C.albicans* and *C.glabrata, Aspergillus nidulans, Schizosaccharomyces pombe* (*S.pombe*), *Pichia pastoris*, and *Yarrowia lipolytica.* Examples of mammalian cells are cultured Chinese hamster ovary (CHO) cells and African green monkey cells. *Xenopus laevis* oocytes, or other cells of amphibian origin, may also be used.

### Uses of the oligopeptides of the present invention

Oligopeptides of the present invention are useful as an active ingredient of a medicament or pharmaceutical composition useful for the treatment and/or amelioration of symptoms of diseases or disorders of abnormal morphogenesis. The oligopeptides of the present invention can be used to induce morphogenesis, induce revascularization effect, induce regeneration effect, induce cardiovascular regeneration, and induce endothelial cell growth. The oligopeptides of the present invention can be used for the treatment of and/or amelioration of symptoms of, for example, burns or wounds or to promote hair growth or prevent hair loss. In some examples, the compositions are useful for hair growth promotion or prevention of hair loss. Accordingly, the present invention provides a composition comprising an oligopeptide of the present invention and a pharmaceutically acceptable excipient. In some examples, a composition useful for promoting hair growth further comprises an agent that enhances endermic absorption, such as a transdermal penetration enhancer and/or a transdermal delivery agent. Such agents are known in the art and described herein. The term "medicament" or "pharmaceutical composition" is used interchangeable herein. A "pharmaceutical composition" as used herein is used in the broadest sense and encompasses compositions comprising a morphogenesis promoting agent and in some examples, a hair growth promoting agent. Morphogenesis agents are used for the amelioration of the symptoms of as well as the therapeutic treatment of diseases or disorders of a mammal, including a human. Hair growth compositions are sometimes classified as a quasi-drug or cosmetic, as well as a medicament or pharmaceutical composition. The administration subject and pharmacological effect of the medicaments of the present invention, and the diseases and/or disorders to be treated by the medicament of the present invention are specifically mentioned below. "Amelioration" as used herein means the prevention, reduction or palliation of a state. Oligopeptides of the present invention and compositions comprising an oligopeptide may be used to provide revascularization effect, regeneration promoting effect, cardiovascular regeneration effect, an inductive effect on vascular endothelial cell, and the like, and are useful for the therapy and amelioration of the symptoms of prevention of chronic obstructive arteriosclerosis, Buerger's disease, sever angina pectoris, arteriosclerosis and the like. (*Exp. Cell. Res.,* 1996, Jan 10, 222(1):189-98). Oligopeptides of the present invention may be involved in morphogenesis of pancreatic endothelium, and compositions comprising an oligopeptide may be useful for the therapy and amelioration of the symptoms of diabetes and the like (*J. Cell. Biol*., 2001, Mar 5;152(5):911-22). The oligopeptides of the present invention may be involved in formation (regeneration) of liver, and compositions comprising an oligopeptide may be useful for the therapy and amelioration of the symptoms of liver metabolism failure (*Biochem. Biophys. Res. Commun*., 1998, Sep 18; 250(2):486-90). Oligopeptides of the present invention may be involved in formation of bone and tooth, and therefore compositions comprising an oligopeptide may be useful for the therapy and amelioration of the symptoms of periodontics, fracture, bone tumor, bone deficiency, and osteoporosis (*Arch. Oral. Biol*., 1995, Feb;40(2):161-4). Oligopeptides of the present invention may be involved in lung branching morphogenesis and pulmonary fibrosis, and compositions comprising an oligopeptide may be useful for the therapy and amelioration of the symptoms of lung diseases (*Biochem. Biophys. Res. Commun.,* 1997, May 19; 234(2):522 and *Am. J. Respir. Cell. Mol. Biol*., 2000, Aug;23(2):168-74). The oligopeptides of the present invention may be involved in crypt-villus morphogenesis, and compositions comprising an oligopeptide may be useful for the therapy and amelioration of the symptoms of intestine diseases (*Am. J. Physiol.,* 1998, Jul; 275(1 Pt1):G114-24). Oligopeptides of the present invention may be involved in maintenance of muscle structure, and compositions comprising an oligopeptide may be useful for the therapy and amelioration of the symptoms of muscular dystrophy and the like (*Histochem. J*., 1998, Dec;30(12):903-8). The oligopeptides of the present invention may be involved in morphogenesis of gallbladder epithelium, and compositions comprising an oligopeptide may be useful for the therapy and amelioration of the symptoms of gallbladder diseases *(Cell. Tissue. Res.,* 2000, May; 300(2):331-44). The oligopeptides of the present invention may be involved in mammary luminal morphogenesis, and compositions comprising an oligopeptide may be useful for the therapy and amelioration of the symptoms of mammary diseases (*J. Cell. Biol.,* 2001, May 14; 153(4):785-94). Most preferably among these, the oligopeptides of the present invention can be used as a hair growth promoting agent.

A composition of the present invention comprising an oligopeptide is administered to a subject in amounts effective to promote morphogenesis. In some examples, the compositions of the present invention comprising an oligopeptide are administered to a subject in amounts effective to promote hair growth. The subject may be experiencing hair loss and/or at risk for hair loss.

Accordingly, the present invention provides a method for promoting hair growth in a mammalian subject experiencing hair loss or at risk for hair loss comprising administering a composition comprising an oligopeptide of the present invention in an amount effective to promote hair growth in said mammalian subject.

As a medicament or pharmaceutical composition of the invention, one or more oligopeptides selected from among the oligopeptides disclosed herein, or their physiologically acceptable salt, may be used. The oligopeptides of the present invention may be in the form of a monomer and, in particular, a monomer that is capable of dimerization under conditions suitable for dimerization, a monomer having a reactive substance bound and as a polymer, such as a dimer, including a homodimer and heterodimer or trimer, including a homotrimer and a heterotrimer. Generally, however, it is preferable to prepare and administer a pharmaceutical composition comprising one or more of the above oligopeptides as an active ingredient by using one or more pharmaceutically acceptable pharmaceutical additives. A hair growth promoting agent containing one or more of the aforementioned oligopeptides as an active ingredient can be applied in a form of external preparations such as a cream, a spray, a coating solution, and a patch. The agent can be administered to a target site directly in a form of an injection. It is possible to provide the agent in any form suitable for the purpose of use as a hair growth promoting agent.

For example, the above oligopeptides as an active ingredient may be added to a shampoo or a rinse, or the above oligopeptide can be encapsulated into a liposome to manufacture a preparation. The composition in the aforementioned forms also falls within the scope of the present invention. In order to achieve an effective transdermal absorption of the oligopeptides of the invention through the keratin layer of skin, it is preferable to add an appropriate detergent, lipid-soluble substance of the like in a cream.

Agents that enhance transdermal penetration and transdermal delivery are described in, for example, U.S. Patent Publication, 2002 0048558A1; U.S. Pat. No. 6,376,557; U.S. Patent No. 6,333,057; U. S. Patent No. 6,358,541; and U.S. Patent No. 6,299,900 and include for example, laurocapram and laurocapram derivatives, such as 1-alkylazacycloheptan-2-specified in U.S. Pat. No. 5,196,410, and oleic acid and its ester derivatives, such as methyl, ethyl, propyl, isopropyl, butyl, vinyl and glycerylmonooleate, and those given in U.S. Pat. No. 5,082,866, particularly dodecyl (N,N-dimethylamino) acetate and dodecyl (N,N-dimethylamino) propionate and in U.S. Pat. No. 4,861,764, particularly 2-n-nonyl-1-3-dioxolane. Other known dermal penetration enhancers include adapalene, tretinoin, retinalaldehyde, tazarotene, salicylic acid, azelaic acid and glycolic acid. Additional dermal penetrating agents include ethoxydiglycol, ethanol, Tween.RTM.80, and lecithin organogel.

For topical administration in mammals, preferably humans, the subject compositions may be provided as a wide variety of product types including, but are not limited to, lotions, creams, gels, sticks, sprays, ointments and pastes. These product types may comprise several types of formulations including, but not limited to solutions, emulsions, gels, solids, and liposomes.

Compositions useful for topical administration of the compositions of the present invention formulated as solutions typically include a pharmaceutically-acceptable aqueous or organic solvent. The terms "pharmaceutically-acceptable organic solvent" refer to a solvent which is capable of having an oligopeptide of the present invention dispersed or dissolved therein, and of possessing acceptable safety properties (e.g., irritation and sensitization characteristics). Examples of suitable organic solvents include: propylene glycol, polyethylene glycol (200-600), polypropylene glycol (425-2025), glycerol, 1,2,4-butanetriol, sorbitol esters, 1,2,6-hexanetriol, ethanol, isopropanol, butanediol, and mixture thereof.

An oligopeptide of the present invention can be dissolved in PBS at approximately 1mg/ml. An oligopeptide dimerized with a cross-linking agent can be dissolved at approximately 0.9mg/ml. The solubility can be determined by measuring absorbance of peptide solution. In the case of monomer, the solubility can be determined by (A₂₁₅-A₂₂₅) x 144 (µg/ml) (Waddell, 1956). In the case of dimer, the value obtained by this calculation may be divided by 1.3.

If the topical compositions useful in the subject invention are formulated as an aerosol and applied to the skin as a spray-on, a propellant is added to a solution composition. Examples of propellants useful herein include, but are not limited to, the chlorinated, fluorinated or chloro-flunorinated lower molecular weight hydrocarbons.

Topical compositions useful in the subject invention may be formulated as a solution comprising an emollient. As used herein, "emollients" refer to materials used for the prevention or relief of dryness, as well as for the protection of the skin. A wide variety of suitable emollients are known and may be used herein.

Another type of product that may be formulated from a composition comprising an oligopeptide is a cream and a lotion. Lotions and creams can be formulated as emulsions as well as solutions.

Yet another type of product that may be formulated from a composition of the present invention is an ointment. An ointment may comprise a simple base of animal or vegetable oils or semi-solid hydrocarbons (oleaginous). Ointments may also comprise absorption ointment bases which absorb water to form emulsions. Ointments carriers may also be water soluble.

Another type of formulation is an emulsion. Emulsifiers may be nonionic, anionic or cationic and examples of emulsifiers are described in, for example, U.S. Patent Nos. 3,755,560 and 4,421,769.

Single emulsions for topical preparations, such as lotions and creams, of the oil-in-water type and water-in-oil type are well-known in the art. Multiphase emulsion compositions, such as the water-in-oil-in-water type, are also known, as disclosed, for example, in U.S. Patent No. 4,254,105. Triple emulsions are also useful for topical administration of the present invention and comprise an oil-in-water-in-silicone fluid emulsion as disclosed, for example in U.S. Patent No. 4,960,764.

Another emulsion useful in the topical compositions is a micro-emulsion system. For example, such system comprises from about 9% to about 15% squalane, from about 25% to about 40% silicone oil; from about 8% to about 20% of a fatty alcohol; from about 15% to about 30% of polyoxyethylene sorbitan mono-fatty acid (commercially available under the trade name TWEENS) or other nonionics; and from about 7% to about 20% water.

Liposomal formulations are also useful for the compositions comprising an oligopeptide of the present invention. Such compositions can be prepared by combining a composition comprising an oligopeptide of the present invention with a phospholipid, such as dipalmitoylphosphatidyl choline, cholesterol and water according to known methods. Epidermal lipids of suitable composition for forming liposomes may be substituted for the phospholiipid. The liposome preparation is then incorporated into one of the above topical formulations (for example, a gel or an oil-in-water emulsion) in order to produce the liposome formulation. Other compositions and pharmaceutical uses of topically applied liposomes are described in, for example, Mezei (1985) Topics in Pharmaceutical Sciences, Breimer et al., eds., Elsevier Science, New York, N.Y., pp.345-358.

The dose of a composition comprising an oligopeptide of the present invention can be selected suitably depending on the purpose of application, the form of the agent, a kind of the active ingredient and the like. For example, it is possible to determine a dose by referring to the dose specifically shown in the Examples of the present specification. For example, the dose of the active ingredient of a composition, that is, of an oligopeptide of the present invention, per day per adult is generally within the range of about 1µg/kg/day to about 10mg /kg/day, in some examples, about 10µg/kg/day to about 1 mg /kg/day, in other examples, about 100 µg/kg/day to about 500 µg/kg/day, in other examples, about 200µg/kg/day to about 400µg/kg/day. In some examples, the lower range of a dose is at least 1µg/kg/day, 10µg/kg/day, 20µg/kg/day, 30µg/kg/day, 40µg/kg/day, 50µg/kg/day, 60µg/kg/day, 70µg/kg/day, 80µg/kg/day, 90µg/kg/day, 100µg/kg/day, 150µg/kg/day, 200µg/kg/day, 250µg/kg/day, 300µg/kg/day, 350µg/kg/day, 400µg/kg/day, 450µg/kg/day, and 500µg/kg/day. In other examples, the upper range is up to 600 µg/kg/day, 700µg/kg/day, 800µg/kg/day, 900µg/kg/day, 1mg/kg/day, 2mg/kg/day, 3mg/kg/day, 4mg/kg/day, 5mg/kg/day, 6mg/kg/day, 7mg/kg/day, 8mg/kg/day, 9mg/kg/day, and 10mg/kg/day, with the lower range and upper range being selected independently.

### Antibodies

The present invention encompasses antibodies that specifically bind an oligopeptide of the present invention wherein said antibodies may be useful for the detection, quantitative determination, separation or purification of the oligopeptide by means known to those of skill in the art. Polyclonal antibodies and monoclonal antibodies, and fragments thereof, that specifically bind an oligopeptide of the present invention are encompassed within the present invention and can be made by conventional methods.

The present invention also encompasses monoclonal antibodies that specifically recognize, that is, that specifically bind, an antigen of about 220 kDa present in epithelial new follicles which is specifically expressed during the growth period of an imago or the developing period of a fetus. Such a monoclonal antibody can be used to assay for hair growth promoting activity of an oligopeptide of the present invention as described herein in the examples.

An example of such monoclonal antibodies is monoclonal antibody mAb27 which is described herein in the Examples. The hybridoma which produced monoclonal antibody mAb27 was deposited with Patent and Bio-Resource Center of National Institute of Advanced Industrial Science and Technology (Chuo-6, 1-1, Higashi 1-chome, Tsukuba-shi, Ibaraki-ken, Japan) on November 2, 2001 under the deposit number of FERM P-18578.

The term "antibody" used in the present specification encompasses polyclonal antibodies as well as monoclonal antibodies and also encompasses fragments of the antibody. According to the present invention, there is provided a fragment of a monoclonal antibody which specifically recognizes the antigen of about 220 kDa present in the epithelial new follicles. The fragment of the antibody is preferably a functional fragment, and its examples are F(ab')₂ and Fab'.

F(ab')₂ and Fab' are prepared by the treatment of immunoglobulin with protease such as pepsin or papain and are antibody fragments produced by the digestion thereof at the sites which are before and after a disulfide bond existing between two H chains in a hinge region. The term "fragment of antibody" used in the present specification shall also include protein which contains an antigen-bonded site derived from a gene encoding said antibody.

For example, when IgG1 is treated with papain, cleavage takes place at the upper stream of the disulfide bond existing between the two H chains of the hinge region to give two homologous antibody fragments where L chain comprising VL (L chain variable region) and CL (L chain constant region) and H chain fragment comprising VH (H chain variable region) and CHγ1 (γ1 region in H chain constant region) are bonded by a disulfide bond at the C terminal region. Each of those two homologous antibody fragments is called Fab'. Further, when IgG is treated with pepsin, cleavage takes place at the downstream of the disulfide bond existing between the two H chains in the hinge region to give antibody fragments which are somewhat bigger than that where the above two Fab' are connected in a hinge region. This antibody fragment is called F(ab')₂.

An antibody of the present invention may be used as an immobilized antibody, that is, immobilized on an insoluble carrier such as solid carrier, or may be used as a labeled antibody labeled with a labeling substance. All of such immobilized antibody and labeled antibody are within the scope of the present invention.

An immobilized antibody is an antibody in a state of being carried on an insoluble carrier by physical adsorption, chemical bond or the like. Such an immobilized antibody may be used for detection, quantitative determination, separation or purification of antigen (i.e., an oligopeptide of the present invention or an antigen of about 200 kDa present in epithelial new follicles) contained in a sample (such as hair, epithelial new follicles or an extract thereof). Examples of the insoluble carrier which can be used for immobilization of the antibody include (1) a container having an inner volume such as plate, test tube or tube, or beads, ball, filter, membrane and the like, each of which are made of water-insoluble substance such as plastics including polystyrene resin, polycarbonate resin, silicone resin or Nylon resin, or glass and; (2) an insoluble carrier used for affinity chromatography, such as cellulose based carrier, agarose based carrier, polyacrylamide based carrier, dextran based carrier, polystyrene based carrier, polyvinyl alcohol based carrier, polyamino acid based carrier or porous silica based carrier.

A labeled antibody means an antibody labeled with a labeling substance, and such a labeled antibody may be used for detection or quantitative determination of antigen (i.e., an antigen of about 220 kDa present in epithelial new follicles) contained in a sample (such as hair, follicles or an extract thereof). There is no particular limitation for the labeling substance used in the present invention, so far as its existence can be detected by bonding to an antibody by means of physical bonding, chemical bonding or the like. Examples of the labeling substance are enzyme, fluorescent substance, chemiluminescent substance, biotin, avidin or radioactive isotope. Specific examples include enzyme such as peroxidase, alkaline phosphatase, β-D-galactosidase, glucose oxidase, glucose-6-phosphate dehydrogenase, alcohol dehydrogenase, malic acid dehydrogenase, penicillinase, catalase, apoglucose oxidase, urease, luciferase or acetylcholine esterase; fluorescent substance such as fluorescein isothiocyanate, phycobilic protein, rare earth metal chelate, dansyl chloride or tetramethylol rhodamine isothiocyanate; radioisotope such as ³H, ¹⁴C, ¹²⁵I or ¹³¹I; biotin; avidin; and chemiluminescent substance. With regard to a method for bonding a labeling substance to an antibody, known methods such as a glutaraldehyde method, a maleimide method, a pyridyl disulfide method and a periodic acid method may be used.

Here, each of the radioisotope and fluorescent substance is able to generate a detectable signal by itself, while each of enzyme, chemiluminescent substance, biotin and avidin is unable to generate a detectable signal by itself and, therefore, a detectable signal is generated as a result of reaction with one or more other substance(s). For example, in the case of an enzyme, at least a substrate is necessary and, depending upon a method for measuring the enzymatic activity (colorimetric method, fluorescent method, bioluminescent method or chemiluminescent method), various substrates are used. In the case of biotin, it is usual that at least avidin or enzyme- bound avidin is react therewith. If necessary, various coloring substances may be used depending upon the said substrate.

### Hybridoma producing a monoclonal antibody

The present invention also provides a hybridoma which produces a monoclonal antibody that specifically recognizes an antigen of about 220 kDa present in epithelial new follicles as described herein. The monoclonal antibody of the present invention may be produced by using said hybridoma.

A process for the preparation of a hybridoma producing a monoclonal antibody of the present invention which specifically recognizes an antigen of about 220 kDa present in the epithelial new follicles, is described below.

First, a mammal is immunized using an immunogen such as protein extracted from hair collected from the skin of the growth period and/or follicles of whiskers of the growth period, whereby antibody-producing cells are prepared in the body of the animal. Although there is no particular limitation for the type of the mammal, the examples generally include mouse, rat, cattle, rabbit, goat and sheep, preferably rodents such as mouse, rat and rabbit, and more preferably, mouse or rat. Examples of the mouse are mouse of an A/J strain, a BALB/C strain, a DBA/2 strain, a C57BL/6 strain, a C3H/He strain, an SJL strain, an NZB strain or a CBA/JNCrj strain. Mouse of a BALB/C strain is preferred since the cell strain derived from myeloma of the same strain is established at the time of the preparation of hybridoma.

In the present invention, protein extracted from hair collected from the skin of the growth period and/or follicles of whiskers of the growth period may be used as the immunogen. Any material may be used as an immunogen, so far as it contains an antigen of about 220 kDa present in the epithelial new follicles which is recognized by the monoclonal antibody of the present invention.

Before immunization, the immunogen may be mixed with an adjuvant for enhancing the immune response. Examples of the adjuvant include water-in-oil type emulsion (such as incomplete Freund adjuvant), water-in-oil-in-water type emulsion, oil-in-water type emulsion, liposome, aluminum hydroxide gel, silica adjuvant, powdery bentonite and tapioca adjuvant, as well as cell body and cell wall of BCG, *Propionibacterium acnes,* etc. and somatic component such as trehalose dicholate (TDM); lipopolysaccharide (LPS) which is an endotoxin of Gram negative bacteria and lipid A fraction; β-glucan (polysaccharide); muramyl dipeptide (MDP); bestatin; synthetic compound such as levamisole; protein or peptidic substance derived from biocomponents such as thymus hormone, liquid factor of thymus hormone and taftsin; and a mixture thereof (such as complete Freund adjuvant). Such an adjuvant is effective for augmentation or suppression of immune response depending upon administration route, dose, administration period, and the like. In addition, depending upon the type of the adjuvant, difference is found in the production of antibody in blood to antigen, induction of cellular immunity, class of immunoglobulin, and the like. Therefore, it is preferred to suitably choose the adjuvant depending upon the aimed immune response. The method for the treatment with adjuvant is known in the art.

Immunization of a mammal is carried out according to methods known by those of skill in the art. For example, an antigen is injected into a mammal either subcutaneously, intracutaneously, intravenously or intraperitoneally. Since immune responses vary depending upon the type and strain of the mammal to be immunized, an immunizing schedule is appropriately designed according to the animal to be used. Administration of antigen is repeatedly carried out for several times after the first immunization. Additional immunizations may be carried out, for example, after four weeks, six weeks and half a year from the first immunization.

After immunization, blood is collected from the mammal and the obtained blood is assayed for the presence of a hair follicle-binding activity to confirm the production of antibody against the follicles in the body of the mammal. The methods for the assay include known methods such as enzyme-linked immunosorbent assay (ELISA), radioimmunoassay (RIA) and fluorescent antibody method

After confirming the production of follicle-binding antibody, a boost (additional injection of immunogen) can be carried out so that the immunocyte capable of producing a specific antibody is made into a state suitable for cell fusion. Although there is no particular limitation for the amount of the immunogen to be administered in the boost, it is preferred to be about 4- to 5-fold of the initially immunized amount. Usually, a boost may be carried out using an emulsion of immunogen and incomplete Freund adjuvant. Route for the administration may be appropriately selected from subcutaneous, intracutaneous, intravenous, intraperitoneal administrations or the like.

After the final immunization, spleen cells are excised from the immunized mammal and subjected to a cell fusion with a cell strain derived from myeloma. In the cell fusion, it is preferred to use a cell strain having a high proliferation potency and it is preferred that a cell strain derived from myeloma has a compatibility to the mammal from which the spleen cells to be fused is derived. Examples of the cell strain derived from myeloma of mouse include P3U1, P3X63-Ag8.653, Sp2/O-Ag14, FO.1, S194/5, XXOBU.1, P3/NS1/1-Ag4-1 and the like.

Cell fusion may be carried out by methods known to those of skill in the art. Examples of the cell fusion method include a polyethylene glycol method, a method using Sendai virus, and a method using electric current. For general methods, see Antibodies, A Laboratory Manual by Ed Harlow David Lane, 1988, Cold Spring Harbor Laboratory.

The resulting fused cells may be proliferated by conditions known in the art. Desired fused cells are selected depending upon the binding ability of the produced antibody.

The ability of an antibody produced from the fused cells to bind a desired antigen is assayed according to methods known in the art. In the present invention, a cell strain of interest is cloned utilizing as a selection the ability of the fused cells to produce antibody which has a high binding ability and is specific to an antigen of epithelial new follicles. Binding ability of the antibody may be assayed by a method such as ELISA, RIA and fluorescent antibody method in the same way as in those mentioned already for the confirmation of production of antibody. Because of its simplicity and high sensitivity, ELISA is preferred.

Cloning of fused cells may be carried out by methods known in the art. The methods for cloning include a limiting dilution method, a soft agar method, and the like. Because of easily operation and high reproducibility, a limiting dilution method is preferred. In order to efficiently select useful cells from many fused cells obtained by cell fusion, it is preferred that selection of the cells is carried out from the initial stage of the cloning. In such a way, it is possible to finally select a fused cell strain which produces an antibody having a desired binding ability.

By culturing the monoclonal antibody producing cell strain selected as mentioned above in a large scale, a monoclonal antibody specific to follicles can be produced in large amount. The methods for a large-scale culturing of the monoclonal antibody-producing cell strain include *in vivo* and *in vitro* culturing. An example of the large-scale *in vivo* culturing is a method where fused cells are intraperitoneally injected into mammal to proliferate so that an antibody is produced in abdominal dropsy, that is abdominal ascites. In the *in vitro* culturing, fused cells are cultured in a medium and an antibody is produced in the medium.

The monoclonal antibody of the present invention can be purified from the abdominal dropsy obtained by a large-scale culturing or from supernatant fluid of the culture medium by methods known in the art. For the purification, an appropriate combination of DEAE anion-exchange chromatography, affinity chromatography, ammonium sulfate fractionation, PEG fractionation, ethanol fractionation, and the like may be used. The antibody of the present invention may be purified preferably to a purity of about 90%, more preferably to a purity of about 95% or, still more preferably, to a purity of about 98%.

### Methods for evaluation of hair growth promoting activity

The present invention further relates to a method for the evaluation of hair growth promoting activity characterized in that an immunoassay is carried out using a monoclonal antibody that specifically binds an antigen of about 220kDa present in the epithelial new follicles, or a fragment of the antibody. This method preferably comprises at least the following steps (a)-(c).
(a) incubating skin tissue derived from living organism in the presence of a substance to be tested, such as an oligopeptide of the present invention;
(b) recovering said skin tissue, and reacting it with a monoclonal antibody that specifically binds an antigen of about 220kDa present in the epithelial new follicles, or an antibody fragment thereof; and
(c) detecting or measuring said monoclonal antibody, or fragment thereof which reacted with the skin tissue pieces.

A method for the evaluation of hair growth promoting activity that relies on a monoclonal antibody that specifically binds an antigen of about 220kDa present in epithelial new follicles, may be any method as long as it is an assay using an antibody or, in other words, an immunoassay. That is, the present invention encompasses the use of a monoclonal antibody that specifically binds an antigen of about 220kDa present in epithelial new follicles, or a fragment of the antibody, to measure the presence of an antigen of about 220kDa present in the epithelial new follicles in immunoassays known by those of skill in the art, wherein the presence of the antigen of about 220kDa present in the epithelial new follicles is correlated with hair growth. Examples of such immunoassays include western blotting, enzyme-linked immunosorbent assay (ELISA), fluoroimmunoassay, radioimmunoassay (RIA), luminoimmunoassay, immunoenzymatic assay, immunofluorescence assay, immunoturbidimetry, latex agglutination reaction, latex turbidimetry, erythrocyte agglutination reaction and particle agglutination reaction.

There is no particular limitation for the type of the test substance which is subjected to the method for the evaluation of the present invention, and the test substance may be either oligopeptides, such as oligopeptides of the present invention, or low-molecular organic compounds. For example, there may be used an oligopeptide having a partial amino acid sequence of epimorphin.

When the method for the evaluation of hair growth promoting activity according to the present invention is carried out by means of an immunoassay using labeled antibody such as enzyme-linked immunosorbent assay (ELISA), fluoroimmunoassay, radioimmunoassay (RIA) or luminoimmunoassay, it is also possible to carry out the assay by a sandwich method or a competition method. In the case of a sandwich method, at least one of solid phase antibody and labeled antibody is the monoclonal antibody of the present invention.

With regard to the solid phase carrier, there may be used the above-mentioned carriers which are described in the present specification as specific examples for the insoluble carrier in relation to the immobilized antibody. Also with regard to the labeled substance, there may be used the above-mentioned substances which are described in the present specification in relation to the labeled antibody.

A method for the measurement may be carried out by a known method ("Immunoassay for Clinical Tests - Technique and Application", Special Issue No. 53 of *Rinsho Byori*, edited by the Japanese Society of Clinical Pathology, published by Rinsho Ryori Kankokai, 1983; "Enzyme-Linked Immunosorbent Assay" edited by Eiji Ishikawa, et al., Third Edition, published by Igaku Shoin, 1987; and "Enzyme-Linked Immunosorbent Assay", Supplementary Issue No. 31 of *Tampakushitsu, Kakusan, Koso,* edited by Tsunehiro Kitagawa, et al., published by Kyoritsu Shuppan, 1987).

For example, a solid phase antibody is reacted with a sample and a labeled antibody at the same time, or a solid phase antibody is reacted with a sample, and after being washed, it is reacted with a labeled antibody, thereby forming a complex of solid phase antibody-antigen-labeled antibody. Then, unbound labeled antibody is separated by washing, and the amount of the antigen in the sample can be measured from the amount of the bound labeled antibody. Specifically, in the case of the enzyme-linked immunosorbent assay (ELISA), the labeled enzyme is reacted with a substrate under an optimum condition and the amount of the reaction product is measured by, for example, an optical method. In the case of the fluoroimmunoassay, intensity of fluorescence by a fluorescent substance labeling is measured, and in the case of the radioimmunoassay, radiation dose by a radioactive substance labeling is measured. In the case of the luminoimmunoassay, amount of luminescence in the luminous reaction system is measured.

In the method for the detection and/or the quantitative determination according to the present invention, when the production of an immune complex aggregate by immunoturbidimetry, latex agglutination reaction, latex turbidimetry, erythrocyte agglutination reaction, particle agglutination reaction or the like is measured by measuring its transmitting light or scattering light by an optical method, or is measured visually, it is possible to use phosphate buffer, glycin buffer, Tris buffer, Good buffer or the like as a solvent, and a reaction promoting agent such as polyethylene glycol or a non-specific reaction suppressing agent may be contained therein.

When an antibody is used by sensitizing it to a solid phase carrier, there may be used particles made of the material such as polystyrene, styrene-butadiene copolymer, (meth)acrylate polymer, latex, gelatin, liposome, microcapsule, erythrocyte, silica, alumina, carbon black, metal compound, metal, ceramics or magnetic substance as a solid phase carrier.

The methods for the sensitization include known method such as physical adsorption, chemical bonding or a combination thereof. A method for the measurement may be carried out by a known method. For example, in the case of the measurement by an optical method, the sample is reacted with the antibody, or the sample is reacted with the antibody which was sensitized with a solid phase carrier. Then, the transmitting light or the scattering light is measured by an end-point method or a rate method.

When the measurement is carried out visually, the sample is reacted with the antibody sensitized with a solid phase carrier in a container such as a plate or a microtiter plate, and the state of agglutination is judged visually. Instead of measuring visually, the measurement may be carried out by an instrument such as a microplate reader.

### Kit for evaluation of hair growth promoting activity

A kit of the present invention for use in evaluating hair growth promoting activity of a test substance comprises a monoclonal antibody, or a fragment thereof, which specifically recognizes the antigen of about 220 kDa present in the epithelial new follicles or a fragment thereof. The monoclonal antibody which specifically recognizes the antigen of about 220 kDa present in the epithelial new follicles, or a fragment thereof, may be in an immobilized or labeled form.

For example, when a monoclonal antibody of the present invention which specifically recognizes the antigen of about 220 kDa present in the epithelial new follicles is used as a primary antibody, the kit of the present invention may further comprises a secondary antibody for the detection of a complex formed by the antigen-antibody reaction. The kit of the present invention may still further comprise various auxiliary agents in addition to those antibodies, so that the said kit can be utilized efficiently and easily. Examples of the auxiliary agent include those which are commonly used in a kit of reagents for immunological measurement, such as a solubilizer for dissolving the solid secondary antibody, a detergent used for washing the insoluble carrier, a substrate for measuring the enzymatic activity when an enzyme is used as a labeling substance for the antibody, and a reaction stopping agent. The kit of the present invention may also comprise the instructions for carrying out the evaluation of hair growth promoting activity.

The present invention will be explained more specifically by the following Examples. However, the scope of the invention is not limited to these examples.

### EXAMPLES

### Example 1: Preparation of library vector

A vector for presenting a peptide of about 10 amino acids on a surface of *E.coli* as a fused form with a surface protein (invasin) (Nakajima et al., *Gene,* 260,121-131(2000)) was used. All BstXI sites were removed by using mutanegenis kit (Takara), the promoter region was replaced with P_{L} promoter, and the amino acids 2-36 of thioredoxin cDNA was ligated to the C-terminal site of invasin cDNA. Further, the region of the 33th cysteine to 36th cysteine of ligated partial thioredoxin cDNA was replaced with 
TCCGGTCCGCCATCACGTTGGCTCGAGCCAGGATATTGGGGTCCGTGA
(SEQ ID NO:125)
 (to prepare a vector (designated as pALinvThio4).

A double stranded cDNA encoding HA epitope was inserted in frame to the BstXI site of this vector, and the obtained vector was introduced into *E.coli* GI724. The obtained strain was examined according to a similar method as in the first and second screening in Example 3 below. As a result, the *E.coli* strain was found to bind to anti-HA antibody. It was demonstrated that the peptide of HA epitope which was inserted as a model was presented on the surface of the cell.

### Example 2: Preparation of a library presented on the surface of E.coli

The following oligonucleotides were synthesized. 
1. 5' CTG CAG AAC CAT CAC GTT GG agT atT gaG caG agT tgT gaT caG gaT
gaG C CAG GAT ATT GGA TGC AT 3' (SEQ ID NO:126)
 
2. 5' CTG CAG AAC CAT CAC GTT GG agT atT gaG caG agT tgT gaT caG gaT
gaG C CAG GAT ATT GGA TGC AT 3' (SEQ ID NO:127)
 wherein T, G, a, t, g, and c indicates the followings.
T: T:G=17:3
G: T:G=3:17
a: A:T:G:C=7:1:1:1
t: A:T:G:C=1:7:1:1
g: A:T:G:C=1:1:7:1
c: A:T:G:C=1:1:1:7

Equal amounts of oligonucleotides 1. and 2. above were mixed, and were annealed to the primer (5' ATG CAT CCA ATA TCC TGG 3') (SEQ ID NO:128). The DNA was extended with Klenow fragment, and was cut with a restriction enzyme BstXI, and the desired band was collected by polyacrylamide gel. The collected DNAs encode a group of peptides having a partial amino acid mutation in SIEQSCDQDE (SEQ ID NO:142).

The vector pALinvThio4 was cut with BstX, and was treated with BAP (bacterial alkaline phosphatase), and was then ligated to the above-obtained library fragments.

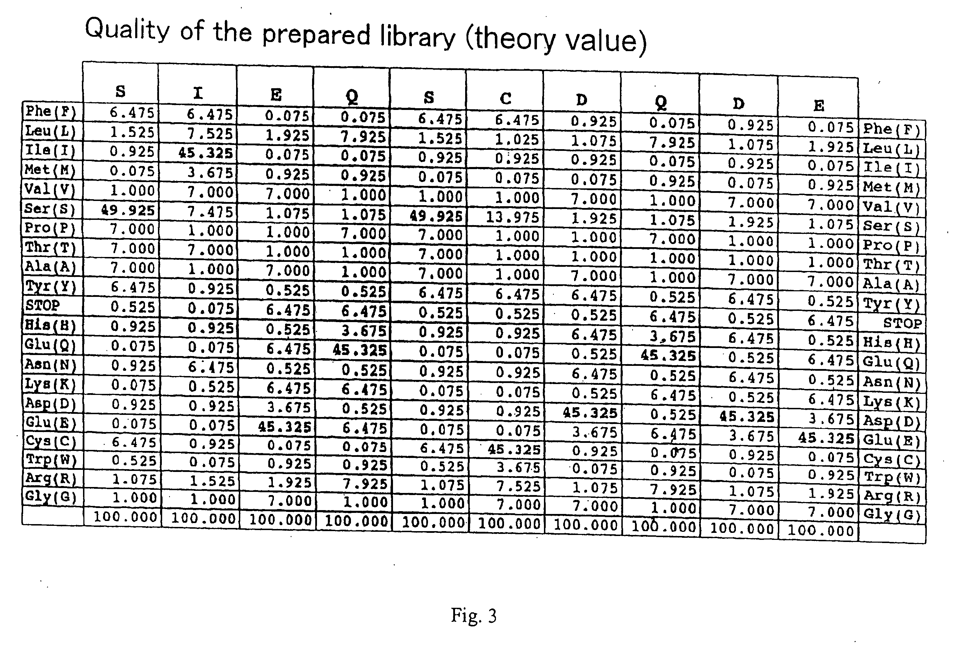

The obtained vector was transformed into electro competent cells of *E.coli* GI724 (150µl) to prepare transformants which was designated as EPM pep7-like peptide library (3.8×10⁶). Fig.3 shows theory values percentage present in the library.

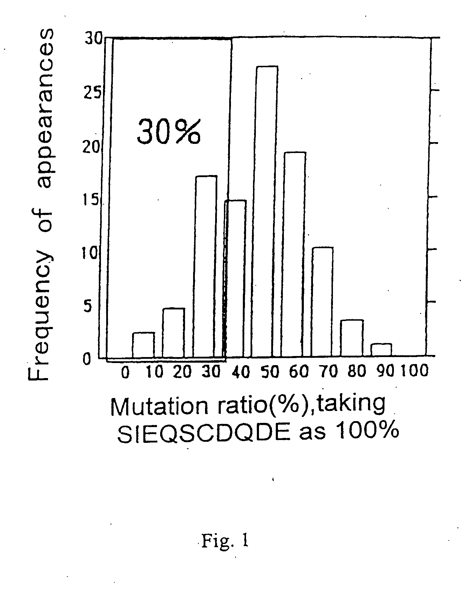

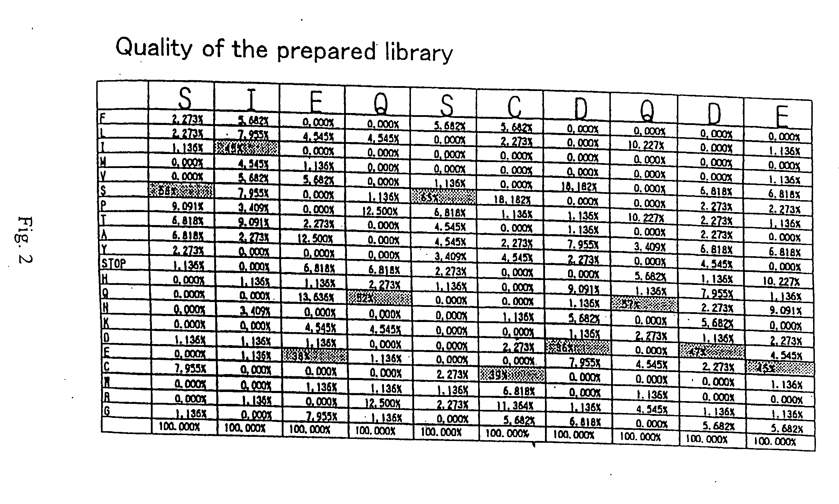

An aliquot of EPM pep7-like peptide library was applied onto LB agar plate, and 100 colonies were randomly picked up, and the plasmids were separately collected and analyzed. As a result, it was found that about 30% of the colonies presented a peptide wherein 1 to 3 amino acids were substituted in the amino acid sequence SIEQSCDQDE (which contains the murine pep7 region) (Fig.1). Further, as a result of the analysis of the contents of the above-prepared library, it was found that the position and type of the amino acids which were substituted were random (Fig.2). Fig.2 shows a result of the analysis of 100 clones.

### Example 3: First and second screening

EPM pep7-like peptide library (library size of 3.6×10⁶) was induced to be expressed for 6 hours at 30°C by addition of 100µg/ml of tryptophan, and was added to a 35mm dish which was previously coated with 10µg of rabbit anti-epimorphin antibody (polyclonal antibody which neutralizes the activity of epimorphin; Hirai et al 1998 *J.Cell.Biol*., 140:159-169) and was blocked with blocking solution (1% skim milk, 150mM NaCl, 1% α-methyl mannoside in IMC medium), and left for 1 hour. Washing solution (1% α-methyl mannoside in IMC medium) was added thereto, and the dish was washed at 100 rpm for 5 minutes. Washing was repeated 5 times. After washing, the bound clones were collected. An aliquot of the bound clones was plated for the measurement of recover ratio, and the remaining was cultured overnight at 30°C in IMC medium (obtained from Invitrogen; medium for culturing *E.coli* which does not contain tryptophan).

The first screened clones after culturing overnight were induced to be expressed by addition of tryptophan in the same way as above. The dish for second screening was coated with 10 µg of rat H12 antibody which was affinity-purified with EPM pep 7, and was blocked with a blocking solution containing 25µg of each of recombinant H1-72, H1-73Δ, H1-78, H1-79 which has no hair growth promoting activity (see below) (total 100µg) or 4 µg of each of synthesized peptides of the variable regions thereof (total 16µg). In the same way as in the first screening, the clones whose expression was induced were added to the dish, and were washed. Then, the bound clones were collected. An aliquot of the bound clones was plated for the measurement of recover ratio, and the remaining was cultured overnight at 30°C in IMC medium.

In both of the first and second screenings, cells where a vector of pALinvThio4 (containing no insert) was introduced were used as control. It was revealed from the plating that the collection ratio by panning under the above condition was less than 1/100 for the first screening, and less than 1/50 for the second screening, as compared with the case of using the library. The size of the thus obtained library was 2.5×10⁵.

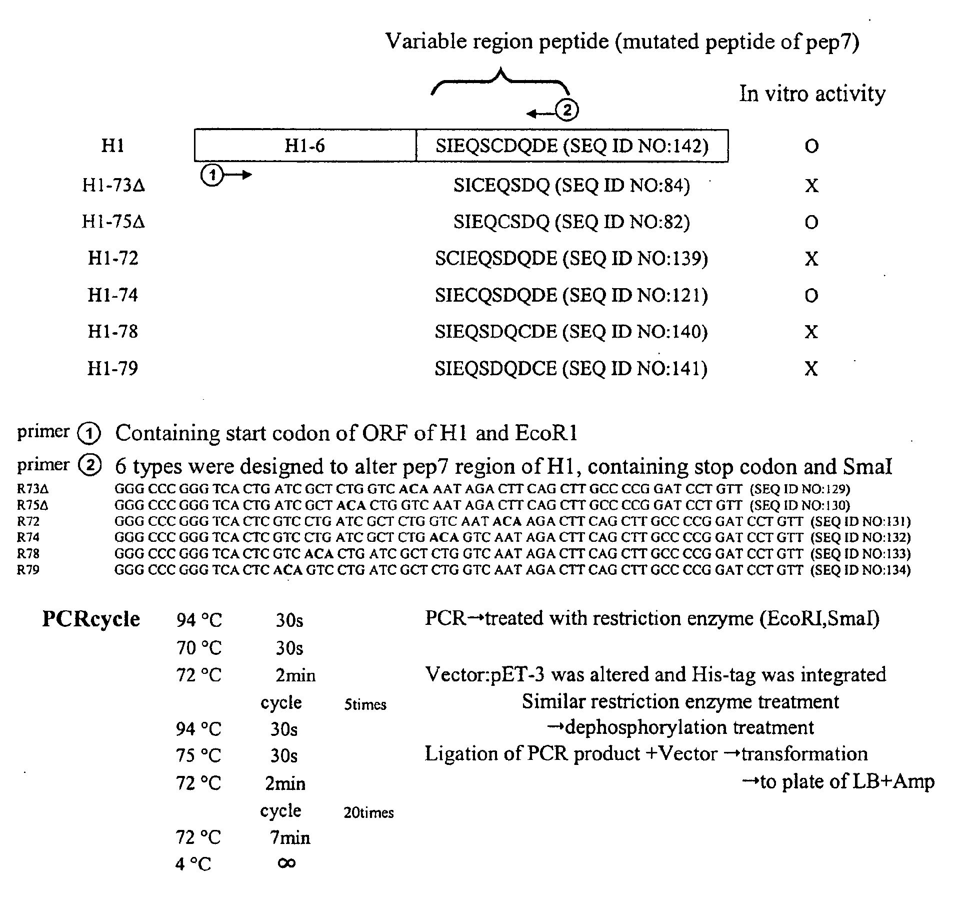

### Example 4: Recombination into a vector for large scale preparation

① A vector for large scale preparation of a high active recombinant epimorphin, SRαEPM (Hirai et al,(1994) *Eur.J.Biochem*., 225, 1133-1139), was cut with HindIII, and the ends were made blunt with T4 polymerase. The vector was subjected to self-ligation to remove HindIII restriction site.
② Mutation introducing kit (TAKARA) was used to replace the AAGCTG which was located immediately upstream to the sequence encoding SIEQSCDQDE of epimorphin which was inserted in the vector obtained in the above ①, with AAGCTT(restriction site of HindIII)
③ cDNA which encodes "NGN to C terminal of 12 fragments (*Eur.J.Biochem,* 225, 1133-1139)" immediately downstream to SIEQSCDQDE of murine epimorphin was prepared by PCR in a form having a restriction site of HindIII, and this cDNA was inserted into HindIII site of the vector prepared in the above ②. The epimorphin which was inserted into the vector is composed of 12 fragments and lacks peptide 7, and HindIII restriction site is added thereto.
④ 
AgCTTCCATCACgTTggTCTAgACCAggATATTggA (SEQ ID
NO:135)
 AggTAgTgCAACCAgATCTggTCCTATAACCTTCgA (SEQ ID NO:136) was prepared by chemically synthesizing each strand and then annealing them at 70-50°C, and was introduced into HindIII site prepared in the above ③ to convert Hind III restriction site to Hind III-BstXI-XbaI-BstXI-Hind III.
⑤ A group of plasmids were collected from *E.coli* group (overnight culture at 30°C) obtained by the second screening, and cDNAs encoding pep7-like peptide groups, which was obtained by cutting at BstXI site, were inserted into BstXI XbaI BstX site of the vector prepared in the above ④.
⑥ An aliquot of the obtained plasmid groups was cloned into *E.coli* HB101, and 15 clones were examined. As a result, it was found that all clones have a sequence which encodes epimorphin 12 fragments wherein one or more amino acids in the pep7 region are substituted with another amino acid.
⑦ The group of plasmids obtained in the above ⑤ was treated with NspV/PvuII to obtain a group of genes of epimorphin fragment containing mutated pep7. These fragments were inserted into the NspV-SmaI site of the vector pet12 (Hirai et al. *J.Cell. Biol*., 2001, 153, 785-794 for large scale preparation of epimorphin 12 fragments having 6 His at N-terminal (referred to as library for large scale preparation).
⑧ An aliquot of the thus obtained plasmid group was cloned in *E.coli* BL21, and the protein expression and the internal sequence of each clone were examined. As a result, all clones showed an induction of protein expression by IPTG, and epimorphin fragments wherein pep7 region is substituted with a different peptide and which lacks the region from C terminal to PvuII site of H12 were confirmed. A library of 2.5×10⁵ for large scale expression was constructed.

### Example 5: Preparation of pep7-like peptide

① The library for large scale preparation was introduced into *E.coli* BL-21, and applied onto LB-agarose (containing Amp) plate. On the next day, the library was cloned from the plate to each well of 96-well plate containing LB, and at the same time, a master plate was prepared. The plates were incubated overnight at 32°C, and then was treated with 1mM (final concentration) of IPTG for 2 hours. Then, His-tag proteins of each well were collected in a form which was bound with Ni-NTA (Qiagen) according to the manual of Qiagen. The details are disclosed below.
   The plate was centrifuged at 2000rpm for 15 minutes, and the medium was discarded. 50µl of 2mg/ml lysozyme was added to each well, and the plate was incubated for 1 hour. The operation of freezing with liquid nitrogen and melting at room temperature was repeated twice. The wells were treated with DNAse, and all proteins were dissolved by 150µl of 8M urea (pH8.0). Ni-NTA gel (Qiagen) equilibrated with 8M urea was added, and the wells were incubated for 30 minutes. The plate was centrifuged at 2000 rpm for 1 minute and the supernatant was discarded. The wells were washed twice with PBS, and once with water.
② Trypsin (sequence grade; Promega) was added to each well at a final concentration of 1µg/ml, and the plate was incubated overnight at 37°C.
③ After the plate was treated at 95°C for 5 minutes, the solution was collected, and the same amount of 2×DH10 (a mixed medium of DMEM and Ham12 (Gibco) supplemented with 10% FCS) was added thereto to prepare the samples. By the analysis with liquid chromatography, it was confirmed that the difference between the samples was the mutation of pep7 regions. Also, the sequence of each clone was analyzed using the master plate.

### Example 6: Preparation of a monoclonal antibody which is specific for new follicle

Hairs were cut off from the skin of B57BL mouse of growing stage (48 to 50 day), and were incubated overnight at 37°C in PBS containing 8M urea, 2% SDS and 100mM DTT, thereby the protein was extracted. Further, whisker follicles of B57BL mouse (where hair ball portion is stained with pigment; growing stage) were collected with a stereoscopic microscope, and were homogenized in PBS. The above 2 samples (0.5mg of protein weight) were mixed, and mixed with the same amount of the complete adjuvant to prepare micelle.

The above-obtained micelle (0.2mg) was subcutaneously (3 sites) administered to a rat (Wister) for immunization. After the first immunization, the booster was performed in the same way as in the above. After 2 weeks, the second booster was performed in the same way as in the above. On the third day after the second booster, a spleen was removed from the immunized rat, and the blood cells were collected by mesh. Antibody producing cells were contained in these blood cells. All amount of the above-collected blood cells were mixed with mouse myeloma P3U1 using polyethyleneglycol 1500, and were suspended in Dulbecco/Hum F12 mixed medium. 100µl of the culture were inoculated in each well of 96-well plate. On the next day, the same amount (100µl) of HAT medium (Sigma) was added to each well. After 2 days, 150µl of the medium was removed under aspiration from each well, and 150µl of the fresh medium was added to each well. The 96-well plate was placed in CO₂ incubator at 37°C.

The follicles of the growing whisker of B57BL mouse were dissolved in 8M urea by ultrasonic treatment. A nitrocellulose membrane was immersed in this solution for 5 minutes, and was washed well with PBS. Biorad dot blotter equipped with the above membrane was used to perform the first screening of the hybridoma supernatant which was recovered from each well of the above 96-well plate. First, the above prepared nitrocellulose membrane was blocked with Tris buffer containing 5% skim milk (TBST), and then 100µl of the hybridoma supernatant was added to each well where the nitrocellulose membrane constitutes the bottom. After incubation for 1 hour, the wells were washed with Tris buffer, and the second antibody, horseradish peroxidase labeled anti-rat IgG (1µg/ml TBST), was added. ECL agent (AmershamPharmacia), which is a coloring substrate, was added, and 50 antibodies in total which reacted with the growing whisker follicle were selected by detection of coloring (first screening).

Among the 50 antibodies selected in the above first screening, the antibodies which specifically reacted with the frozen segment (10µm) of the growing whisker follicle were selected (second screening). Specifically, the frozen segment of the growing whisker follicle was placed on a slide glass, and the hybridoma supernatant selected in the first screening was added thereto, and the coloring was developed with the second antibody. More specifically, the frozen segment of the growing whisker follicle which was prepared by Cryosdat (Bright) was treated with methanol at -20°C, and was blocked with TBST for 1 hour, and then was reacted with the hybridoma supernatant for 1 hour. The sample was washed with Tris buffer, and was reacted with FITC-labeled anti-rat IgG (100µg/ml in TBST). The sample was washed with Tris buffer, and was covered with a cover glass. The observation was carried out under fluorescent microscope.

As a result of the second screening, 8 antibodies were selected. These antibodies did not react with epidermis, and specifically reacted with follicle. These 8 antibodies were cloned by the limiting dilution.

The reactivity of these 8 antibodies was examined by Western Blotting using growing whisker follicle (anagen phase) or resting (telogen) whisker follicle as a sample, and using slide samples of the skins having follicles derived from 14 day fetal mouse. As a result, mAb27 was obtained as a monoclonal antibody which specifically reacted with growing whisker follicle and plastic follicle (new follicle), and did not react with resting whisker follicle. The hybridoma which produced monoclonal antibody mAb27 was deposited with Patent and Bio-Resource Center of National Institute of Advanced Industrial Science and Technology (Chuo-6, 1-1, Higashi 1-chome, Tsukuba-shi, Ibaraki-ken, Japan) on November 2, 2001 under the deposit number of FERM P-18578.

Fig.12 (A) shows the result of detection of the antigen of mAb27 by Western blotting. Each of the lanes from left to right shows the result of the analysis where each protein extracted from anagen and telogen of whisker and anagen and telogen of back skin was subjected to electrophoresis and Western blotting, and then the antigen was detected by using the monoclonal antibody mAb27 of the present invention.

Specifically, the solution obtained by milling whisker follicle with ultrasonic treatment in 8M urea was used as an antigen without dilution. The electrophoresis was carried out under a condition of a constant electric current of 30mA in SDS-PAGE (acrylamide 4 to 20%). Running buffer is 14.4g/L of glycine, 1g/L of Tris, and 1g/L of SDS. After electrophoresis, the sample was transferred to PVDF membrane, and was incubated in Tris buffer containing 5% skim milk (TBST) for 1 hour. Then, the sample was reacted with mAb27 (100µl of hybridoma supernatant) for 1 hour, and washed well with TBS, and was reacted with peroxidase-labeled anti-rat IgG (Amersham) (dissolved at 1 mg/ml in TBST) as a second antibody. After washing well, the intensity of the reaction of mAb27 was measured by using ECL kit (Amersham).

As a result, it was found that the monoclonal antibody mAb27 of the present invention detected an antigen of about 220kDa specifically present in an anagen sample.

Fig.12 (B) shows the result of histological staining using hair of adult and Fig. 12 (C) maxilla of 14th day mouse embryo. The procedure is same as in the procedure of the second screening of the antibodies in Example 1.

As a result, it was found that, in the case of using the monoclonal antibody mAb27 of the present invention, epidermis was not stained and only hair bulb was stained in hair of adult, and epidermis was not stained and only hair follicle was stained in maxilla of 14^{th} day mouse embryo.

From the results shown in Fig. 12 (A)-(C), it was demonstrated that the monoclonal antibody mAb27 of the present invention specifically recognized an antigen of about 220kDa present in new follicle.

### Example 7: Evaluation of hair-growth promoting activity

The skin of the back of ICR mouse (pregnancy 14 days) was pealed off by pincette, and was collected in HCMF. The collected skin was transferred to a plate, and was cut into pieces using 2 knives with grip. The cut skin was transferred to 15ml centrifuge tube, and was centrifuged at 1000 rpm for 1 second. 50 µl of DNase (2mg/ml) was added to the precipitate, and the mixture was homogenized. 10 ml of DH10 medium was added and the mixture was centrifuged. 10 ml of DH10 medium was added to the precipitate, and the mixture was suspended. After the mixture was suspended to be homogenized using a tip, 100µl of the suspension was applied onto each 96-well plate.

Onto the suspension in 96 well plates, 50µl of each sample obtained in Example 5 ③ above was added respectively, and the plates were incubated at 37°C for 2 days in CO₂ incubator. During incubation, PBS was filled in the gaps between wells for avoiding drying, and the plate was sealed. After incubation, 100µl of 8M urea was added.

A paper and a nitrocellulose membrane which have been immersed in TBS were set on Dot blotter (96 well) produced by BioRad. Such equipment was prepared in 3 sets. 10 µl was applied to 2 membranes where one (panel A) is used for monoclonal antibody mAb27, and the other is used for anti-E-cadherin (TAKARA) which detects all epitheliums. After blotting the solution into membrane by aspiration, the membranes were removed and dried at room temperature for 10 minutes. The membranes were washed once with TBS, and blocked with skim milk at room temperature for 1 hour. Then, the membranes were incubated with the primary antibody, monoclonal antibody mAb 27 (the culture supernatant of hybridoma was diluted to 1/30) or anti-E-cadherin antibody (1/2000 diluted, TAKARA) at room temperature for 1 hour. The membranes were washed twice with TBS for each 10 minutes. Then, peroxidase labeled anti-rat IgG or peroxidase labeled anti-rabbit IgG (Amersham) (1/1000 diluted) was reacted as the second antibody, and the membranes were washed twice with TBS for each 10 minutes. The intensity of the reaction was examined using ECL plus (Amersham).

### Example 8: Data analysis

The autora-film of those detected by ECL was uptaken using Fuji Photo Film luminoimage analyzer (LAS-1000plus). The amount of the antigen of monoclonal antibody mAb27 was measured from panel A as an index of induction of new follicle, and the amount of E-cadherin was measured from panel B as an index of total numbers of epithelial cells.

The ratio of the measured amounts obtained from panel A and panel B was analyzed by computer, and the degree of the induction of hair-growth per epithelial cell was analyzed.

As a result of the analysis of 960 samples in total, samples were obtained where the measured amount from panel A is higher than the measured amount from panel B (i.e., samples having a high ability of inducing new follicle). The pep7-like sequences of 65 samples among such samples are shown herein in Table I. The requirements of the structure of the amino acid sequences of these 65 clones are also shown in Table II.

The samples where the measured amount from panel A is higher than the measured amount from panel B, are shown in Table III. The amino acid sequences of these samples are: Tyr Asn Glu Gln Ser Cys Asp Arg Glu Glu (SEQ ID NO:17) Thr Ser Asp Gln Cys Cys Asp Pro Asp Lys (SEQ ID NO:75) Pro Ser Glu Gln Ser Cys Ala Glu Glu Glu (SEQ ID NO:61) Ser Asn Glu Gln Ser Cys Ala Val Ala Glu (SEQ ID NO:29) Thr Thr Glu Gln Ser Cys Ala Val Asp Glu (SEQ ID NO:63) Ser Ile Glu Gln Ser Cys Gly Gln His Glu (SEQ ID NO:80) Ser Ser Ala Gln Ser Cys Leu Gln Asp Thr (SEQ ID NO:48) Tyr Ile Glu Gln Tyr Cys Asp Gln Asp Glu (SEQ ID NO:64) Thr Ile Trp Gln Ser Cys Asp Gln Glu Glu (SEQ ID NO:32) The activity of these peptides is about 3 times higher than the peptide of amino acid sequence Ser Ile Glu Gln Ser Cys Asp, from the pep7 region of murine epimorphin.

Among the 65 clones, there is a peptide of 7 amino acid residues having an amino acid sequence of Ser-Ile-Glu-Gln-Ser-Cys-Asp. Therefore, this 7 amino acid residues is considered to be a region necessary for hair growth promoting activity.

In the 65 clones, Cys residue is not substituted with another amino acid, and therefore, a Cys residue is important in the oligopeptides of the present invention. Monomers comprising a Cys amino acid residue would be capable of dimerizing under suitable conditions.

In the 65 clones, the amino acid sequence of Glu-Gln-Ser-Cys-Asp (SEQ ID NO:108) is relatively highly conserved (i.e., conserved in 23 clones), and the amino acid sequence of Glu-Gln-Ser-Cys (SEQ ID NO:109) is further highly conserved (i.e., conserved in 30 clones).

In the Examples, 65 peptide clones having a high ability to induce a new follicle were obtained from 960 samples. 2.5 x 10⁵ peptide clones were obtained in the second screening as shown in Example 3, and peptides having the same ability other than the above 65 clones are considered to be contained therein. In some examples, 3 amino acid residues among 7 amino acid residues may be substituted. If the candidate amino acid for respective amino acid is composed of 8 types, the total number of combination is estimated as ₇C₃ ×8³ = 17920. The ratio of 17920 clones in the library of 2.5×10⁵ is 7.1%. On the other hand, the ratio of 65 clones in the population of 960 samples is 6.8%. The aforementioned assumption is well consistent with the result of the Examples of the present application (the ratio of the peptides which is positive in the screening).

The following oligopeptides based on the sequence provided in Table II (shown in one letter amino acid code) are predicted to exhibit hair-growth promoting activity:

SIDQSCD (SEQ ID NO:110)

SIEESCD (SEQ ID NO:111)

SIEQACD (SEQ ID NO:112)

SIEQSCR (SEQ ID NO:113)

SIEQFCH (SEQ ID NO:114)

SFDQSCD  (SEQ ID NO:115)

SFEESCD (SEQ ID NO:116)

SFEQACD (SEQ ID NO:117)

SFEQSCR (SEQ ID NO:118)

SFEQFCH (SEQ ID NO:119)

### Example 9: Altered oligopeptides

Oligopeptides (A), (B), (C) and (D) represented by the following amino acid sequences were synthesized by solid phase method using Fmoc.

A. Ser-Ile-Glu-Gln-Cys-Ser-Asp-Gln-Asp-Glu (SEQ ID NO:120)

B. Ser-Ile-Glu-Cys-Gln-Ser-Asp-Gln-Asp-Glu  (SEQ ID NO:121)

C. Ser-Ile-Cys-Glu-Gln-Ser-Asp-Gln-Asp-Glu  (SEQ ID NO:122)

D. Ser-Ile-Glu-Cys-Gln-Ser-Asp-Gln (SEQ ID NO:83)

The synthesized oligopeptides were dimerized using a cross-linking agent (bismaleimide hexane, trade name BMH, produced by Pierce) to prepare a homodimer. Specifically, the synthesis was carried out according to the instructions of this cross-linking agent.

The obtained reaction mixture contained not only homodimer oligopeptide, but also monomer oligopeptide to which a cross-linking agent was bound.

In the same way as in the above, oligopeptides Ser-Ile-Glu-Gln-Ser-Cys-Asp and Ser-Ile-Glu-Gln-Cys-Ser-Asp (SEQ ID NO:4) were used in an equal amount to prepare heterodimer oligopeptide. The obtained reaction mixture contained not only a heterodimer oligopeptide, but also monomer oligopeptide to which a cross-linking agent was bound.

Each of the synthesized oligopeptides (A), (B) and (C) was purified by a high performance liquid chromatography (HPLC), and it was confirmed by HPLC and Mass that the purity was 90% or more.

The conditions of HPLC are mentioned below.
Column: ODS-UG3 (Monomeric ODS, Nomura Kagaku), 1.0 mm in inside diameter, 100 mm in length
Measurement: room temperature (25°C)
Detection: UV 214 nm, 280 nm

Eluting solvent: gradient of solvent A and solvent B (solvent A: 0.1% trifluoroacetic acid; solvent B: 90% acetonitrile/0.1% trifluoroacetic acid, linear concentration gradient from 5 minutes after (solvent B: 0%) to 55 minutes (solvent B: 55%)
Flow rate: 75 ml/ml
Retention time of oligopeptides
21.52 minutes (dimer), 20.59 minutes (monomer)

### Example 10: Evaluation of hair growth promoting activity of altered oligopeptides

The skin tissues of maxilla of ICR mice (pregnancy 12 days) were collected by stereoscopic microscope, and left and right sides were respectively recovered from 5 mice. 5 pieces of each of the thus collected left (for control) and right (for test oligopeptides) skins from 5 mice were respectively placed on 1 nuclepore membrane (pore diameter 8 µm; diameter 13mm), and were set in such a way that the outside upturns by observing the samples with stereoscopic microscope. 500µl of Dulbecco's MEM/Ham F12 medium containing 1% BSA was added to 2 wells of 24 well dish. The test oligopeptide in a solvent (PBS) was added in a final concentration of 20µM to one well, and solvent (PBS) was added in the same amount to the other well as a control. The oligopeptides synthesized in Example 9 were used as the test oligopeptides.

Each membrane having skin tissues thereon was launched on the solution in the above wells, and was incubated at 37°C for 6 days. 5 pieces of tissue were recovered from the membrane into 100µl of SDS sample buffer (SDS 0.02g/ml, glycerol 0.2g/ml, pH6.8), and dissolved by ultrasonic treatment. The control membrane was also treated similarly. The solution obtained by such a treatment was subjected to electrophoresis (35mA, 1.5 hour) in SDS-PAGE (acrylamide 4-20%), and transferred to PVDF membrane, and incubated in Tris buffer containing 5% skim milk (TBST) for 1 hour. The membrane was reacted with mAb 27 (10µg/ml in TBST) obtained in Example 2 for 1 hour, and was washed well with TBS. Then, peroxidase labeled anti-rat IgG (Amersham) (1/1000 diluted in TBST) was reacted as the second antibody, and the membrane was washed well with TBS. The intensity of the reaction of mAb 27 was examined using ECL kit (Amersham).

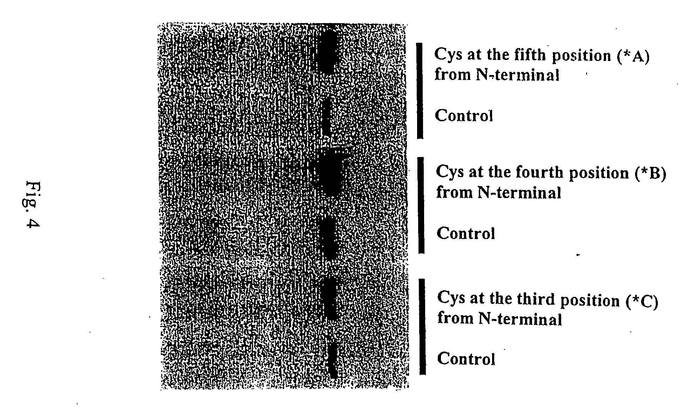

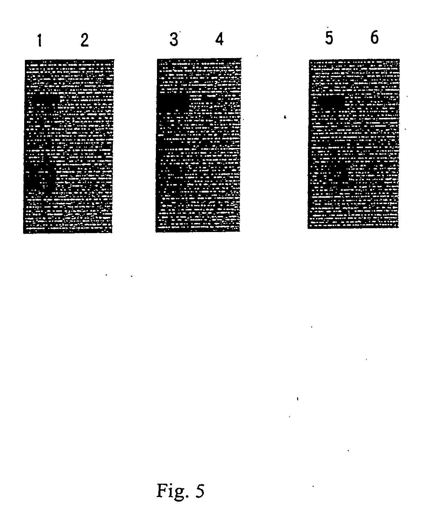

The obtained results are shown in Fig.4 and Fig.5.

The test oligopeptides in each lane of Fig.4 are as follows.
First from left: Ser-Ile-Glu-Gln-Cys-Ser-Asp-Gln-Asp-Glu (A) (SEQ ID NO:120)
Second from left: Control
Third from left: Ser-Ile-Glu-Cys-Gln-Ser-Asp-Gln-Asp-Glu (B) (SEQ ID NO:121)
Fourth from left: Control
Fifth from left: Ser-Ile-Cys-Glu-Gln-Ser-Asp-Gln-Asp-Glu (C) (SEQ ID NO:122)
Sixth from left: Control

As is understood from the results of Fig.4, the bands in the first, third and fifth lane from the left were stronger than the bands in the second, fourth and sixth lane from the left respectively. These results demonstrate that the test oligopeptides (A), (B) and (C) have a hair growth promoting activity.

The test oligopeptides in each lane of Fig.5 are as follows.
First from left: a mixture obtained by mixing oligopeptide (A) and oligopeptide (B) after treating them.
Second from left: Control
Third from left: a mixture obtained by treating oligopeptide (A) and oligopeptide (B) after mixing them.
Fourth from left : Control
Fifth from left : Ser-Ile-Glu-Cys-Gln-Ser-Asp-Gln (D) (SEQ ID NO:83) (having two amino acids at the C-terminus of (b) removed).
Sixth from left : Control

As is understood from the results of Fig.5, similar activities were observed in the mixture obtained by mixing oligopeptide (A) and oligopeptide (B) after treating them, and the mixture obtained by treating oligopeptide (A) and oligopeptide (B) after mixing them. Therefore, it was demonstrated that heterodimer oligopeptide had also a hair growth promoting activity. It was also found that oligopeptide (D) with 2 amino acids at C-terminal being deleted had a hair growth promoting activity.

### Examplel 1:

Various oligopeptides wherein the cysteine residue in the oligopeptides: Ser Ile Asp Gln Ser Cys Asp  (SEQ ID NO:110) Ser Ile Glu Glu Ser Cys Asp (SEQ ID NO:111) Ser Ile Glu Gln Ala Cys Asp (SEQ ID NO:112) Ser Ile Glu Gln Ser Cys Arg  (SEQ ID NO:113) Ser Ile Glu Gln Phe Cys His (SEQ ID NO:114) Ser Phe Asp Gln Ser Cys Asp (SEQ ID NO:115) Ser Phe Glu Glu Ser Cys Asp (SEQ ID NO:116) Ser Phe Glu Gln Ala Cys Asp (SEQ ID NO:117) Ser Phe Glu Gln Ser Cys Arg  (SEQ ID NO:118) Ser Phe Glu Gln Phe Cys His (SEQ ID NO:119) was moved upstream by 1, 2 or 3 amino acids, were synthesized as in Example 9, and were evaluated as to hair-growth promoting activity in the same way as in the above.

As a result, the activity is observed. That is, it was found that the amino acid sequence of Ser-Ile-Glu-Gln-Cys-Ser-Asp (SEQ ID NO:4), Ser-Ile-Glu-Cys-Gln-Ser-Asp (SEQ ID NO:5) or Ser-Ile-Cys-Glu-Gln-Ser-Asp (SEQ ID NO:6) wherein the position of Cys is altered, showed a hair growth promoting activity. Each amino acid in these peptides can also be substituted as in the case of Ser-Ile-Glu-Gln-Ser-Cys-Asp. For example, the peptide of the amino acid sequence Ser Asn Glu Pro Cys Ser Asp Gln Gly Gly (SEQ ID NO:24) is obtained by substituting Ile and Gln with Asn and Pro respectively in the peptide of amino acid sequence of Ser-Ile-Glu-Gln-Cys-Ser-Asp (SEQ ID NO:4), and it is considered that the peptide having such substitutions will show a hair growth promoting activity, as is the case shown in the present specification.

### Example 12: Preparation of modified oligopeptides

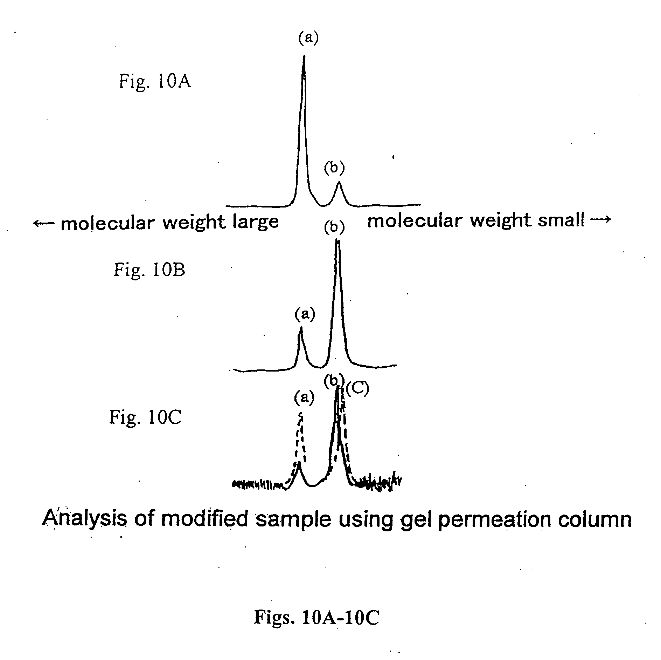

An oligopeptide represented by the following amino acid sequence was synthesized by solid phase method using Fmoc.
Ser-Ile-Glu-Gln-Ser-Cys-Asp-Gln-Asp-Glu (comprising murine pep7 region). Then, the synthesized oligopeptide was reacted with a cross-linking agent (bismaleimide hexane, trade name BMH, produced by Pierce) according to the instructions of this cross-linking agent. The reactions were carried out in various ratios of the oligopeptide and the cross-linking agent. Figs. 10A-10C shows the result obtained by analyzing the reaction product of the oligopeptide and the cross-linking agent with gel permeation column (Amersham Pharmacia, Superdex™ peptide PC 3.2/30, 250mM NaCL, 20mM Na-phosphate buffer (pH7.2) was used as a developing solution).

The upper diagram (A) in Fig. 10 shows the result obtained by reacting the oligopeptide with bismaleimide in a ratio of 7:3 and separating with liquid chromatography (Amersham Pharmacia, Smart system). Peak (a) represents a peptide dimer, and peak (b) represents a peptide dimer having a reactive maleimide. Peak (a) corresponds to the time when 1.2 ml was eluted, and peak (b) corresponds to the time when 1.4 ml was eluted.

The middle diagram (B) in Fig. 10 shows the result obtained by reacting the oligopeptide with bismaleimide in a ratio of 2:5. Peak (a) represents a peptide dimer, and peak (b) represents a peptide dimer having a reactive maleimide. Since bismaleimide hexane exists in excess, the peptide dimer having a reactive maleimide (b) has been formed in larger amount than the peptide dimer (a).

The lower diagram (C) in Fig. 10 shows the result of the sample obtained by adding an non-modified oligopeptide Ser-Ile-Glu-Gln-Ser-Cys-Asp-Gln-Asp-Glu in excess to the sample of the above (B) (i.e., the sample obtained by reacting the oligopeptide with bismaleimide in a ratio of 2:5). From the result where Peak (b) decreases and peak (a) increases, it is understood that a part of (c) which was added is reacted with (b) to form a dimer (a).

### Example 13: Evaluation of hair growth promoting activity of various oligopeptides

### (1) Preparation of various oligopeptides

Oligopeptides (A) and (B) represented by the following amino acid sequences were synthesized by solid phase method using Fmoc (hereinafter, these are referred to as the unmodified oligopeptides).

(A) Ser-Ile-Glu-Gln-Ser-Cys-Asp-Gln-Asp-Glu (SEQ ID NO:142)

(B) Ser-Ile-Glu-Gln-Ser-Lys-Asp-Gln-Asp-Glu (SEQ ID NO:123)

The synthesized oligopeptide (A) was reacted with a cross-linking agent (bismaleimide hexane, trade name BMH, produced by Pierce) according to the instructions of this cross-linking agent. Also, the synthesized oligopeptide (B) was reacted with a cross-linking agent DSG (disuccinimidyl glutarate). Using a fraction collector, the monomer to which the cross-linking agent was bound was fractioned and collected.

### (2) Evaluation of hair growth promoting activity

The skin tissues of maxilla of ICR mice (pregnancy 12 days) were collected by stereoscopic microscope, and left and right sides were respectively recovered from 5 mice. 5 pieces of each of the thus collected left (for control) and right (for test oligopeptides) skins from 5 mice were respectively placed on 1 nuclepore membrane (pore diameter 8 µm; diameter 13mm), and were set in such a way that the outside upturns by observing the samples with stereoscopic microscope. 500µl of Dalbecco's MEM/Ham F12 medium containing 1% BSA was added to 2 wells of 24 well dish. The test oligopeptide in a solvent (PBS) was added in a final concentration of 20µM to one well, and solvent (PBS) was added in the same amount to the other well as a control. The unmodified and modified oligopeptides synthesized in the above (1) were used as the test oligopeptides.

Each membrane having skin tissues thereon was launched on the solution in the above wells, and was incubated at 37°C for 6 days. 5 pieces of tissue were recovered from the membrane into 100µl of SDS sample buffer (SDS 0.02g/ml, glycerol 0.2g/ml, pH6.8), and dissolved by ultrasonic treatment. The control membrane was also treated similarly. The solution obtained by such a treatment was subjected to electrophoresis (35mA, 1.5 hour) in SDS-PAGE (acrylamide 4-20%), and transferred to PVDF membrane, and incubated in Tris buffer containing 5% skim milk (TBST) for 1 hour. The membrane was reacted with mAb 27 (10µg/ml in TBST) obtained in Example 6 for 1 hour, and was washed well with TBS. Then, peroxidase labeled anti-rat IgG (Amersham) (1/1000 diluted in TBST) was reacted as the second antibody, and the membrane was washed well with TBS. The intensity of the reaction of mAb 27 was examined using ECL kit (Amersham).

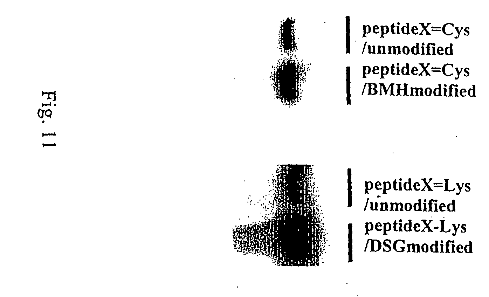

The results obtained are shown in Fig. 11.

The test oligopeptides in each lane of Fig. 11 are as follows.
- First from left:: Unmodified oligopeptide (A)
- Second from left:: BMH modified oligopeptide (A)
- Third from left:: Unmodified oligopeptide (B)
- Fourth from left:: DSG modified oligopeptide (B)

As is understood from the results of Fig.11, the bands in the second and fourth lane from the left (modified oligopeptides) were stronger than the bands in the first and third lane from the left (unmodified oligopeptides) respectively. These results demonstrate that the modified oligopeptides have a hair growth promoting activity, especially a higher hair growth promoting activity than that of the corresponding unmodified oligopeptide.

Moreover, by using the oligopeptides having an altered amino acid sequence and various cross-linking agents, the hair growth promoting activity was evaluated in the same manner as in the above.

As a result, it was demonstrated that all of the modified oligopeptides which were obtained by modifying Ser-Ile-Glu-Gln-Ser-Cys-Asp-Gln-Asp-Glu; Ser-Ile-Cys-Glu-Gln-Ser-Asp-Gln-Asp-Glu (SEQ ID NO:122); or Ser-Ile-Glu-Gln-Ser-Cys-Asp-Gln with bismaleimide hexane (BMH), 1,4-bismaleimide butane (BMB) or bismaleimide ethane (BMOE), showed a hair growth promoting activity.

### Example 14: Preparation of oligopeptides

An oligopeptide represented by the following amino acid sequence Ser-Ile-Glu-Gln-Ser-Cys-Asp-Gln-Asp-Glu (comprising murine pep7 region) was synthesized by solid phase method using Fmoc. Also, a modified oligopeptide (referred to herein as "b7") having a modification by biotin at the N-terminus of the above sequence was prepared.
The synthesized oligopeptides were purified by a high performance liquid chromatography (HPLC), and it was confirmed by HPLC and Mass that the purity was 90% or more.

The conditions of HPLC are disclosed below.
Column: ODS-UG3 (Monomeric ODS, Nomura Kagaku), 1.0 mm in inside diameter, 100 mm in length
Measurement: room temperature (25°C)
Detection: UV 214 nm, 280 nm

Eluenting solvent: gradient of solvent A and solvent B (solvent A: 0.1% trifluoroacetic acid; solvent B: 90% acetonitrile/0.1% trifluoroacetic acid, linear concentration gradient from 5 minutes after (solvent B: 0%) to 55 minutes (solvent B: 55%)
Flow rate: 75 ml/ml
Retention time of oligopeptides:
Ser-Ile-Glu-Gln-Ser-Cys-Asp-Gln-Asp-Glu: 21.52 minutes (dimer), 20.59 minutes (monomer)
b7; 29.89 minutes (monomer), 32.85 minutes (dimer)

The above-prepared oligopeptides were dissolved in phosphate buffered saline (PBS) in 0.3 mg/ml, and the same amount of 100% ethanol was added to this solution to prepare 50% ethanol/PBS solution (0.15mg/ml). The cross-linking of the oligopeptide was carried out as follows. BMH dissolved in dimethylsulfoxide (65µl) was slowly added with stirring in a final concentration 33µg/µl to the 1mg/ml oligopeptide solution (5ml) in PBS, and the mixture was reacted overnight at 4°C. To this solution, 6.6 ml of PBS and a solution of cysteine hydrochloride dissolved in PBS at 5 ml so as to give a final concentration of 5mg/ml were added and mixed to prepare a S-S bridged oligopeptide (referred to as "ss7") solution (containing monomer to which a cross-linking agent was bound, as well as dimer) at a final concentration of 0.3mg/ml. The retention time is 33.01 minutes by HPLC as performed under the conditions disclosed above. A control solution was prepared by reacting ni the same manner with the addition of the reagents except for using PBS instead of the oligopeptide solution. A solution of the cross-linked oligopeptide was also added with the same volume of ethanol to prepare a 50% ethanol/PBS solution at the final concentration of 0.15mg/ml. This S-S bridged oligopeptide was used for the evaluation of hair growth promoting activity in Examples 15 and Example 16.

### Example 15: Evaluation of hair growth promoting activity by in vivo method

C3H and C57BL/6 mice are known to have sustained telogen for about 50 days from the 45th day after the birth to around the 95th day. Their hair cycle is easily judged based on the skin color changes, i.e., from pink in telogen to gray or black in anagen. A test for evaluating whether or not the administration the oligopeptides of the present invention promotes the transition from telogen to anagen was carried out using the mice. Seven weeks old (48 to 50-day old, female) C57BL/6 mice were purchased and hair of the back (about 3×2.5 cm²) was carefully shaved with electric clippers for animals so as not to injure the skin, and the hair cycle was confirmed to be in telogen from the skin color. The oligopeptide solution prepared above was applied to five mice in each group, once a day and 5 days in a week in the amount of 0.2 ml until 38th day from the start of the test. The application was carried out by using a syringe without needle. Dipeptide (Ile-Lys) and tripeptide (Glu-Ile-Lys) of which N-terminal is biotinylated were mixed, and then added with the cross-linking agent to prepare a control solution.

Plural persons (two persons) observed the mice twice a week by naked eye for evaluation, and gave a 6-graded score depending on a ratio of hair restoration area based on the hair shaved area. At the same time, photographs were taken. Hair growth scores were calculated as follows. At first, the following score was given depending on a ratio of areas where the skin color changes to gray or black in the hair shaved area; 0-20% : 1, 20-40% : 2, 40-60% : 3, 60-80% :4, 80-100% : 5. The sum of the above scores in each group was determined as the hair growth score. The maximum value of the hair growth score of each group was 50 for each of the persons for judgment, and the maximum value of hair growth score was 100 because the judgment was made by two persons. In the group wherein the S-S bridged oligopeptide (containing monomer to which a cross-linking agent was bound, as well as dimer) and biotynalated oligopeptide was applied, the transition to anagen was 7 days or more earlier than the control group, and the hair restoration was promoted at any time until the hair grew and restored almost completely. In the group wherein the biotinylated oligopeptide was applied, the hair restoration was promoted until about 30 days from the start of the test as compared with the control group similarly to the S-S bridged group. The results are shown in Figures 6 and 7. As shown by these results, the oligopeptide represented by the amino acid sequence Ser-Ile-Glu-Gln-Ser-Cys-Asp-Gln-Asp-Glu has hair growth promoting activity.

### Example 16: Evaluation of hair growth promoting activity using the monoclonal antibody of the present invention

The skin tissues of maxilla of ICR mice (pregnancy 12 days) were collected by stereoscopic microscope, and left and right sides were respectively recovered from 5 mice. 5 pieces of each of the thus collected left (for control) and right (for test oligopeptides) skins from 5 mice were respectively placed on 1 nuclepore membrane (pore diameter 8 µm; diameter 13mm), and were set in such a way that the outside upturns by observing the samples with stereoscopic microscope. 500µl of Dalbecco's MEM/Ham F12 medium containing 1% BSA was added to 2 wells of 24 well dish. The test oligopeptide in a solvent (PBS) was added in a final concentration of 20µM to one well, and solvent (PBS) was added in the same amount to the other well as a control. The S-S bridged oligopeptide prepared in the above Example was used as the test oligopeptide.

Each membrane having skin tissues thereon was launched on the solution in the above wells, and was incubated at 37°C for 6 days. 5 pieces of tissue were recovered from the membrane into 100µl of SDS sample buffer (SDS 0.02g/ml, glycerol 0.2g/ml, pH6.8), and dissolved by ultrasonic treatment. The control membrane was also treated similarly. The solution obtained by such a treatment was subjected to electrophoresis (35mA, 1.5 hour) in SDS-PAGE (acrylamide 4-20%), and transferred to PVDF membrane, and incubated in Tris buffer containing 5% skim milk (TBST) for 1 hour. The membrane was reacted with mAb 27 (10µg/ml in TBST) obtained in Example 6 for 1 hour, and was washed well with TBS. Then, peroxidase labeled anti-rat IgG (Amersham) (1/1000 diluted in TBST) was reacted as the second antibody, and the membrane was washed well with TBS. The intensity of the reaction of mAb 27 was examined using ECL kit (Amersham).

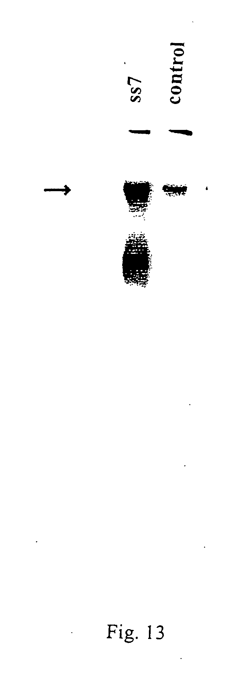

The obtained results are shown in Fig. 13.

In Fig.13, left lane shows the result of the sample where the S-S bridged oligopeptide (ss7) prepared in Example 14 was added, and right lane (control) shows the sample where only solvent was added. As is understood from the results of Fig.13, a stronger band was detected in the sample where the S-S bridged oligopeptide (ss7) was added, than in the control. This suggests that the expressed amount of an antigen recognized by mAb27 is increased.

This Example demonstrates that ss7 has hair growth promoting activity. From this example, it is understood that ss7 increases the expressed amount of the antigen of mAb27. From the examples, it is understood that the antigen of mAb27 is specific for anagen of follicle. Therefore, the increase of antigen of mAb27 is considered to be an index which represents a hair growth promoting activity. That is, a hair growth promoting activity can be evaluated by examining the expression of the antigen of about 220 kDa present in new follicle using mAb27.

The monoclonal antibody of the present invention can specifically recognize an antigen present in epithelial new follicle, and is useful for the evaluation of hair growth promoting activity.

### Example 17

Oligopeptide b7ΔC1, b7ΔC2, b7ΔC3, b7ΔC4, and b7ΔC5 were synthesized by deleting one, two, three, four, or five amino acids from the C-terminal of b7, respectively, and then blocking sulfhydryl group. Oligopeptide b7ΔN1, b7ΔN2, and b7ΔN3 were synthesized by deleting one, two, or three amino acids from the N-terminal of b7. b7ΔC1 refers to the oligopeptide sequence (SIEQSCDQD); b7ΔC2 refers to the oligopeptide sequence (SIEQSCDQ); b7ΔC3 refers to the oligopeptide sequence (SIEQSCD); b7ΔC4 refers to the oligopeptide sequence (SIEQSC); b7ΔC5 refers to the oligopeptide sequence (SIEQS); b7ΔN1 refers to the oligopeptide sequence (IEQSCDQDE); b7ΔN2 refers to the oligopeptide sequence (EQSCDQDE); and b7ΔN3 refers to the oligopeptide sequence (QSCDQDE). Oligopeptide bk7 (without block of sulfhydryl group) represented by the amino acid sequence Lys-Ser-Ile-Glu-Gln-Ser-Cys-Asp-Gln-Asp-Glu was synthesized whose N-terminal was bound with lysine.

Human keratinocytes (NHEK cell, Clonetics, available from Sanko Jyun-yaku, Ltd.) were cultivated in a medium for proliferation (Clontics) comprising 30µg/ml BPE, 0.1ng/ml human EGF, 5µg/ml insulin, 0.5µg/ml hydrocortisone, 50µg/ml gentamycin, and 50 ng/ml amphoterin. These cells were re-suspended at the concentration of 1 × 10⁴ cells/ml in a medium wherein EGF, insulin, and hydrocortisone were removed from the above medium for proliferation, and then 100µl of the suspension was placed on each well of a 96-well plate. At the same time of the plating of the cells, 5µl of 1 mg/ml oligopeptide was added to the suspension so as to be final concentration of 50µg/ml. After cultivation for 16-20 hours, the amount of IL-8 in the culture supernatants was measured by ELISA kit (ENDOGEN). Table IV shows the correlation between inducing activity on IL-8 secretion by NHEK cells and hair growth promoting activity.

**Table IV**

| Oligopeptide | IL-8 inducing activity | Hair growth Activity |
|---|---|---|
| b7 | ⓞ | ○ |
| cross-linked b7 | ⓞ | ⓞ |
| ss7 | ⓞ | ⓞ |
| control | × | × |

b7 refers to b7 is SIEQSCDQDE. ssb7 refers to cross-linked b7. ss7 refers to S-S bridged and biotinylated oligopeptide.

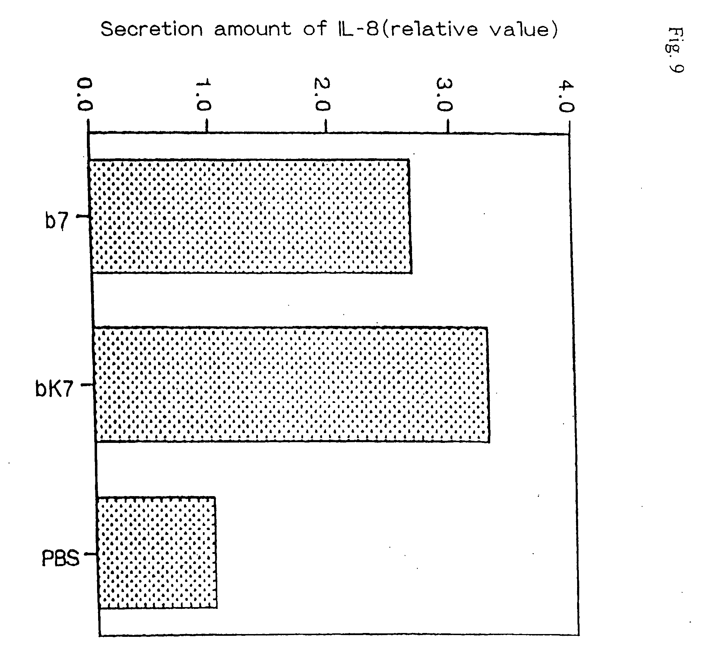

The results obtained by evaluation of oligopeptides b7ΔC1, b7ΔC2, b7ΔC3, b7ΔC4, b7ΔC5, b7ΔN1, b7ΔN2, and b7ΔN3 on IL-8 inducing activity are shown in Fig. 8. When one or more amino acids were deleted from the N-terminal, the secretion amount of IL-8 was slightly decreased, and when one or more amino acids were deleted from the C-terminal, the secretion amount of IL-8 was maintained until deletion of four amino acids. When five amino acids were deleted, the secretion amount of IL-8 was significantly decreased. From these results, it is revealed that almost the same hair growth activity as that of oligopeptide b7 can be expected by the deletion of up to four amino acids from the C-terminal, or the deletion of up to three amino acids from the N-terminal of the b7 region. As shown in Figure 9, 11-mer oligopeptide bk7 had also almost the same IL-8 inducing activity as that of oligopeptide b7.

### Example 18

The N-terminus of oligopeptide murine pep7 was biotinylated using NHS-biotin or NHC-biotin (Pierce) in accordance with the instructions of the attached manual. When NHS-biotin was used, -O-CO-(CH₂)₄₋₍13.5Å) was introduced as a spacer between the N-terminus and the biotin, and when NHS-LC-biotin was used, -O-CO-(CH₂)₅-NH-CO-(CH₂)₄-(22.4Å) was introduced as a spacer between the N-terminal and the biotin. The secretion amount of IL-8 was determined using these biotinylated oligopeptides in the same manner as in Example 17. These oligopeptides had almost the same IL-8 inducing activity as that of oligopeptide b7. These results show that the oligopeptide of which N-terminal is directly biotinylated has almost the same hair growth promoting activity as that of the oligopeptide of which N-terminal is biotinylated by means of a spacer, and both of them are active as compared to the b7 region without biotin at the N-terminus.

## Claims

1. An isolated oligopeptide of between about 5 and about 104 amino acid residues in length having hair growth promoting activity, comprising the following amino acid sequence:
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Ala, Tyr, Thr, Pro, Phe, Val, Gly, Leu, Ile or Met, or is deleted from said oligopeptide;
X2 is an amino acid residue of Ile, Gly, Asn, Thr, Val, Ser, Phe, Leu, Ala, Pro, Cys, or Met, or is deleted from said oligopeptide;
X3 is an amino acid residue of Glu, Lys, Gln, Arg, Ala, Val, Trp, Cys, or Asp;
X4 is an amino acid residue of Gln, Pro, Glu, Thr, Arg, Ser, His, Cys, or Lys;
X5 is an amino acid residue of Ser, Trp, Phe, Thr, Cys, Tyr, Pro, Ala, Gly, Val, Leu, Ile, or Met;
X6 is an amino acid residue Cys; a reactive substance-bound Cys or a reactive substance-bound Lys; and
X7 is an amino acid residue of Asp, Glu, His, Ser, Ala, Gly, Asn, Tyr, Arg, or Leu, is deleted from said oligopeptide, with the proviso that the oligopeptide is not identical to SEQ ID NO:1 or SEQ ID NO:2, and is not identical to oligopeptide Ser-Ile-Glu-Gln-Ser-Cys-Asp or Ser-Ile-Glu-Gln-Ser-Cys.

2. The oligopeptide of claim 1 comprising the following amino acid sequence,
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Tyr, Thr, or Pro, or is deleted from said oligopeptide;
X2 is an amino acid residue of Ile, Asn, Thr, or Ser, or is deleted from said oligopeptide;
X3 is an amino acid residue of Glu, Ala, Trp, or Asp,
X4 is an amino acid residue of Gln,
X5 is an amino acid residue of Ser, Cys, or Tyr,
X6 is an amino acid residue of Cys; a reactive substance-bound Cys or a reactive substance-bound Lys; and
X7 is an amino acid residue of Asp, Ala, Gly, or Leu, or is deleted from said oligopeptide, with the proviso that the oligopeptide is not SEQ ID NO:2, and is not identical to oligopeptide Ser-Ile-Glu-Gln-Ser-Cys-Asp or Ser-Ile-Glu-Gln-Ser-Cys.

3. An isolated oligopeptide of between about 7 and about 100 amino acid residues in length having hair growth promoting activity, comprising the following amino acid sequence:
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Ala, Tyr, Thr, Pro, Phe, Val, Gly, Leu, Ile or Met, or is deleted from said oligopeptide;
X2 is an amino acid residue of Ile, Gly, Asn, Thr, Val, Ser, Phe, Leu, Ala, Pro, Cys, or Met, or is deleted from said oligopeptide;
X3 is an amino acid residue of Glu, Lys, Gln, Arg, Ala, Val, Trp, Cys, or Asp,
X4 is an amino acid residue of Gln, Pro, Glu, Thr, Arg, Ser, His, Cys, or Lys,
X5 is an amino acid residue of Ser, Trp, Phe, Thr, Cys, Tyr, Pro, Ala, Gly, Val, Leu, Ile, or Met,
X6 is an amino acid residue Cys; a reactive substance-bound Cys or a reactive substance-bound Lys; and
X7 is an amino acid residue of Asp, Glu, His, Ser, Ala, Gly, Asn, Tyr, Arg, or Leu, or is deleted from said oligopeptide, with the proviso that the oligopeptide is not identical to oligopeptide Ser-Ile-Glu-Gln-Ser-Cys-Asp or Ser-Ile-Glu-Gln-Ser-Cys.

4. The oligopeptide of claim 1 comprising the following amino acid sequence:
X1-X2-X3-X4-X5-X6-X7;
X1-X2-X3-X4-X6-X5-X7;
X1-X2-X3-X6-X4-X5-X7; or
X1-X2-X6-X3-X4-X5-X7;
wherein X1 is an amino acid residue of Ser, Tyr, Thr, or Pro, or is deleted from said oligopeptide;
X2 is an amino acid residue of Ile, Asn, Thr, or Ser, or is deleted from said oligopeptide;
X3 is an amino acid residue of Glu, Ala, Trp, or Asp,
X4 is an amino acid residue of Gln,
X5 is an amino acid residue of Ser, Cys, or Tyr,
X6 is an amino acid residue of Cys;
X7 is an amino acid residue of Asp, Ala, Gly, or Leu, or is deleted from said oligopeptide, with the proviso that the oligopeptide is not identical to oligopeptide Ser-Ile-Glu-Gln-Ser-Cys-Asp or Ser-Ile-Glu-Gln-Ser-Cys.

5. The oligopeptide of claim 1 comprising the following amino acid sequence:

T/Y-S/N-E-Q-S-C-A. (SEQ ID NO:3).

6. The oligopeptide of claim 3 comprising the following amino acid sequence:

T/Y-S/N-E-Q-S-C-A. (SEQ ID NO:3).

7. The oligopeptide of claim 1 wherein 1 to 3 amino acid residues are substitute in the amino acid sequence Ser-Ile-Glu-Gln-Ser-Cys-Asp wherein the amino acid residue to be substituted is other than Cys or the amino acid residue to be substituted is other than the third to sixth amino acid residues Glu-Gln-Ser-Cys.

8. The oligopeptide of claim 1 wherein 0 to 2 amino acid residues are substitute in the amino acid sequence represented by Glu-Gln-Ser-Cys-Asp; wherein the amino acid residue to be substituted is other than Cys or the amino acid residue to be substituted is other than the third to sixth amino acid residues Glu-Gln-Ser-Cys.

9. The oligopeptide of claim 1 wherein the first Ser is substituted with a hydrophobic amino acid residue or a neutral amino acid residue in the amino acid sequence of Ser-Ile-Glu-Gln-Ser-Cys-Asp.

10. The oligopeptide of claim 1 wherein the first Ser is substituted with Ala, Tyr, Thr, Pro, Phe, Val or Gly in the amino acid sequence of Ser-Ile-Glu-Gln-Ser-Cys-Asp.

11. The oligopeptide of claim 1 wherein the second Ile is substituted with a neutral amino acid residue or a hydrophobic amino acid residue in the amino acid sequence of Ser-Ile-Glu-Gln-Ser-Cys-Asp.

12. The oligopeptide of claim 1 wherein the second Ile is substituted with Gly, Asn, Thr, Val, Ser, Phe or Leu in the amino acid sequence of Ser-Ile-Glu-Gln-Ser-Cys-Asp.

13. The oligopeptide of claim 1 wherein the fifth Ser is substituted with a neutral amino acid residue or a hydrophobic amino acid residue in the amino acid sequence of Ser-Ile-Glu-Gln-Ser-Cys-Asp.

14. The oligopeptide of claim 1 wherein the fifth Ser is substituted with Trp, Phe, Thr, Cys, Tyr, Pro or Ala in the amino acid sequence of Ser-Ile-Glu-Gln-Ser-Cys-Asp.

15. The oligopeptide of claim 1 wherein the seventh Asp is substituted with a hydrophilic amino acid residue, Gly, Ala or Leu in the amino acid sequence of Ser-Ile-Glu-Gln-Ser-Cys-Asp.

16. The oligopeptide of claim 1 wherein the seventh Asp is substituted with Glu, His, Ser, Ala, Gly, Asn, Tyr or Leu in the amino acid sequence of Ser-Ile-Glu-Gln-Ser-Cys-Asp.

17. The oligopeptide of claim 1 wherein the third Glu is substituted with Lys, Gly, Gln, Arg, Ala, Val Asp or Trp in the amino acid sequence of Ser-Ile-Glu-Gln-Ser-Cys-Asp.

18. The oligopeptide of claim 1 wherein the fourth Gln is substituted with Pro, Glu, Thr, Arg, Ser, His or Lys in the amino acid sequence of Ser-Ile-Glu-Gln-Ser-Cys-Asp.

19. The oligopeptide of claim 1 wherein the first Ser is substituted with Thr or Tyr, the second Ile is substituted with Ser, Asn or Thr, the third Glu is substituted with Ala, Asp or Trp, the fifth Ser is substituted with Cys or Tyr, and/or the seventh Asp is substituted with Gly, Ala or Leu in the amino acid sequence of Ser-Ile-Glu-Gln-Ser-Cys-Asp.

20. The oligopeptide of claim 1 wherein 1 to 3 amino acid residues other than the third to sixth amino acid residues Glu-Gln-Ser-Cys are substituted in the amino acid sequence of Ser-Ile-Glu-Gln-Ser-Cys-Asp, the first Ser is substituted with Thr or Tyr, the second Ile is substituted with Ser, Asn or Thr, and/or the seventh Asp is substituted with Gly, Ala or Leu.

21. The oligopeptide of claim 1 which comprises at least an amino acid sequence wherein 1 to 3 amino acid residues are substituted in the amino acid sequence represented by Ser-Ile-Glu-Gln-Cys-Ser-Asp wherein the amino acid residue to be substituted is other than Cys or other than the third to sixth amino acid residues Glu-Gln-Cys-Ser.

22. The oligopeptide of claim 1 wherein 0 to 2 amino acid residues are substituted in the amino acid sequence represented by Glu-Gln-Cys-Ser-Asp; wherein the amino acid residue to be substituted is other than Cys or other than the third to sixth amino acid residues Glu-Gln-Cys-Ser.

23. The oligopeptide of claim 1 wherein the first Ser is substituted with a hydrophobic amino acid residue or a neutral amino acid residue in the amino acid sequence of Ser-Ile-Glu-Gln-Cys-Ser-Asp.

24. The oligopeptide of claim 1 wherein the first Ser is substituted with Ala, Tyr, Thr, Pro, Phe, Val or Gly in the amino acid sequence of Ser-Ile-Glu-Gln-Cys-Ser-Asp.

25. The oligopeptide of claim 1 wherein the second Ile is substituted with a neutral amino acid residue or a hydrophobic amino acid residue in the amino acid sequence of Ser-Ile-Glu-Gln-Cys-Ser-Asp.

26. The oligopeptide of claim 1 wherein the second Ile is substituted with Gly, Asn, Thr, Val, Ser, Phe or Leu in the amino acid sequence of Ser-Ile-Glu-Gln-Cys-Ser-Asp.

27. The oligopeptide of claim 1 wherein the sixth Ser is substituted with a neutral amino acid residue or a hydrophobic amino acid residue in the amino acid sequence of Ser-Ile-Glu-Gln-Cys-Ser-Asp.

28. The oligopeptide of claim 1 wherein the sixth Ser is substituted with Trp, Phe, Thr, Cys, Tyr, Pro or Ala in the amino acid sequence of Ser-Ile-Glu-Gln-Cys-Ser-Asp.

29. The oligopeptide of claim 1 wherein the seventh Asp is substituted with a hydrophilic amino acid residue, Gly, Ala or Leu in the amino acid sequence of Ser-Ile-Glu-Gln-Cys-Ser-Asp.

30. The oligopeptide of claim 1 wherein the seventh Asp is substituted with Glu, His, Ser, Ala, Gly, Asn, Tyr or Leu in the amino acid sequence of Ser-Ile-Glu-Gln-Cys-Ser-Asp.

31. The oligopeptide of claim 1 wherein the third Glu is substituted with Lys, Gly, Gln, Arg, Ala, Val, Asp or Trp in the amino acid sequence of Ser-Ile-Glu-Gln-Cys-Ser-Asp.

32. The oligopeptide of claim 1 wherein the fourth Gln is substituted with Pro, Glu, Thr, Arg, Ser, His or Lys in the amino acid sequence of Ser-Ile-Glu-Gln-Cys-Ser-Asp.

33. The oligopeptide of claim 1 wherein the first Ser is substituted with Thr or Tyr, the second Ile is substituted with Ser, Asn or Thr, the third Glu is substituted with Ala, Asp or Trp, the sixth Ser is substituted with Cys or Tyr, and/or the seventh Asp is substituted with Gly, Ala or Leu in the amino acid sequence of Ser-Ile-Glu-Gln-Cys-Ser-Asp.

34. The oligopeptide of claim 1 wherein 1 to 3 amino acid residues other than the third to sixth amino acid residues Glu-Gln-Cys-Ser are substituted in the amino acid sequence of Ser-Ile-Glu-Gln-Cys-Ser-Asp, the first Ser is substituted with Thr or Tyr, the second Ile is substituted with Ser, Asn or Thr, and/or the seventh Asp is substituted with Gly, Ala or Leu.

35. The oligopeptide of claim 1 which comprises at least an amino acid sequence wherein 1 to 3 amino acid residues are substituted in the amino acid sequence represented by Ser-Ile-Glu-Cys-Gln-Ser-Asp wherein the amino acid residue to be substituted is other than Cys or wherein the amino acid residue to be substituted is other than the third to sixth amino acid residues Glu-Cys-Gln-Ser.

36. The oligopeptide of claim 1 wherein 0 to 2 amino acid residues are substituted in the amino acid sequence represented by Glu-Cys-Gln-Ser-Asp; wherein the amino acid residue to be substituted is other than Cys or wherein the amino acid residue to be substituted is other than the third to sixth amino acid residues Glu-Cys-Gln-Ser.

37. The oligopeptide of claim 1 wherein the first Ser is substituted with a hydrophobic amino acid residue or a neutral amino acid residue in the amino acid sequence of Ser-Ile-Glu-Cys-Gln-Ser-Asp.

38. The oligopeptide of claim 1 wherein the first Ser is substituted with Ala, Tyr, Thr, Pro, Phe, Val or Gly in the amino acid sequence of Ser-Ile-Glu-Cys-Gln-Ser-Asp.

39. The oligopeptide of claim 1 wherein the second Ile is substituted with a neutral amino acid residue or a hydrophobic amino acid residue in the amino acid sequence of Ser-Ile-Glu-Cys-Gln-Ser-Asp.

40. The oligopeptide of claim 1 wherein the second Ile is substituted with Gly, Asn, Thr, Val, Ser, Phe or Leu in the amino acid sequence of Ser-Ile-Glu-Cys-Gln-Ser-Asp.

41. The oligopeptide of claim 1 wherein the sixth Ser is substituted with a neutral amino acid residue or a hydrophobic amino acid residue in the amino acid sequence of Ser-Ile-Glu-Cys-Gln-Ser-Asp.

42. The oligopeptide of claim 1 wherein the sixth Ser is substituted with Trp, Phe, Thr, Cys, Tyr, Pro or Ala in the amino acid sequence of Ser-Ile-Glu-Cys-Gln-Ser-Asp.

43. The oligopeptide of claim 1 wherein the seventh Asp is substituted with a hydrophilic amino acid residue, Gly, Ala or Leu in the amino acid sequence of Ser-Ile-Glu-Cys-Gln-Ser-Asp.

44. The oligopeptide of claim 1 wherein the seventh Asp is substituted with Glu, His, Ser, Ala, Gly, Asn, Tyr or Leu in the amino acid sequence of Ser-Ile-Glu-Cys-Gln-Ser-Asp.

45. The oligopeptide of claim 1 wherein the third Glu is substituted with Lys, Gly, Gln, Arg, Ala, Val, Asp or Trp in the amino acid sequence of Ser-Ile-Glu-Cys-Gln-Ser-Asp.

46. The oligopeptide of claim 1 wherein the fifth Gln is substituted with Pro, Glu, Thr, Arg, Ser, His or Lys in the amino acid sequence of Ser-Ile-Glu-Cys-Gln-Ser-Asp.

47. The oligopeptide of claim 1 wherein the first Ser is substituted with Thr or Tyr, the second Ile is substituted with Ser, Asn or Thr, the third Glu is substituted with Ala, Asp or Trp, the sixth Ser is substituted with Cys or Tyr, and/or the seventh Asp is substituted with Gly, Ala or Leu in the amino acid sequence of Ser-Ile-Glu-Cys-Gln-Ser-Asp.

48. The oligopeptide of claim 1 wherein 1 to 3 amino acid residues other than the third to sixth amino acid residues Glu-Cys-Gln-Ser are substituted in the amino acid sequence of Ser-Ile-Glu-Cys-Gln-Ser-Asp, the first Ser is substituted with Thr or Tyr, the second Ile is substituted with Ser, Asn or Thr, and/or the seventh Asp is substituted with Gly, Ala or Leu.

49. The oligopeptide of claim 1 wherein 1 to 3 amino acid residues are substituted in the amino acid sequence represented by Ser-Ile-Cys-Glu-Gln-Ser-Asp wherein the amino acid residue to be substituted is other than Cys or other than the third to sixth amino acid residues Cys-Glu-Gln-Ser.

50. The oligopeptide of claim 1 wherein 0 to 2 amino acid residues are substituted in the amino acid sequence represented by Cys-Glu-Gln-Ser-Asp, wherein the amino acid residue to be substituted is other than Cys or other than the third to sixth amino acid residues Cys-Glu-Gln-Ser.

51. The oligopeptide of claim 1 wherein the first Ser is substituted with a hydrophobic amino acid residue or a neutral amino acid residue in the amino acid sequence of Ser-Ile-Cys-Glu-Gln-Ser-Asp.

52. The oligopeptide of claim 1 wherein the first Ser is substituted with Ala, Tyr, Thr, Pro, Phe, Val or Gly in the amino acid sequence of Ser-Ile-Cys-Glu-Gln-Ser-Asp.

53. The oligopeptide of claim 1 wherein the second Ile is substituted with a neutral amino acid residue or a hydrophobic amino acid residue in the amino acid sequence of Ser-Ile-Cys-Glu-Gln-Ser-Asp.

54. The oligopeptide of claim 1 wherein the second Ile is substituted with Gly, Asn, Thr, Val, Ser, Phe or Leu in the amino acid sequence of Ser-Ile-Cys-Glu-Gln-Ser-Asp.

55. The oligopeptide of claim 1 wherein the sixth Ser is substituted with a neutral amino acid residue or a hydrophobic amino acid residue in the amino acid sequence of Ser-Ile-Cys-Glu-Gln-Ser-Asp.

56. The oligopeptide of claim 1 wherein the sixth Ser is substituted with Trp, Phe, Thr, Cys, Tyr, Pro or Ala in the amino acid sequence of Ser-Ile-Cys-Glu-Gln-Ser-Asp.

57. The oligopeptide of claim 1 wherein the seventh Asp is substituted with a hydrophilic amino acid residue, Gly, Ala or Leu in the amino acid sequence of Ser-Ile-Cys-Glu-Gln-Ser-Asp.

58. The oligopeptide of claim 1 wherein the seventh Asp is substituted with Glu, His, Ser, Ala, Gly, Asn, Tyr or Leu in the amino acid sequence of Ser-Ile-Cys-Glu-Gln-Ser-Asp.

59. The oligopeptide of claim 1 wherein the fourth Glu is substituted with Lys, Gly, Gln, Arg, Ala, Val, Asp or Trp in the amino acid sequence of Ser-Ile-Cys-Glu-Gln-Ser-Asp.

60. The oligopeptide of claim 1 wherein the fifth Gln is substituted with Pro, Glu, Thr, Arg, Ser, His or Lys in the amino acid sequence of Ser-Ile-Cys-Glu-Gln-Ser-Asp.

61. The oligopeptide of claim 1 wherein the first Ser is substituted with Thr or Tyr, the second Ile is substituted with Ser, Asn or Thr, the fourth Glu is substituted with Ala, Asp or Trp, the sixth Ser is substituted with Cys or Tyr, and/or the seventh Asp is substituted with Gly, Ala or Leu in the amino acid sequence of Ser-Ile-Cys-Glu-Gln-Ser-Asp.

62. The oligopeptide of claim 1 wherein 1 to 3 amino acid residues other than the third to sixth amino acid residues Cys-Glu-Gln-Ser are substituted in the amino acid sequence of Ser-Ile-Cys-Glu-Gln-Ser-Asp, the first Ser is substituted with Thr or Tyr, the second Ile is substituted with Ser, Asn or Thr, and/or the seventh Asp is substituted with Gly, Ala or Leu.

63. The oligopeptide of claim 1 wherein 1 to 3 amino acid residues are substituted in the amino acid sequence represented by Glu-Gln-Ser-Cys-Asp.

64. The oligopeptide of claim 1 wherein 1 to 3 amino acid residues are substituted in the amino acid sequence represented by Glu-Gln-Cys-Ser-Asp.

65. The oligopeptide of claim 1 wherein 1 to 3 amino acid residues are substituted in the amino acid sequence represented by Glu-Cys-Gln-Ser-Asp.

66. The oligopeptide of claim 1 wherein 1 to 3 amino acid residues are substituted in the amino acid sequence represented by Cys-Glu-Gln-Ser-Asp.

67. The oligopeptide of claim 1 comprising about 8 to about 20 amino acid . residues comprising an amino acid sequence selected from the group consisting of: 
Ser-Ile-Glu-Gln-Xaa-Ser-Asp-Gln;
Ser-Ile-Glu-Xaa-Gln-Ser-Asp-Gln; and
Ser-Ile-Xaa-Glu-Gln-Ser-Asp-Gln,
 wherein Xaa is Cys or a reactive substance-bound Cys.

68. The oligopeptide of claim 67 comprising an amino acid sequence selected from the group consisting of:

Ser-Ile-Glu-Gln-Cys-Ser-Asp-Gln;
Ser-Ile-Glu-Cys-Gln-Ser-Asp-Gln; and
Ser-Ile-Cys-Glu-Gln-Ser-Asp-Gln.

69. The oligopeptide of claim 1 wherein said oligopeptide has the amino acid sequence of any one of SEQ ID NO:3 through SEQ ID NO:124.

70. The oligopeptide of claim 1 comprising the following amino acid sequence:

Tyr Asn Glu Gln Ser Cys Asp Arg Glu Glu.

71. The oligopeptide of claim 1 comprising the following amino acid sequence:

Thr Ser Asp Gln Cys Cys Asp Pro Asp Lys.

72. The oligopeptide of claim 1 comprising the following amino acid sequence:

Pro Ser Glu Gln Ser Cys Ala Glu Glu Glu.

73. The oligopeptide of claim 1 comprising the following amino acid sequence:

Ser Asn Glu Gln Ser Cys Ala Val Ala Glu.

74. The oligopeptide of claim 1 comprising the following amino acid sequence:

Thr Thr Glu Gln Ser Cys Ala Val Asp Glu.

75. The oligopeptide of claim 1 comprising the following amino acid sequence:

Ser Ile Glu Gln Ser Cys Gly Gln His Glu.

76. The oligopeptide of claim 1 comprising the following amino acid sequence:

Ser Ser Ala Gln Ser Cys Leu Gln Asp Thr.

77. The oligopeptide of claim 1 comprising the following amino acid sequence:

Tyr Ile Glu Gln Tyr Cys Asp Gln Asp Glu.

78. The oligopeptide of claim 1 comprising the following amino acid sequence:

Thr Ile Trp Gln Ser Cys Asp Gln Glu Glu.

79. The oligopeptide of claim 1, wherein the amino acid sequence comprises natural amino acid residues, non-natural amino acid residues, or a mixture of both.

80. The oligopeptide of claim 3, wherein the amino acid sequence comprises natural amino acid residues, non-natural amino acid residues, or a mixture of both.

81. The oligopeptide of claim 1, which is a modified with a cross-linking agent.

82. The oligopeptide of claim 3, which is a modified with a cross-linking agent.

83. An oligopeptide polymer which is obtained by cross-linking an oligopeptide of claim 1, with the proviso that the polymer is not a homopolymer of SEQ ID NO:1 or SEQ ID NO:2 and not a homopolymer of oligopeptide Ser-Ile-Glu-Gln-Ser-Cys-Asp or Ser-Ile-Glu-Gln-Ser-Cys.

84. An oligopeptide polymer which is obtained by cross-linking an oligopeptide of claim 3, with the proviso that the polymer is not a homopolymer of oligopeptide Ser-Ile-Glu-Gln-Ser-Cys-Asp or Ser-Ile-Glu-Gln-Ser-Cys.

85. The oligopeptide polymer of claim 83 that is a dimer.

86. The oligopeptide polymer of claim 84 that is a dimer.

87. The oligopeptide of claim 85 wherein said dimer is a homodimer.

88. The oligopeptide of claim 85 wherein said dimer is a heterodimer.

89. The oligopeptide of claim 86 wherein said dimer is a homodimer.

90. The oligopeptide of claim 86 wherein said dimer is a heterodimer.

91. A composition comprising the oligopeptide of claim 1.

92. A composition comprising the oligopeptide of claim 3.

93. A composition comprising the oligopeptide of claim 67.

94. A composition comprising the oligopeptide of claim 68.

95. A composition comprising the oligopeptide polymer of claim 83.

96. A composition comprising the oligopeptide polymer of claim 84.

97. The composition of claim 91 further comprising a pharmaceutically acceptable excipient.

98. The composition of claim 91 further comprising an agent that enhances transdermal penetration.

99. A method of promoting hair growth in a mammal comprising administering a composition of claim 91 in an amount effective to promote hair growth in said mammal.

100. A method of promoting hair growth in a mammal comprising administering a composition of claim 92 in an amount effective to promote hair growth in said mammal.

101. A method of promoting hair growth in a mammal comprising administering a composition of claim 93 in an amount effective to promote hair growth in said mammal.

102. A method of promoting hair growth in a mammal comprising administering a composition of claim 95 in an amount effective to promote hair growth in said mammal.

103. A method of promoting hair growth in a mammal comprising administering a composition of claim 96 in an amount effective to promote hair growth in said mammal.

104. A monoclonal antibody, or fragment thereof, which specifically recognizes an antigen of about 220 kDa present in epithelial new follicles.

105. The monoclonal antibody of claim 104 wherein the antigen of about 220 kDa present in epithelial new follicles is an antigen which is specifically expressed during the growth period of an imago or the developing period of a fetus.

106. A monoclonal antibody, or a fragment thereof, produced by the hybridoma deposited with the Patent and Bio-Resource Center of National Institute of Advanced Industrial Science and Technology and having an accession number of FERM P-18578 (PERM BP-8121).

107. An antigen, or fragment thereof, recognized by the monoclonal antibody of claim 106.

108. A hybridoma which produces the monoclonal antibody of claim 104.

109. The hybridoma of claim 108 deposited with the Patent and Bio-Resource Center of National Institute of Advanced Industrial Science and Technology and having an accession number of FERM P-18578 (FERM BP-8121).

110. The hybridoma of claim 108 produced by the method comprising fusing immunocytes of a mammal immunized with an immunogen containing protein extracted from hair collected from the skin of a mammal in the growth period and/or follicles of whiskers of a mammal in a growth period, and myeloma cells of a mammal.

111. A process for the production of a monoclonal antibody specific for an antigen of about 220 kDa present in epithelial new follicles, which comprises the steps of incubating the hybridoma of claim 108, and collecting the monoclonal antibody produced by said hybridoma.

112. A method for the evaluation of hair growth promoting activity comprising the steps of;
(1) incubating skin tissue derived from a mammal in the presence of a substance to be tested under suitable conditions and for a time effective to promote hair growth;
(2) recovering said skin tissue from step (1);
(3) reacting said skin tissue with the monoclonal antibody of claim 106, or a fragment thereof; and;
(4) detecting said monoclonal antibody or a fragment thereof that reacted with the skin tissue.

113. The method of claim 112 wherein said monoclonal antibody is produced by the hybridoma of claim 109.

114. A kit for the evaluation of hair growth promoting activity which comprises the monoclonal antibody of claim 104 or a fragment thereof.

115. A kit of claim 114 which comprises the monoclonal antibody of claim 105, or a fragment thereof.

116. A kit of claim 114 which comprises the monoclonal antibody of claim 106, or a fragment thereof.
